(19) Europäisches Patentamt
European Patent Office
Office européen des brevets

(11) EP 4 327 889 A1

(12) EUROPEAN PATENT APPLICATION
published in accordance with Art. 153(4) EPC

(43) Date of publication:
**28.02.2024 Bulletin 2024/09**

(21) Application number: **22791819.0**

(22) Date of filing: **22.04.2022**

(51) International Patent Classification (IPC):
***A61Q 19/00*** (2006.01) ***C09K 3/00*** (2006.01)
***A61Q 5/00*** (2006.01) ***A61K 8/36*** (2006.01)
***A61K 8/44*** (2006.01)

(52) Cooperative Patent Classification (CPC):
**A61K 8/36; A61K 8/44; A61Q 5/00; A61Q 19/00; C09K 3/00**

(86) International application number:
**PCT/JP2022/018556**

(87) International publication number:
**WO 2022/225048 (27.10.2022 Gazette 2022/43)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **23.04.2021 JP 2021073004**

(71) Applicant: **Miyoshi Oil & Fat Co., Ltd.**
**Tokyo 124-8510 (JP)**

(72) Inventors:
- **YASHITA Akira**
  **Tokyo 124-8510 (JP)**
- **NAKAMURA Daisuke**
  **Tokyo 124-8510 (JP)**
- **TAKEKOSHI Kazuma**
  **Tokyo 124-8510 (JP)**
- **MIYAKE Shota**
  **Tokyo 124-8510 (JP)**
- **KANEKO Kotaro**
  **Tokyo 124-8510 (JP)**
- **KAWAI Koji**
  **Tokyo 124-8510 (JP)**

(74) Representative: **Angerhausen, Christoph**
**Boehmert & Boehmert**
**Anwaltspartnerschaft mbB**
**Pettenkoferstrasse 22**
**80336 München (DE)**

# (54) FORMULATION USING AMINO ACID AND CARBOXYLIC ACID, ORGANIC SALT, AND COMPOSITION COMPRISING SAME AND USE THEREOF

(57) Provided are a novel formulation using an amino acid and a carboxylic acid, said formulation being excellent in water retentivity, moisture absorbency, dissolution properties for a hardly soluble substance, antimicrobial properties, low skin irritation, biodegradability and gel-forming properties (thickening effect), an organic salt and a composition comprising the same. The formulation of the present invention comprises the following components (A) and (B). (A) An amino acid or a salt thereof. (B) A carboxylic acid or a salt thereof.



Processed by Luminess, 75001 PARIS (FR)

## Description

### TECHNICAL FIELD

The present invention relates to a formulation using an amino acid and a carboxylic acid, an organic salt thereof, and a composition comprising the same and a use thereof.

### BACKGROUND ART

Conventionally, formulations used in the fields of cosmetics or daily goods require physical safety and/or reduced environmental burden, and balancing of which with their functionality are being extensively discussed.

Further, a variety of antimicrobials has been used to date in various fields for preventing contamination or festering by microorganisms or for treating and/or preventing infection with microorganisms. Public health is becoming an increasingly important concern in recent years due to, for example, the outbreaks of new infectious diseases. In view of such historical background, there is a growing need of an agent for keeping the surfaces of human body or of an object to be in contact with the human body in a sanitary condition. Alcohol-based agents such as ethanol have normally been used for these purposes. Unfortunately, they have issues in that they are difficult to use for people who are constitutionally sensitive to alcohols and have poor long-term antimicrobial properties because they are highly volatile and do not remain on the surface. As an alternative, chlorine-based agents such as sodium hypochlorite aqueous solution or hypochlorous acid aqueous solution have been used. However, sodium hypochlorite is a strong alkaline for which an application of it to the human body is not recommended, and sodium hypochlorite releases chlorine gas when mixed with an acidic solution, which are examples of restrictions in use to which these agents are subjected. Moreover, hypochlorous acid solution has been problematic in that, for example, it is poor in long lasting antimicrobial property due to its problematic preservation stability, and that the active chlorine concentration is prone to be reduced when used. Thus, there is a need for an antimicrobial agent that is excellent in terms of safety, has low impact to the human body, and has low volatility and long-lasting efficacy.

Further, it is known that ingredients for imparting a water/moisture retention effect are added to a cosmetic for hair treatment and skin care, etc. However, these ingredients may sometimes cause a poor feeling of use such as a stickiness; desired are ingredients satisfying a water/moisture retention property and a feeling(s) of use.

Hair dries upon losing the moisture content therein due to, for example, a dry air, a shampoo with strong detergency, the heat of a dryer, natural drying after bathing, and damages from getting one's hair permed or colored. When the moisture content in the hair has reached an inadequate level due the drying of the hair, cuticle peeling will occur more easily; the cuticles are important tissues for imparting a gloss to the hair and keeping the hair healthy by covering the surface of the hair so as to prevent water from evaporating. As a result of cuticle peeling, the hair will dry and, for example, due to a loss in smoothness and a dryness of the hair, not only a poor flexibility and finger-combing capability of the hair will be observed, but the hair will be more likely to spread, and split ends and breakages will occur more easily. Therefore, water/moisture retention of the hair is essential in terms of avoiding problems owing to the drying of the hair and keeping the hair beautiful and healthy.

Conventionally, it is suggested that a multivalent alcohol(s) as a moisture-retaining ingredient be added to a hair treatment composition such as a shampoo (e.g., Patent documents 1 and 2). However, in the case of a conventional hair treatment composition, for example, there may not be achieved a satisfactory water/moisture retention property under a low-humidity environment such as an environment involving dryness after washing one's hair and an environment involving dryness during winter season, and the water/moisture retention property may not be able to be maintained for a long period of time due to a volatility of the composition; a novel hair treatment composition capable of compensating for these imperfections has been desired.

In Patent document 3, there are disclosed organic ammonium salts such as imidazolium salt, pyrrolidinium salt, piperidinium salt, pyridinium salt and phosphonium salt. While these types of organic ammonium salts have a property of retaining moisture, they have, for example, safety problems as well as a problem of not being able to exert a satisfactory water/moisture retention effect when used on the hair.

Although the applicant of the present invention has proposed an organic salt (ionic liquid) having a hydrogen-bonding functional group(s) in a cation or an anion (Patent documents 4 and 5), there has not specifically been considered any water/moisture retention effect thereof using an amino acid-based compound(s) as well as an application thereof to the hair and for skin care purpose.

It is known that skin roughness is mainly caused by reduction in moisture of the skin. For example, skin dries due to, for example, a dryness in the air during the winter season, skin cleansing, aging and a reduction in skin secretion. If neglecting a dry condition of the skin, the resilience and/or glow of the skin will decrease, which will easily lead to a so-called rough condition of the skin. In order to avoid skin roughness, it is essential to maintain a normal skin function by preventing the reduction in moisture contained in the horny layer. In order to retain the moisture content in the horny

layer, there are conventionally known various skin care agents for moisture retention purpose that are capable of imparting a proper amount of moisture to the skin.

Conventionally, it is suggested that a multivalent alcohol(s) as a moisture-retaining ingredient be added to a skin care agent (See, e.g., Patent document 1). However, in the case of a conventional skin care agent, for example, there may not be achieved a satisfactory water/moisture retention property under a low-humidity environment such as an environment involving dryness during winter season, and the water/moisture retention property may not be able to be maintained for a long period of time due to a volatility of the agent; a novel skin care agent capable of compensating for these imperfections has been desired.

In Patent document 3, there are disclosed organic ammonium salts such as imidazolium salt, pyrrolidinium salt, piperidinium salt, pyridinium salt and phosphonium salt. While these types of organic ammonium salts have a property of retaining moisture, they have, for example, safety problems as well as a problem of not being able to exert a satisfactory water/moisture retention effect when used for skin care purpose.

The applicant has proposed an organic ammonium salt (ionic liquid) that is liquid at room temperature having a hydrogen-bonding functional group(s) in cations or anions (Patent documents 4 and 5).

## PRIOR ART DOCUMENTS

### Patent documents

Patent document 1: JP-A-2014-131974
Patent document 2: JP-A-2014-131975
Patent document 3: JP-A-2019-023185
Patent document 4: WO2020/166674(A1)
Patent document 5: WO2020/166678(A1)


## SUMMARY OF THE INVENTION

### Problems to be solved by the invention

Nevertheless, a new compound that is suitable for the above-mentioned problems is highly desired. Moreover, no study has ever been made for a combination of an anion and an amine as a cation of an organic ammonium salt, particularly for a compound having a cationic structure of an amino acid, with the focuses on water retentivity, moisture absorbency, dissolution properties for a hardly soluble substance, antimicrobial properties, low dermal irritancy, biodegradability and gel-forming properties (thickening effect).

The present invention has been made in view of the aforementioned circumstances. A main object of the present invention is to provide a novel formulation using an amino acid and a carboxylic acid, said formulation being excellent in water retentivity, moisture absorbency, dissolution properties for a hardly soluble substance, antimicrobial properties, low skin irritation, biodegradability and gel-forming properties (thickening effect), an organic salt and a composition comprising the same. It is also an object of the invention to maintain the above-mentioned properties for a long period of time without having any volatility.

### Means to solve the problems

In order to solve the above problems, the present invention provide a formulation comprising of the following components (A) and (B): (A) an amino acid or a salt thereof; and (B) a carboxylic acid or a salt thereof.

Further, the present invention provides an organic salt formed by a cation originated from the component (A); and an anion originated from anionic residues of the component (B), wherein the cation originated from the component (A) optionally contains a cationic residue of the component (B). The invention also provides the formulation containing the same.

The composition according to the present invention contains the above-mentioned formulation.

The formulation or composition may also be used for cosmetics to impart water retentivity, moisture absorbency and/or antimicrobial properties. The formulation or composition may also be used as a gel composition that contains a polymer compound and water.

Effects of the invention

The novel formulation using the amino acid and the carboxylic acid as well as the composition containing the same, according to the present invention, are excellent in water retentivity, moisture absorbency, dissolution properties for a hardly soluble substance, antimicrobial properties, low skin irritation, biodegradability and gel-forming properties (thickening effect). They have no volatility and are excellent in retaining the above-listed effects for a long time.

MODE FOR CARRYING OUT THE INVENTION

The present invention is described in detail hereunder.

The term "formulation" as used herein includes the one having components (A) and (B) being compounded before finally preparing the intended formulation. The term also encompasses a case in which a salt is formed by the components (A) and (B) as starting materials, and the salt is used as a formulation, and a case in which the aforementioned salt is mixed, as needed, with further components such as water to make a formulation. The formulation as used herein may be a mixture composed only of the components (A) and (B) (including a case in which the salts thereof are contained therein), or a composition further containing a component, such as water, other than the components (A) and (B) and their salts, or a composition serving as an additive to be added when manufacturing a product, or a composition for a product such as antibacterial/ antivirus agents and cosmetics. The number of carbon atoms as used herein is an integer.

The component (A) according to the present invention is an amino acid or a salt thereof. In the present invention, the amino acid encompasses a compound having a carboxy group (-COOH), as an acidic group, and an amino group (primary amino group, secondary amino group, tertiary amino group), as a basic group, in the molecule. Typical preferable examples of such include, for example, a proteinogenic amino acid and a free amino acid in a living body.

An example of the amino acid salt includes a carboxylate salt in which at least one of the carboxy groups of the amino acid is substituted by a cation (such as an alkali metal cation, alkali earth metal cation or ammonium cation).

It is preferred that the component (A) be represented by the following formula (I):

[Chemical formula 1]

$$R^1_lNH_mC(R^2)_2(R^3_nCOOX) \qquad (I)$$

wherein $R^1$ represents a monovalent or bivalent organic group having 1 to 22 carbon atoms, $R^2$ independently represents a hydrogen atom or a monovalent or bivalent organic group having 1 to 22 carbon atoms, $R^3$ represents a bivalent organic group having 1 to 22 carbon atoms, 1 represents any one of 0 to 2, m represents any one of 0 to 2, and n represents 0 or 1. $R^1$ and $R^2$ may together form a ring having 3 to 22 carbon atoms. X represents a hydrogen atom or a monovalent cation. In the formula (I), 1 is preferably 0 or 1, and m is preferably 1 or 2. The wording "$R^1$ and $R^2$ may together form a ring having 3 to 22 carbon atoms" as used herein refers to that $R^1$ and $R^2$ in combination form a ring of $R^1$, $R^2$ and C having nitrogen N in the ring in the unit of $R^1_lNH_mCR^2$ with the total number of carbons being 3 to 22, preferably 4 to 10. The ring optionally contains not only 3 to 22 carbons that form the ring but also a monovalent or bivalent organic group having 1 to 22, preferably 1 to 10, more preferably 1 to 3 carbon atoms.

[Organic group]

The "organic group" as used herein essentially contains carbon atom(s) and optionally contains at least one further species selected from hydrogen atom and heteroatoms. Examples of the heteroatoms are not particularly limited but preferably include oxygen atom, nitrogen atom, sulfur atom, phosphorus atom, and halogen atom among which more preferred are oxygen atom, nitrogen atom and sulfur atom. Examples of atom group(s) contained in the organic group include, but are not limited to, a hydrocarbon group, a heterocyclic group, and a substituent group as set forth in the section of "substituent group" to be hereinafter described. Examples of such also include a group in which the organic group or the substituent group, as set forth in the section of "substituent group" to be hereinafter described, or other group substitutes a hydrogen atom (s) in a hydrocarbon group, interrupts a hydrocarbon group, and/or is present at a base end(s) of a hydrocarbon group, as well as a group in which the substituent group, as set forth in the section of "substituent group" to be hereinafter described, or other group forms a condensed ring with an aromatic hydrocarbon group.

[Hydrocarbon group]

Examples of the hydrocarbon group as used herein include, but are not limited to, a saturated or unsaturated aliphatic hydrocarbon group, a saturated or unsaturated alicyclic hydrocarbon group, an aromatic hydrocarbon group and a hydrocarbon group of any combination of the preceding. The group may be monovalent or multivalent depending

on the context of the application, and examples of the saturated or unsaturated monovalent aliphatic hydrocarbon group include, but are not limited to, a linear or branched alkyl group, alkenyl group, and alkynyl group. Examples of the alkyl group include linear or branched ones, and include, but are not particularly limited to, a methyl group, ethane-1-yl group, propane-1-yl group, 1-methyl ethane-1-yl group, butane-1-yl group, butane-2-yl group, 2-methyl propane-1-yl group, 2-methyl propane-2-yl group, pentane-1-yl group, pentane-2-yl group, hexane-1-yl group, heptane-1-yl group, octane-1-yl group, 2-ethyl hexane-1-yl group, 1,1,3,3-tetramethyl butane-1-yl group, nonane-1-yl group, decane-1-yl group, undecane-1-yl group, dodecane-1-yl group, tridecane-1-yl group, tetradecane-1-yl group, pentadecane-1-yl group, hexadecane-1-yl group, 2-hexyl decane-1-yl group, heptadecane-1-yl group, octadecane-1-yl group, nonadecane-1-yl group, icosane-1-yl group, henicosane-1-yl group, docosane-1-yl group, and 4,8,12-trimethyl tridecane-1-yl group. Examples of the alkenyl group include linear or branched ones, and include, but are not particularly limited to, a vinyl group, prop-1-en-1-yl group, allyl group, isopropenyl group, but-1-en-1-yl group, but-2-en-1-yl group, but-3-en-1-yl group, 2-methylprop-2-en-1-yl group, 1-methylprop-2-en-1-yl group, pent-1-en-1-yl group,pent-2-en-1-yl group, pent-3-en-1-yl group, pent-4-en-1-yl group, 3-methylbut-2-en-1-yl group, 3-methylbut-3-en-1-yl group, hex-1-en-1-yl group, hex-2-en-1-yl group, hex-3-en-1-yl group,hex-4-en-1-yl group, hex-5-en-1-yl group, 4-methylpent-3-en-1-yl group, hept-1-en-1-yl group, hept-6-en-1-yl group, oct-1-en-1-yl group, oct-7-en-1-yl group, non-1-en-1-yl group, non-8-en-1-yl group, dec-1-en-1-yl group, dec-9-en-1-yl group, undec-1-en-1-yl group, undec-10-en-1-yl group, dodec-1-en-1-yl group, dodec-11-en-1-yl group, tridec-1-en-1-yl group, tridec-12-en-1-yl group, tetradec-1-en-1-yl group, tetradec-13-en-1-yl group, pentadec-1-en-1-yl group, pentadec-14-en-1-yl group, hexadec-1-en-1-yl group, hexadec-15-en-1-yl group, heptadec-1-en-1-yl group, heptadec-16-en-1-yl group, octadec-1-en-1-yl group, octadec-9-en-1-yl group, octadec-17-en-1-yl group, nonadec-1-en-1-yl group, icos-1-en-1-yl group, henicos-1-en-1-yl group, and docos-1-en-1-yl group. Examples of the alkynyl group include linear or branched ones, and include, but are not particularly limited to, an ethynyl, prop-1-yn-1-yl group, prop-2-yn-1-yl group, but-1-yn-1-yl group, but-3-yn-1-yl group, 1-methylprop-2-yn-1-yl group, pent-1-yn-1-yl group, pent-4-yn-1-yl group, hex-1-yn-1-yl group, hex-5-yn-1-yl group, hept-1-yn-1-yl group, hept-6-yn-1-yl group, oct-1-yn-1-yl group, oct-7-yn-1-yl group, non-1-yn-1-yl group, non-8-yn-1-yl group, dec-1-yn-1-yl group, dec-9-yn-1-yl group, undec-1-yn-1-yl group, undec-10-yn-1-yl group, dodec-1-yn-1-yl group, dodec-11-yn-1-yl group, tridec-1-yn-1-yl group, tridec-12-yn-1-yl group, tetradec-1-yn-1-yl group, tetradec-13-yn-1-yl group, pentadec-1-yn-1-yl group, pentadec-14-yn-1-yl group, hexadec-1-yn-1-yl group, hexadec-15-yn-1-yl group, heptadec-1-yn-1-yl group, heptadec-16-yn-1-yl group, octadec-1-yn-1-yl group, octadec-17-yn-1-yl group, nonadec-1-yn-1-yl group, icos-1-yn-1-yl group, henicos-1-yn-1-yl group, and a docos-1-yn-1-yl group.

The saturated or unsaturated alicyclic hydrocarbon group is preferably a saturated alicyclic hydrocarbon group, and examples of which include, but are not particularly limited to, as monovalent groups, a cyclopropyl group, cyclobutyl group, cyclopentyl group, cyclohexyl group, cycloheptyl group and a cyclooctyl group, as well as groups containing alicyclic residues of those or other residues.

Examples of the aromatic hydrocarbon group include, but are not particularly limited to, a phenyl group, naphthalene group, an anthracene group, as well as groups containing aromatic ring residues of those or other residues. The group may form a condensed ring together with the substituent groups as set forth in [Substituent Group] to be described below. Examples of the monovalent aromatic hydrocarbon group include, but are not particularly limited to, a phenyl group, 2-methylphenyl group, 3-methylphenyl group, 4-methylphenyl group, 2,4-dimethylphenyl group, 2,5-dimethylphenyl group, 3,4-dimethylphenyl group, 3,5-dimethylphenyl group, 2,4,5-trimethylphenyl group, 2,4,6-trimethylphenyl group, 4-ethylphenyl group, 4-propylphenyl group, 4-isopropylphenyl group, 4-butylphenyl group, 4-tert-butylphenyl group, benzyl group, $\alpha,\alpha$-dimethylbenzyl group, 4-pentylphenyl group, 4-tert-pentylphenyl group, 2,4-bis(4-tert-pentyl)phenyl group, 1,1,3,3-tetramethylbutylphenyl group, 2-methyl-5-tert-butylphenyl group, 4-pentylphenyl group, 4-hexylphenyl group, 4- heptylphenyl group, 4-octylphenyl group, 4-nonylphenyl group, 4-decanylphenyl group, 4-undecylphenyl group, 4-dodecylphenyl group, 4-tridecylphenyl group, 4-tetradecylphenyl group, 4-pentadecylphenyl group, 4-hexadecylphenyl group, 4-heptadecylphenyl group, 4-octadecylphenyl group,4-biphenyl group, 2-methoxyphenyl group, 3-methoxyphenyl group, 4-methoxyphenyl group, 2-ethoxyphenyl group, 3-ethoxyphenyl group, 4-ethoxyphenyl group, 2-chlorophenyl group, 2-fluorophenyl group, 4-fluorophenyl group, 2-trifluoromethylphenyl group, 4-trifluoromethylphenyl group, 4-hydroxyphenyl group, 1-naphthyl group, 2-naphthyl group, 1-anthracenyl group, 2-anthracenyl group and 9-anthracenyl group.

Examples of the bivalent aromatic group include groups having a structure established by subtracting one hydrogen atom from the aforementioned groups.

[Substituent Group]

Examples of the above-mentioned substituent group include, but are particularly limited to, a hydrocarbon group, an oxygen-containing group, a nitrogen-containing group, a sulfur-containing group, a phosphorus-containing group, and a halogen. The substituent group also includes a group to which these substituent groups are connected. Examples of the above-mentioned hydrocarbon group include those listed in [Substituent Group] as mentioned above.

Although not particularly limited, examples of the oxygen-containing group include a hydroxy group, an alkoxy group, an acetoxy group, an acetyl group, an aldehyde group, a carboxy group, a carboxylate group, a urea group, a urethane group, an amide group, an imide group, an ether group, a carbonyl group, an ester group, an oxazole group, a morpholin group, a carbamate group, a carbamoyl group, a polyoxyethylene group, a tocopheryl group, a chroman group, a dihydropyran group, a glyceryl group, and a glyceryl ether group.

Examples of the nitrogen-containing group include, but are not particularly limited to, a cyano group, a cyanato group, an isocyanate group, a nitro group, a nitroalkyl group, an amide group, a urea group, a urethane group, an imide group, a carbodiimide group, an azo group, a pyridine group, a guanidino group, an imidazolyl group, an indolyl group, a primary amino group, a secondary amino group, a tertiary amino group, a quaternary ammonium group, and an aminoalkyl group.

Examples of the sulfur-containing group include, but are not particularly limited to, a sulfate group, a sulfonyl group, a sulfonate group, a mercapto group, a thioether group, a thiocarbonyl group, a thiourea group, a thiocarboxy group, a thiocarboxylate group, a dithiocarboxy group, a dithiocarboxylate group, a sulfuric ester, a thiophene group, a thiazole group, a thiol group, a sulfo group, a sulfide group, a disulfide group, a thioester group, a thioamide group, a thiocarbamate group, and a dithiocarbamate group, and esters thereof.

Examples of the phosphorus-containing group include, but are not particularly limited to, a phosphate group, a phosphorous acid group, a phosphonic acid group, a phosphinic acid group, a phosphonous acid group, a phosphinous acid group, a pyrophosphate group, a phosphate group, a phosphorous acid ester group, a phosphonic acid ester group, a pyrophosphate group and esters thereof. Examples of the halogen include fluorine, chlorine, bromine, and iodine.

Examples of the organic group for $R^1$ or $R^2$ in the formula (I) include hydrocarbon groups which optionally contain a substituent group(s), and the hydrocarbon moiety thereof may contain an oxygen atom(s). Regarding such hydrocarbon group, the list as described in [Substituent Group] shown above may be referred to. The hydrocarbon group is preferably an aliphatic hydrocarbon group, among which a saturated aliphatic hydrocarbon group (such as an alkyl group) is more preferred. Such alkyl group may be, for example, a linear or branched group having 1 to 22, 1 to 10, or 1 to 5 carbon atoms.

The aforementioned hydrocarbon groups may contain a substituent group(s), and examples of such substituent groups include, but are not particularly limited to, those listed in the [Substituent Group] as mentioned above. Among the substituent groups, preferred are those having an oxygen-containing group,
a nitrogen-containing group, and a sulfur-containing group, among which a hydroxy group, a carboxy group, a carboxylate group, an ester group, an ether group, an alkoxy group, an amino group, an amide group, a guanidino group a imidazolyl group an indolyl group a mercapto group and a thioether group are preferred.

The aforementioned hydrocarbon moiety may contain an oxygen atom(s), and in which case such hydrocarbon moiety contains the aforementioned oxygen-containing group, and forms or contains, for example, an ether bond, a carbonyl group, a hydroxy group, a carboxylate group, an ester bond, an amide bond, a urea bond or a urethane bond. Therefore, in this invention, the wording "hydrocarbon moiety contains an oxygen atom(s)" as used herein encompasses a case where the hydrocarbon moiety is interrupted by, or the hydrogen atom(s) thereof are substituted by a group, serving as an oxygen atom-containing atom group, that even may contain a hetero atom(s) such as a nitrogen atom, or a case where the group is contained therein is present at its base end.

The aforementioned hydrocarbon groups may have a hydrogen-bonding functional group(s), and examples of the hydrogen-bonding functional group include, but not particularly be limited to, an oxygen-containing group, a nitrogen-containing group, a sulfur-containing group, a phosphorus-containing group, and a hydrogen atom directly bonded to nitrogen which are all as listed in the above.

Examples of the organic group for $R^3$ in the formula (I) include hydrocarbon groups which optionally contain a substituent group(s), and the hydrocarbon moiety thereof may contain an oxygen atom(s). Examples of the hydrocarbon group include the contents as referred to and set forth in the section of [Substituent Group] which are bivalent groups having a structure established by subtracting one hydrogen atom from the aforementioned hydrocarbon groups. The hydrocarbon group is preferably an aliphatic hydrocarbon group, among which a saturated aliphatic hydrocarbon group (such as an alkylene group) is more preferred. Such alkylene group may be, for example, a linear or branched group having 1 to 22, 1 to 10 or 1 to 5 carbon atoms.

The aforementioned hydrocarbon groups may contain a substituent group(s), and examples of such substituent group include, but are not particularly limited to, those listed in the [Substituent Group] as mentioned above.

The component (A) in (I) is preferably an amino acid wherein X is a hydrogen atom.

The amino acid includes a compound having, in one molecule, at least one amino group (primary, secondary, and/or tertiary amino group(s)) and at least one carboxy group (-COO-). The amido group is not included in the amino group.

Preferable embodiments of the amino acids in terms of isoelectric point and the ratio between the number of amino groups and the number of carboxy groups include the following (heteafter referred to as amino acids (a) through (f)).

**Amino acid (a):**

A ratio of the total number of primary or secondary amino groups to the number of carboxy groups in the component (A) is greater than 1.
Examples of such amino acids include, but are not particularly limited to, arginine, histidine, lysine and tryptophan.

**Amino acid (b):**

A ratio of the total number of primary or secondary amino groups to the number of carboxy groups in the component (A) is 1.
Examples of such amino acids include, but are not particularly limited to, leucine, isoleucine, phenylalanine, proline, valine, serine, alanine, threonine, glutamine, asparagine, aminobutyric acid, cysteine, glycine and methionine.

**Amino acid (c):**

A ratio of the total number of primary or secondary amino groups to the number of carboxy groups in the component (A) is less than 1.
Examples of such amino acids include, but are not particularly limited to, glutamic acid and aspartic acid.

**Amino acid (d):**

The component (A) has an isoelectric point of greater than 7.
Examples of such amino acids include, but are not particularly limited to, those categorized as basic amino acids such as arginine (10.76), histidine (7.59), lysine (9.75) and aminobutyric acid (7.85). The numerals in the parenthesis indicate isoelectric points of the respective amino acids.

**Amino acid (e):**

The component (A) has an isoelectric point of 4 or more and 7 or less.
Examples of such amino acids include, but are not particularly limited to, those categorized as neutral amino acids such as leucine (5.98), isoleucine (6.02), phenylalanine (5.48), proline (6.30), valine (5.96), tryptophan (5.89), serine (5.68), alanine (6.00), threonine (6.16), glutamine (5.65), asparagine (5.41), cysteine (5.07), glycine (5.97), methionine (5.74) and tyrosine (5.66). The numerals in the parenthesis indicate isoelectric points of the respective amino acids.

**Amino acid (f):**

The component (A) has an isoelectric point of less than 4.
Examples of such amino acids include, but are not particularly limited to, those categorized as acidic amino acids such as glutamic acid (3.22) and aspartic acid (2.77). The numerals in the parenthesis indicate isoelectric points of the respective amino acids.
Examples of preferable combinations of $R^1$, $R^2$ and $R^3$ in the formula (I) are as follows.
In the formula (I), $R^1$ is an aliphatic hydrocarbon group, $R^2$ is a hydrocarbon group that may contain an oxygen atom, a nitrogen atom, and/or a sulfur atom, and $R^3$ is an aliphatic hydrocarbon group. Examples of such amino acids include, but are not particularly limited to, arginine, histidine, lysine, glutamic acid, aspartic acid, leucine, phenylalanine, proline, valine, tryptophan, serine, isoleucine, alanine, threonine, glutamine, asparagine, aminobutyric acid, cysteine, glycine and methionine.
In the formula (I), l is 0, m is 2, n is 0, and $R^2$ is a primary amino group or a hydrocarbon group having at least two nitrogen atoms. Examples of such amino acids include, but are not particularly limited to, arginine, histidine and lysine.
In the formula (I), $R^1$ and $R^2$ are each a hydrocarbon group where at least one of them contains a carboxy group. Examples of such amino acids include, but are not particularly limited to, glutamic acid and aspartic acid.
In the formula (I), $R^1$ and $R^2$ are each a hydrocarbon group where at least one of them contains any one of a hydroxy group, an amide group, a secondary amino group and a sulfur-containing group, or $R^1$ and $R^2$ together form a ring. Examples of such amino acids include, but are not particularly limited to, leucine, phenylalanine, proline, valine, tryptophan, serine, isoleucine, alanine, threonine, glutamine, asparagine, aminobutyric acid, cysteine, glycine and methionine.
Examples of the amino acids include, but are not particularly limited to, those categorized as amino acids having

isoelectric points of more than 7, those categorized as amino acids having isoelectric points of 4 or more and 7 or less, and amino acids having isoelectric points of less than 4.

Examples of the amino acids having isoelectric points of 4 or more and 7 or less include, but are not particularly limited to, an amino acid having an alkyl-chain in $R^2$, an amino acid having a hydroxy group in $R^2$, an amino acid having sulfur in $R^2$, an amino acid having an amide group in $R^2$, an amino acid having an imino group in $R^2$, an amino acid having an aromatic group in $R^2$, and β-, γ-, δ-, and ε-amino acids.

Examples of the amino acid having an alkyl-chain in $R^2$ include, but are not particularly limited to, amino acids where $R^2$is an hydrogen atom or a liners or branched alkyl group having 1 to 10, preferably 1 to 4 carbon atoms. In this case, it is preferred that 1 be 0, or that 1 be 1 and $R^1$ be a liners or branched alkyl group having 1 to 3 carbon atoms. It is also preferred that n be 0. Specific examples thereof include, for example, glycine, alanine, valine, leucine, isoleucine and sarcosine.

The amino acid having a hydroxy group in $R^2$ may be an amino acid where $R^2$ is a linear or branched hydroxyalkyl group having 1 to 5, preferably 1 to 3, carbon atoms and having 1 to 3, preferably one, hydroxy group(s). In this case, it is preferred that l be 0, or that 1 be 1 and $R^1$ be a liners or branched alkyl group having 1 to 3 carbon atoms. The case where I is 0 is more preferred. It is also preferred that n be 0. Specific examples thereof include, for example, serine and threonine.

Examples of the amino acid having sulfur in $R^2$ include an amino acid where $R^2$ is represented by the following formula:

[Chemical formula 2] $(-R^{21}-)_{a1}(-S-)_{a2}R^{22}$

(In the formula, $R^{21}$ represents a methylene group and $R^{22}$ represents a methyl group or $-CH_2CH(NH_2)(COOH)$. a1 indicates any of 1 to 5, preferably of 1 to 3, and a2 indicates any of 1 to 4, preferably of 1 or 2. The a1 sets of S and a2 sets of $R^{22}$ may be in any order.)

In this case, it is preferred that 1 be 0, or that 1 be 1 and $R^1$ be a liners or branched alkyl group having 1 to 3 carbon atoms. The case where I is 0 is more preferred. It is also preferred that n be 0. Specific examples thereof include, for example, cysteine, methionine and cystathionine.

Examples of the amino acid having an amide group in $R^2$ include an amino acid where $R^2$ is represented by the following formula:

[Chemical formula 3] $-R^{23}-C(=O)NH_2$

(In the formula, $R^{23}$ represents a linear or branched alkylene group having 1 to 5, preferably 1 or 2 carbon atoms.)

In this case, it is preferred that 1 be 0, or that 1 be 1 and $R^1$ be a liners or branched alkyl group having a secondary amino group and 1 to 3 carbon atoms. The case where lis 0 is more preferred. It is also preferred that n be 0. Specific examples thereof include, for example, asparagine, glutamine and citrulline.

Examples of the amino acid having an imino group in $R^2$ include a compound where N and $R^1$ together form a hetero ring. $R^1$ represents an alkylene group having 3 to 4 carbon atoms and optionally having a hydroxy group, and forms a pyrrolidine ring or a piperidine ring. It is preferred that $R^1$ form a pyrrolidine ring. In this case, 1 is preferably 0. It is also preferred that n be 0. Specific examples thereof include, for example, proline and hydroxyproline. Examples of the amino acid having an aromatic group in $R^2$ include an amino acid where $R^2$ is represented by the following formula:

[Chemical formula 4] $-R^{24}-R^{25}$

(In the formula, $R^{24}$ represents a linear or branched alkylene group having 1 to 5, preferably 1 or 2 carbon atoms and $R^{25}$ represents an aromatic hydrocarbon group having 6 to 10 carbon atoms and optionally having a substituted group, or a hetero ring group having 6 to 10 carbon atoms.) $R^{25}$ preferably represents a phenyl group, a hydroxyphenyl group or an indole group.

In this case, 1 is preferably 0, or it is preferred that 1 be 1 and $R^1$ be a liners or branched alkyl group having 1 to 3 carbon atoms., or that 1 be 1 and $R^1$ be a group having 1 to 3 carbon atom(s) and a secondary amino group. The case where I is 0 is more preferred. It is also preferred that n be 0. Specific examples of such include, for example, phenylalanine, tyrosine, tryptophan, 1-methyl histidine, 3-methyl histidine, anserine, and carnosine.

Examples of the β, γ, δ, or ε-amino acid include an amino acid where $R^3$ is a linear or branched alkylene group having 1 to 4 carbon atoms. In this case, it is preferred that 1 be 0, or that 1 be 1 and $R^1$ be a liners or branched alkyl group having 1 to 3 carbon atoms. It is more preferred that 1 be 0. It is also preferred that n be 1. Specific examples of such include, for example, β-alanine, β-aminoisobutyric acid, γ-aminobutyric acid and ε-aminocaproic acid.

Examples of the amino acid having an isoelectric point of less than 4 include an amino acid where $R^2$ is represented by the following formula:

[Chemical formula 5] $-R^{26}COOH$

(In the formula, $R^{26}$ represents a bivalent aliphatic hydrocarbon group having 1 to 10 carbon atoms.)

In this case, it is preferred that 1 be 0, or that 1 be 1 and $R^1$ be a liners or branched alkyl group having 1 to 3 carbon atoms. It is more preferred that l be 0. It is also preferred that n be 0. Specific examples of such include, for example, glutamic acid, aspartic acid, and $\alpha$-aminoadipic acid.

Examples of the amino acid having an isoelectric point of greater than 7 include an amino acid where $R^2$ is represented by the following formula:

[Chemical formula 6] $-R^{27}-R^{28}$

(In the formula, $R^{27}$ represents a linear or branched alkylene group having 1 to 10 carbon atoms, which optionally has a hydroxy group, and $R^{28}$ represents $-NH_2$, $-NHC(=NH)(NH_2)$, or an imidazolyl group.)

In this case, it is preferred that 1 be 0, or that 1 be 1 and $R^1$ be a liners or branched alkyl group having 1 to 3 carbon atoms. It is more preferred that 1 be 0. It is also preferred that n be 0. Specific examples of such include, for example, arginine, lysine, histidine, 5-hydroxylysine and omithine.

According to the present invention, the component (B) is a carboxylic acid or a salt thereof. It is preferred that the component (B) be a carboxylic acid. The carboxylic acid is an organic acid having at least one carboxy group (-COO-) in the molecule, and optionally contains, for example, an oxygen-containing group, a nitrogen-containing group, a sulfur-containing group, a phosphorus-containing group, a hydrocarbon group, among which a carboxylic acid having a hydrocarbon group is preferred. Examples of the carboxylic acid having a hydrocarbon group include, but are not particularly limited to, those having a carboxy group and a hydrocarbon group of, for example, a saturated or unsaturated aliphatic hydrocarbon group, a saturated or unsaturated alicyclic hydrocarbon group and an aromatic hydrocarbon group or the combination of the preceding, and the specific examples include, for example, a saturated aliphatic carboxylic acid, an unsaturated aliphatic carboxylic acid, a saturated or unsaturated alicyclic carboxylic acid, an aromatic carboxylic acid, a saturated aliphatic hydroxycarboxylic acid, an unsaturated aliphatic hydroxycarboxylic acid, a saturated or unsaturated alicyclic hydroxycarboxylic acid, an aromatic hydroxycarboxylic acid, a carbonyl carboxylic acid, an alkyl ether carboxylic acid and a halogen carboxylic acid. Note that the number of carbon atoms as used hereunder include the number of carbons in carboxy group(s).

The saturated aliphatic carboxylic acid is comprised of a linear or branched saturated aliphatic hydrocarbon group and at least one carboxy group, and preferabl y has 1 to 22 carbon atoms. Examples of the saturated aliphatic carboxylic acid include, for example, a saturated aliphatic monocarboxylic acid having one carboxy group and a saturated aliphatic dicarboxylic acid having two carboxy groups. The saturated aliphatic monocarboxylic acid is comprised of a linear or branched saturat ed aliphatic hydrocarbon group and one carboxy group, and preferably has 1 to 22 carbon atoms. Particularly, preferred are a saturated aliphatic monocarboxylic acid selected from HCOOH and $CH_3(CH_2)_pCOOH$ (p is an integer of 0 to 8) and a satu rated aliphatic monocarboxylic acid having a branched-chain. Although not particul arly limited, specific examples thereof include, for example, formic acid, acetic aci d, propionic acid, butyric acid, valeric acid, caproic acid, enanthic acid, caprylic ac id, pelargonic acid, capric acid, lauric acid, myristic acid, pentadecylic acid, palmiti c acid, margaric acid, stearic acid, arachidic acid, heneicosylic acid, behenic acid, i sobutyric acid, 2-methylbutyric acid, isovaleric acid, 2-ethylhexanoic acid, isononano ic acid, isopalmitic acid and isostearic acid. The saturated aliphatic dicarboxylic a cid is comprised of a linear or branched saturated aliphatic hydrocarbon group and two carboxy groups, and preferably has 2 to 22 carbon atoms. Particularly, a satur ated dicarboxylic acid represented by $HOOC(CH_2)_xCOOH$ (x is an integer of 0 to 4) is preferred. Although not particularly limited, specific examples thereof includ e, for example, oxalic acid, malonic acid, succinic acid, glutaric acid, adipic acid, pimelic acid, suberic acid, azelaic acid, sebacic acid and glutamic acid.

The unsaturated aliphatic carboxylic acid is comprised of a linear or branch ed unsaturated aliphatic hydrocarbon group and at least one carboxy group, and pre ferably has 3 to 22 carbon atoms. Examples of the unsaturated aliphatic carboxyli c acid include, for example, an unsaturated aliphatic monocarboxylic acid having on e carboxy group and an unsaturated aliphatic dicarboxylic acid having two carboxy groups. The unsaturated aliphatic monocarboxylic acid is comprised of a linear or branched unsaturated aliphatic hydrocarbon group and one carboxy group, and prefe rably has 1 to 22 carbon atoms. Particularly, preferred is an unsaturated aliphatic monocarboxylic acid represented by $R^1CH=CH(CH_2)_rCOOH$ ($R^1$ represents a hydroge n atom or $CH_3(CH_2)_q$- (q is an integer of 0 to 7) and r represents an integer of 0 to 4). Although not particularly limited, specific examples thereof include, for ex ample, acrylic acid, methacrylic acid, crotonic acid, palmitoleic acid, oleic acid, vac cenic acid, linoleic acid, linolenic acid, eleostearic acid, and arachidonic acid. The unsaturated aliphatic dicarboxylic acid is comprised of a linear or branched unsatu rated aliphatic hydrocarbon group and two carboxy groups, and preferably has 1 to 4 carbon atoms. Although not particularly limited, specific examples thereof includ e, for example, maleic acid and fumaric acid.

The saturated or unsaturated alicyclic carboxylic acid is comprised of a non-aromatic saturated or unsaturated carbon ring and at least one carboxy group, and preferably has 6 to 20 carbon atoms. Particularly, a saturated alicyclic carboxylic acid having a cyclohexane ring skeleton is preferred. Examples of the saturated or unsaturated alicyclic carboxylic acid include a saturated or unsaturated alicyclic monocarboxylic acid having one carboxy group and a saturated or unsaturated alicyclic dicarboxylic acid having two carboxy groups. Although not particularly limited, specific examples of the saturated or unsaturated alicyclic monocarboxylic acid include, for example, cyclohexanecarboxylic acid. Although not particularly limited, specific examples of the saturated or unsaturated alicyclic dicarboxylic acid include, for example, cyclohexanedicarboxylic acid.

The aromatic carboxylic acid is comprised of one or multiple aromatic rings and at least one carboxylic acid, and preferably has 6 to 20 carbon atoms. Particularly, an aromatic carboxylic acid having a benzene ring skeleton is preferred. Examples of the aromatic carboxylic acid include an aromatic monocarboxylic acid having one carboxy group and an aromatic dicarboxylic acid having two carboxy groups. Although not particularly limited, specific examples of the aromatic monocarboxylic acid include, for example, benzoic acid and cinnamic acid. Although not particularly limited, examples of the aromatic dicarboxylic acid include, for example, phthalic acid, isophthalic acid and terephthalic acid.

The saturated aliphatic hydroxycarboxylic acid is comprised of a linear or b ranched saturated aliphatic hydrocarbon group, at least one carboxy group and at le ast one hydroxy group, and preferably has 2 to 24 carbon atoms. Particularly, a s aturated aliphatic hydroxycarboxylic acid having 1 to 5 hydroxy groups and 2 to 7 carbon atoms is preferred. Examples of the saturated aliphatic hydroxycarboxylic a cid include a saturated aliphatic hydroxy monocarboxylic acid having one carboxy g roup and a saturated aliphatic hydroxy di- or tricarboxylic acid having two or three carboxy groups. The saturated aliphatic hydroxy monocarboxylic acid has preferab ly 2 to 20, more preferably 2 to 7 carbon atoms. It is preferred that the number of hydroxy groups be 1 to 5. Particularly, a saturated aliphatic hydroxy monocarb oxylic acid represented by $(R^2)_3C(C(R^3)_2)_sCOOH$ is preferred (s represents an intege r of 1 to 4, and the three $R^2$'s and the 2×s $R^3$'s each independently represents a h ydrogen atom or a hydroxy group. The total number of hydroxy groups is 1 to 5). Although not particularly limited, specific examples thereof include, for example, glycolic acid, lactic acid, glyceric acid, hydroxyacetic acid, hydroxybutyric acid, 2-h ydroxydecanoic acid, 3-hydroxydecanoic acid, 12-hydroxystearic acid, dihydroxysteari c acid, cerebronic acid, leucine acid, mevalonic acid, pantoic acid, gluconic acid, g alactonic acid, mannonic acid, arabinonic acid, fructuronic acid, tagathuronic acid, a nd aldonic acid. It is preferred that the saturated aliphatic hydroxy di- or tricarbo xylic acid has 4 to 22 carbon atoms. It is preferred that the number of hydroxy g roups be 1 to 3. Particularly, a saturated hydroxy di- or tricarboxylic acid represe nted by $HOOCC(R^4R^5)C(R^6R^7)C(R^8R^9)COO^-$ is preferred ($R^4$ to $R^9$ each independe ntly represents a hydrogen atom, a hydroxy group or a carboxy group, the total nu mber of hydroxy groups is 1 to 2, and the total number of carboxy groups is 2 to 1.) Although not particularly limited, specific examples thereof include, for exampl e, tartronic acid, malic acid, tartaric acid, citramalic acid, citric acid and isocitric a cid

The unsaturated aliphatic hydroxycarboxylic acid is comprised of a linear or branched saturated aliphatic hydrocarbon group, at least one carboxy group and at least one hydroxy group, and preferably has 3 to 22 carbon atoms. Although not particularly limited, specific examples thereof include ricinolic acid, ricinoleic acid, and ricineraidic acid.

The aforementioned saturated or unsaturated alicyclic hydroxycarboxylic acid is comprised of a non-aromatic saturated or unsaturated carbon ring, at least one ca rboxy group and at least one hydroxy group, and preferably has 4 to 20 carbon at oms. Particularly, an alicyclic hydroxycarboxylic acid having a six-membered ring skeleton and 1 to 4 hydroxy groups is preferred. Although not particularly limited, specific examples thereof include, for example, hydroxycyclohexanecarboxylic acid, dihydroxycyclohexanecarboxylic acid, quinic acid (1,3,4,5-tetrahydroxycyclohexanecar boxylic acid), shikimic acid, glucuronic acid, galacturonic acid, mannuronic acid, id uronic acid and guluronic acid. Further, hydroxy group-containing cyclic lactones may also be preferably used; although not particularly limited, specific examples th ereof include, for example, ascorbic acid and erythorbic acid.

The aforementioned aromatic hydroxycarboxylic acid is comprised of one or multiple aromatic rings, at least one carboxy group and at least one hydroxy group, and preferably has 6 to 20 carbon atoms. Particularly, an aromatic carboxylic aci d having a benzene ring skeleton and 1 to 3 hydroxy groups is preferred; Although not particularly limited, specific examples thereof include, for example, salicylic a cid, hydroxybenzoic acid, dihydroxybenzoic acid, trihydroxybenzoic acid, hydroxyme thylbenzoic acid, vanillic acid, syringic acid, protocatechuic acid, gentisic acid, orse llinic acid, mandelic acid, benzylic acid, atrolactic acid, phloretic acid, coumaric ac id, umbellic acid, caffeic acid, ferulic acid and sinapic acid.

The carbonyl carboxylic acid is a carboxylic acid having a carbonyl group (s) in a molecule and having 3 to 22 carbon atoms; preferred is a carbonyl carbox ylic acid having 1 to 2 carbonyl groups and 3 to 7 carbon atoms. Particularly, pr eferred is a carbonyl carboxylic acid represented by $CH_3((CH_2)_pCO(CH_2)_q)COO^-$ (p a nd q each represent an integer of 0 to 2). Although not particularly limited, spec ific examples thereof include, for example, pyruvic acid.

The alkyl ether carboxylic acid is a carboxylic acid having an ether group (s) in a molecule, and having 2 to 22 carbon atoms, including a polyoxyalkylene a lkyl ether carboxylic acid; preferred is an alkyl carboxylic acid having 1 to 2 ether groups and 2 to 12 carbon atoms. Particularly, preferred is an alkyl ether carbox ylic acid or polyoxyethylene

alkyl ether carboxylic acid represented by $CH_3(CH_2)_rO\ (CH_2)_sCOO^-$ (r and s each represents an integer of 0 to 4). Although not particularl y limited, specific examples thereof include, for example, methoxyacetic acid, ethox yacetic acid, methoxybutyric acid and ethoxybutyric acid.

The halogen carboxylic acid is preferably a halogen carboxylic acid having 2 to 22 carbon atoms. Although not particularly limited, specific examples thereof include, for example, halogen-substituted halogen carboxylic acids such as trifluoroacetic acid, trichloroacetic acid, tribromoacetic acid, pentafluoropropionic acid, pentachloropropionic acid, pentabromopropionic acid, perfluorononanoic acid, perchlorononanoic acid and perbromononanoic acid.

Among the above-mentioned carboxylic acids, preferred are a linear or branched saturated aliphatic monocarboxylic acid, a linear or branched unsaturated aliphatic carboxylic acid, a saturated aliphatic dicarboxylic acid, an unsaturated aliphatic dicarboxylic acid, a saturated hydroxy monocarboxylic acid, a saturated hydroxy di- or tricarboxylic acid, an aromatic carboxylic acid, a hydroxy aromatic carboxylic acid, and a hydroxy group-containing cyclic lactone, among which a linear or branched saturated aliphatic monocarboxylic acid, a linear or branched unsaturated aliphatic carboxylic acid, a saturated hydroxy monocarboxylic acid, a saturated hydroxy di- or tricarboxylic acid, an aromatic carboxylic acid, and a hydroxy aromatic carboxylic acid are more preferred.

In a preferable embodiment, the component (B) is a carboxylic acid having a hydrogen-bonding functional group(s) in the hydrocarbon moiety. That is, it is preferred that the carboxylic acid contain not only one carboxy group(-COOH) but also a hydrogen-bonding functional group(s). Although not particularly limited, examples of the hydrogen-bonding functional group include, for example, an oxygen-containing group, a nitrogen-containing group, a sulfur-containing group, and a phosphorus-containing group as listed in the above. Among them, in terms of exhibiting the advantages of the formulation or composition according to the present invention, it preferably contains an oxygen-containing group, particularly preferably a hydroxy group or a carboxy group. It is preferred that the hydrogen-bonding functional group be a hydroxy group and/or a carboxy group. It is preferred that the hydrogen-bonding functional group be a hydroxy group. It is preferred that both of the hydroxy and carboxy groups are contained therein as hydrogen-bonding functional groups.

In a preferable embodiment, the component (B) is an unsaturated or branched aliphatic carboxylic acid having 8 to 22 carbon atoms.

In a preferable embodiment, the formulation according to the present invention contains an organic salt of the components (A) and (B). It is preferred that the formulation contain a cation originated from the component (A); and an anion originated from anionic residues of the component (B), wherein the cation originated from the component (A) optionally contains a cationic residue of the component (B).

The residues in the component (B) as used herein refer to atoms or atomic groups without a charge, and those having charges and of cations are referred to as cationic residues while those of anions are referred to as anionic residues.

In the present invention, the carboxylic acid or a salt thereof of the component (B) has cationic and anionic residues. The cationic residues are hydrogen atoms, or groups (atom groups) that are bonded to nitrogen atom of the component (A) to be hydrogen-bonding functional groups or organic groups. It is preferred that the acid of the component (B) be a compound composed of a hydrogen atom(s), serving as a proton, and an anionic residue(s).

The organic salt according to the present invention may be formed from an amino acid and a carboxylic acid and represented by the following formula (II):

[Chemical formula 7]

$$[R^1{}_lN^+H_mC(R^2)_2(R^3{}_nCOOX)]R^4COO^- \qquad (I\ I)$$

(In the formula, $R^1$ represents a monovalent organic group having 1 to 22 carbon atoms, $R^2$ represents a hydrogen atom or a monovalent or bivalent organic group having 1 to 22 carbon atoms, $R^3$ represents a bivalent organic group having 1 to 22 carbon atoms, $R^4$ represents a hydrogen atom or a monovalent organic group having 1 to 21 carbon atoms, 1 represents 0 to 3, m represents 0 to 3, and n represents 0 or 1, $R^1$ and $R^2$ together and optionally form a ring of 3 to 22 carbon atoms, and X represents a hydrogen atom or a monovalent cation.) It is preferred in the formula (II) that 1 be 0 or 1 and m be 2 or 3.

As for the amino and carboxylic acids forming the organic salt of the formula (II), those already listed above may be referred to. For specific details and preferred examples, all the above descriptions of components (A), (B) and formula (I) are referenced and cited as explanations of formula (II). In addition to that, preferred embodiments of the organic salts of formula (II) are listed below. It is preferred that X be a hydrogen atom. In a preferable embodiment, a ratio of the total number of primary or secondary amino groups to the number of carboxy groups in the amino acid (the total number of primary or secondary amino groups/ the number of carboxy groups) is greater than 1. In another preferable embodiment, a ratio of the total number of primary or secondary amino groups to the number of carboxy groups in the amino acid (the total number of primary or secondary amino groups/ the number of carboxy groups) is 1. In another preferable embodiment, a ratio of the total number of primary or secondary amino groups to the number of carboxy

groups in the amino acid (the total number of primary or secondary amino groups/ the number of carboxy groups) is less than 1. In a preferable embodiment, the amino acid has an isoelectric point of greater than 7. In another preferable embodiment, the amino acid has an isoelectric point of 4 or more and 7 or less. In another preferable embodiment, the amino acid has an isoelectric point of less than 4. In a preferable embodiment, $R^4$ in formula (II) is a hydrocarbon group having a hydrogen-bonding functional group(s). In this case, it is preferred that $R^4$ in formula (II) be a hydrocarbon group having a hydroxy group and/or a carboxy group. Alternatively, it is preferred that $R^4$ in formula (II) be a hydrocarbon group having a hydroxy group. Alternatively, it is preferred that $R^4$ in formula (II) be a hydrocarbon group having both of the hydroxy and carboxy groups. In another preferable embodiment, $R^4$ in formula (II) is an unsaturated or branched aliphatic hydrocarbon group having 7 to 21 carbon atoms.

The formulations of the invention may be prepared, for example, by the methods as explained below although the methods shall not particularly be limited. An amino acid and a carboxylic acid are mixed and stirred in water. Although the preparation temperature and time depend on, for example, the type of raw materials, it can be done at room temperature for about 1 hour to 1 day. The water is then distilled off under reduced pressure to obtain an intended formulation.

The formulation according to the present invention may exhibit advantages that are innate to the component (A) and/or (B). For example, although not limited to the followings, skin moisturizing and hair repairing effects can be obtained when arginine or histidine is used as the component (A); stratum corneum transparency retaining effect can be obtained when lysine is used as the component (A); improvement in skin barrier functionality and epidermal cell growth can be obtained when γ-aminobutyric acid is used as the component (A); melanin production suppressing effect can be obtained when cysteine is used as the component (A); and melanin production suppressing effect can also be obtained when linolenic acid is used as the component (B).

As for the formulation of the present invention, it may be provided that the mixture or salt of the components (A) and (B) is in an anhydrous state (anhydride) or a hydrate that has absorbed the water in the air. A hydrate refers to a compound whose moisture rate has reached a saturated state after being left in the air at 25°C and absorbing the water therein. A compound that does not absorb water after having being left in the air at 25°C is not a hydrate but an anhydride.

As for the formulation of the present invention, the mixture or salt of the components (A) and (B) may be either a liquid or a solid at 25°C in an anhydrous or hydrous state. Nevertheless, for example, when a liquid having the formulation of the present invention is sprayed or applied onto the application site and then the solvent is evaporated, the formulation remains in liquid state, which allows the formulation to exsert its functionality in an extensive area without causing any usage problems such as crystal precipitation, solidification and agglomeration. Further, if the formulation is liquid at 25°C, it may be used as a solvent or a base when the formulation is used in combination with another additive. In view of these respects, it is preferred that an anhydride and/or hydrate of the mixture or salt of the components (A) and (B) be in a liquid state at 25°C. It is more preferred that the anhydride or hydrate thereof be liquid at 25°C.

In order to allow the mixture or salt of the components (A) and (B) to be liquid at 25°C, preferable examples of the combination of components (A) and (B) include a combination in which the component (A) is an amino acid having an isoelectric point of greater than 7 and the component (B) is a saturated hydroxy monocarboxylic acid, a saturated hydroxy di-or tricarboxylic acid.

Preferable examples of the combination of the components (A) and (B), in terms of allowing the mixture or salt of the components (A) and (B) to be liquid at 25°C are listed in the followings:

**A)** Combination of the components (A) and (B) in which the component (A) is an amino acid having an isoelectric point of greater than 7 and the component (B) is a saturated hydroxy monocarboxylic acid, preferably having a multiple of hydroxy groups, which is more preferably gluconic acid.

**B)** Combination of the components (A) and (B) in which the component (A) is an amino acid having an isoelectric point of greater than 7 and the component (B) is a saturated hydroxy di- or tricarboxylic acid, preferably having at least three hydroxy groups, which is more preferably citric acid.

**C)** Combination of the components (A) and (B) in which

the component (A) is an amino acid of formula (I) where $R^2$ has an organic group, preferably having a substitute group, which is more preferably a nitrogen-containing group, even more preferably having a primary amino group, which is particularly more preferably L-lysine, and 1 is 0, m is 2, and n is 0; and
the component (B) is a saturated hydroxy monocarboxylic acid, preferably having a multiple of hydroxy groups, which is more preferably gluconic acid.

**D)** Combination of the components (A) and (B) in which

the component (A) is an amino acid of formula (I) where $R^2$ has an organic group, preferably having a substitute group, which is more preferably a nitrogen-containing group, even more preferably having a guanidino group, which is particularly more preferably L-arginine, and 1 is 0, m is 2, and n is 0; and
the component (B) is a branched aliphatic carboxylic acid.

**E)** Combination of the components (A) and (B) in which

the component (A) is an amino acid of formula (I) where $R^2$ has an organic group, preferably having a substitute group, which is more preferably a nitrogen-containing group, even more preferably having a guanidino group, which is particularly more preferably L-arginine, and 1 is 0, m is 2, and n is 0; and
the component (B) is an unsaturated aliphatic carboxylic acid.

**F)** Combination of the components (A) and (B) in which

the component (A) is an amino acid of formula (I) where $R^2$ has an organic group, preferably having a substitute group, which is more preferably a nitrogen-containing group, even more preferably having a guanidino group, which is particularly more preferably L-arginine, and 1 is 0, m is 2, and n is 0; and
the component (B) is a saturated hydroxy monocarboxylic acid or a saturated hydroxy di- or tricarboxylic acid.

**G)** Combination of the components (A) and (B) in which

the component (A) is an amino acid of formula (I) where $R^2$ has an organic group, preferably having a substitute group, which is more preferably a nitrogen-containing group, even more preferably having an imidazolyl group, which is particularly preferably L-histidine, and 1 is 0, m is 2, and n is 0; and
the component (B) is a saturated hydroxy monocarboxylic acid.

**H)** Combination of the components (A) and (B) in which

the component (A) is an amino acid of formula (I) where $R^2$ has an organic group having 1 to 22 carbon atoms, preferably 4 to 12 carbon atoms, more preferably 4 to 8 carbon atoms, which is particularly preferably γ-aminobutyric acid, and 1 is 0, m is 2, and n is 1; and

the component (B) is an unsaturated aliphatic carboxylic acid.

**I)** Combination of the components (A) and (B) in which

the component (A) is an amino acid of formula (I) where $R^2$ has an organic group having 1 to 22 carbon atoms, preferably 4 to 12 carbon atoms, more preferably 4 to 8 carbon atoms, which is particularly preferably γ-aminobutyric acid, and 1 is 0, m is 2, and n is 1; and

the component (B) is a saturated hydroxy monocarboxylic acid.

**J)** Combination of the components (A) and (B) in which

the component (A) is an amino acid of formula (I) where $R^2$ has an organic group, preferably having a substitute group, which is more preferably an oxygen-containing group, still more preferably having a hydroxy group, and even more preferably having a hydroxy group at a terminal end of the hydrocarbon group, which is most preferably L-serine, and 1 is 0, m is 2, and n is 0; and
the component (B) is preferably a saturated hydroxy monocarboxylic acid, more preferably having at least two hydroxy groups, which is still more preferably gluconic acid.

**K)** Combination of the components (A) and (B) in which

the component (A) is an amino acid of formula (I) where $R^2$ has an organic group, preferably having a substitute group, which is more preferably an oxygen-containing group, still more preferably having a hydroxy group, even more preferably having a hydroxy group at a terminal end of the hydrocarbon group, which is most preferably L-serine, and 1 is 0, m is 2, and n is 0; and
the component (B) is preferably a saturated hydroxy di- or tri-carboxylic acid, preferably having one hydroxy

group and at least two hydroxy groups, and is still more preferably malic acid or citric acid.

**L)** Combination of the components (A) and (B) in which

the component (A) is an amino acid of formula (I) where $R^1$ and $R^2$ are hydrocarbon groups, preferably forming a ring, and is more preferably L-proline, and 1 is 1, m is 1 and n is 0; and
the component (B) is a branched aliphatic carboxylic acid.

**M)** Combination of the components (A) and (B) in which

the component (A) is an amino acid of formula (I) where $R^1$ and $R^2$ are hydrocarbon groups, preferably forming a ring, and is more preferably L-proline, and 1 is 1, m is 1 and n is 0; and
the component (B) is an unsaturated aliphatic carboxylic acid.

**N)** Combination of the components (A) and (B) in which

the component (A) is an amino acid of formula (I) where $R^1$ and $R^2$ are hydrocarbon groups, preferably forming a ring, and is more preferably L-proline, and 1 is 1, m is 1 and n is 0; and
the component (B) is a saturated hydroxy monocarboxylic acid.

**O)** Combination of the components (A) and (B) in which

the component (A) is an amino acid of formula (I) where $R^1$ and $R^2$ are hydrocarbon groups, preferably forming a ring, and is more preferably L-proline, and 1 is 1, m is 1 and n is 0; and
the component (B) is preferably a saturated hydroxy di- or tri-carboxylic acid, preferably having one hydroxy group and at least two carboxy groups, and is still more preferably malic acid or citric acid.

Specifically, in order to allow the mixture or salt of the components (A) and (B) to be liquid at 25°C, preferable examples of the combination and molar ratio of the components (A) and (B) include the followings:

1) Molar ratio of components (A) and (B) is 1:1, where the component (A) is lysine and the component (B) is gluconic acid.
2) Molar ratio of the components (A) and (B) is 1:2, where the component (A) is lysine and the component (B) is gluconic acid.
3) Molar ratio of the components (A) and (B) is 1:1, where the component (A) is lysine and the component (B) is citric acid.
4) Molar ratio of the components (A) and (B) is 3:2, where the component (A) is lysine and the component (B) is citric acid.
5) Molar ratio of the components (A) and (B) is 1:1, where the component (A) is arginine and the component (B) is isostearic acid.
6) Molar ratio of the components (A) and (B) is 1:2, where the component (A) is arginine and the component (B) is isostearic acid.
7) Molar ratio of the components (A) and (B) is 1:1, where the component (A) is arginine and the component (B) is oleic acid.
8) Molar ratio of the components (A) and (B) is 1:2, where the component (A) is arginine and the component (B) is oleic acid.
9) Molar ratio of the components (A) and (B) is 1:1, where the component (A) is arginine and the component (B) is linoleic acid.
10) Molar ratio of the components (A) and (B) is 1:2, where the component (A) is arginine and the component (B) is linoleic acid.
11) Molar ratio of the components (A) and (B) is 1:1, where the component (A) is arginine and the component (B) is gluconic acid.
12) Molar ratio of the components (A) and (B) is 1:2, where the component (A) is arginine and the component (B) is gluconic acid.
13) Molar ratio of the components (A) and (B) is 1:1, where the component (A) is arginine and the component (B) is lactic acid.
14) Molar ratio of the components (A) and (B) is 1:2, where the component (A) is arginine and the component (B) is lactic acid.

15) Molar ratio of the components (A) and (B) is 1:1, where the component (A) is arginine and the component (B) is malic acid.
16) Molar ratio of the components (A) and (B) is 1:2, where the component (A) is arginine and the component (B) is malic acid.
17) Molar ratio of the components (A) and (B) is 1:1, where the component (A) is arginine and the component (B) is citric acid.
18) Molar ratio of the components (A) and (B) is 3:2, where the component (A) is arginine and the component (B) is citric acid.
19) Molar ratio of the components (A) and (B) is 1:1, where the component (A) is arginine and the component (B) is tartaric acid.
20) Molar ratio of the components (A) and (B) is 1:2, where the component (A) is arginine and the component (B) is tartaric acid.
21) Molar ratio of the components (A) and (B) is 1:1, where the component (A) is arginine and the component (B) is benzoic acid.
22) Molar ratio of the components (A) and (B) is 1:1, where the component (A) is histidine and the component (B) is gluconic acid.
23) Molar ratio of the components (A) and (B) is 1:2, where the component (A) is histidine and the component (B) is gluconic acid.
24) Molar ratio of the components (A) and (B) is 1:2, where the component (A) is histidine and the component (B) is lactic acid.
25) Molar ratio of the components (A) and (B) is 1:1, where the component (A) is histidine and the component (B) is citric acid.
26) Molar ratio of the components (A) and (B) is 3:2, where the component (A) is histidine and the component (B) is citric acid.
27) Molar ratio of the components (A) and (B) is 1:1, where the component (A) is glycine and the component (B) is citric acid.
28) Molar ratio of the components (A) and (B) is 1:1, where the component (A) is γ-aminobutyric acid and the component (B) is oleic acid.
29) Molar ratio of the components (A) and (B) is 1:1, where the component (A) is γ-aminobutyric acid and the component (B) is gluconic acid.
30) Molar ratio of the components (A) and (B) is 1:1, where the component (A) is γ-aminobutyric acid and the component (B) is lactic acid.
31) Molar ratio of the components (A) and (B) is 1:1, where the component (A) is γ-aminobutyric acid and the component (B) is citric acid.
32) Molar ratio of the components (A) and (B) is 1:1, where the component (A) is serine and the component (B) is gluconic acid.
33) Molar ratio of the components (A) and (B) is 1:1, where the component (A) is serine and the component (B) is malic acid.
34) Molar ratio of the components (A) and (B) is 1:1, where the component (A) is serine and the component (B) is citric acid.
35) Molar ratio of the components (A) and (B) is 1:1, where the component (A) is proline and the component (B) is isostearic acid.
36) Molar ratio of the components (A) and (B) is 1:1, where the component (A) is proline and the component (B) is oleic acid.
37) Molar ratio of the components (A) and (B) is 1:1, where the component (A) is proline and the component (B) is linoleic acid.
38) Molar ratio of the components (A) and (B) is 1:1, where the component (A) is proline and the component (B) is gluconic acid.
39) Molar ratio of the components (A) and (B) is 1:1, where the component (A) is proline and the component (B) is lactic acid.
40) Molar ratio of the components (A) and (B) is 1:1, where the component (A) is proline and the component (B) is malic acid.
41) Molar ratio of the components (A) and (B) is 1:1, where the component (A) is proline and the component (B) is citric acid.

The formulation according to the present invention contains the components (A) and (B) wherein the amino acid of the component (A) has a hydrogen-bonding functional group(s)(a carboxy group, a hydrogen atom bonded to nitrogen atom), which allows it to improve the affinity to water and make it excellent in water retentivity and moisture

absorbency.

In terms of water retentivity, it is preferred that the component (A) be an amino acid having an isoelectric point of greater than 7 and the component (B) be a carboxylic acid having a hydrogen-bonding functional group(s) in the hydrocarbon moiety. It is more preferred that the component (A) be an amino acid having an isoelectric point of greater than 7 and the component (B) be a hydroxycarboxylic acid. It is even more preferred that the component (A) be an amino acid having an isoelectric point of greater than 7 and the component (B) be a hydroxy tricarboxylic acid. Among them, it is particularly preferable for the combinations of the components (A) and (B) to be the above-listed combinations of 3, 4, 17, 18, 25 to 27, 31, 34 and 41.

In terms of moisture absorbency, it is preferred that the component (A) be an amino acid having an isoelectric point of greater than 7 and the component (B) be an unsaturated carboxylic acid. It is more preferred that the component (A) be L-arginine and the component (B) be oleic acid. Among them, it is even more preferred that the combinations of the components (A) and (B) be the combinations of 7 and 8 in the above-mentioned list.

As for the formulation according to the present invention, the components (A) and (B) are respectively an amino acid and a carboxylic acid, which therefore makes it excellent in solubility of a hardly soluble substance. The formulation therefore allows a hardly soluble substance to be compounded at a higher concentration, on top of which the hardly soluble substance may persistently remain in a dissolved form to be uniformly coated on the surface of a target even if the hardly soluble substance is in a solid form, which therefore makes it fully demonstrate the effects of the hardly-soluble substance.

The formulation according to the present invention has the components (A) and (B), which renders the formulation excellent, when applied to cosmetics, in all aspects of feeling of use for the spreadability thereof when applied to the skin, a moisture retention feeling and a non-stickiness; and is particularly superior in a persistent moisturizer feeling due to the non-volatility of the mixture or organic salt of the components (A) and (B) used in the present invention.

Especially, a mixture, organic salt or the solution of the preceding of the components (A) and (B) having a melting point of lower than 25°C is superior in feeling of use, such as the spreadability, moisture retention feeling, non-stickiness and refreshing feeling.

It is preferred in terms of sensory evaluation that the component (A) be an amino acid having an isoelectric point of greater than 7 or an amino acid having an isoelectric point of 4 or more and 7 or less, and the component (B) have a hydrogen-bonding functional group(s) in the hydrocarbon moiety, said hydrogen-bonding functional group preferably having a hydroxy group and/or a carboxy group, more preferably having at least two carboxy groups.

Moreover, it is preferred that the component (A) be an amino acid having an isoelectric point of greater than 7 or an amino acid having an isoelectric point of 4 or more and 7 or less, and the component (B) be an unsaturated or branched aliphatic carboxylic acid; it is more preferred that the component (A) be arginine or proline and the component (B) be an unsaturated or branched aliphatic carboxylic acid. It is preferred in terms of feeling of use that the compounding molar ratio of the components (A) and (B) be 1:9 to 9:1, more preferably 3:1 to 1:3, and even more preferably 3:2 to 1:2.

Among them, it is preferred that the combinations of the components (A) and (B) be the above-listed combinations of 3 to 10, 15 to 20, 25 to 27, 31, 33 to 37 among which the combinations of 6, 8, 25, 26, 31, 33 to 37, 40, 41 are more preferred.

When a target surface to which the formulation of the present invention is applied contains a functional group that interacts or couples to a hydrogen-bonding functional group in the formulation of the present invention, such as an oxygen-containing group, a nitrogen-containing group, a sulfur-containing group and a phosphorus-containing group, the hydrogen-bonding functional group contained in the components (A) and/or (B) allows it to be favorably attached to the target for a long period of time, which therefore enables it to demonstrate the effects thereof. Examples of such target include organic or inorganic objects such as, although not particularly limited to the followings, living tissues (hair or skin), resins, papers, metals and metal oxides.

It is preferred in terms of adherability to the hair or skin that the compone nt (B) be a carboxylic acid having a hydrogen-bonding functional group(s) in the h ydrocarbon moiety; the hydrogen-bonding functional group(s) preferably having a hy droxy group and/or a carboxy group, more preferably having a hydroxy group, parti cularly preferably having both of the hydroxy and carboxy groups, and most prefer ably having a hydroxy group and at least two carboxy groups.

Moreover, it is preferred that the component (A) be an amino acid having a n isoelectric point of greater than 7 or be an amino acid having an isoelectric poin t of 4 or more and 7 or less, and the component (B) be an unsaturated or branche d aliphatic carboxylic acid.

Among them, it is preferred that the combinations of the components (A) and (B) be the above-listed combinations of 1 to 4, 11, 12, 17, 18, 25 to 27, 31, 32, 34, 38 and 41.

Thus, the formulation according to the present invention is superior in antibiotic properties due to the carboxy group in the component(s) (A) and/or (B). As for the total number of amino groups and the number of carboxy groups in the components (A) and (B), it is preferable for the combination to have a greater number of carboxy groups, and when the components (A) and (B) form an organic salt, it is preferred that un-neutralized carboxy groups(-COOH) remain therein. That is, it is preferable for a ratio of the number of the amino groups to the number of the carboxy groups (the

number of amino groups/the number of carboxy groups) in the components (A) and (B) to be 1 or less.

As for the formulation according to the present invention, the mixture or organic salt of the components (A) and (B) is non-volatile, and the liquid mixture or salt thereof in particular is capable of being coated in a uniform and highly concentrated manner after the volatile components are evaporated, which therefore provides an effective and persistent antimicrobial property. Moreover, the formulation is also effective as a solvent for an agent effective on bacterium and/or viruses, such as existing antibacterial agents and/or antivirals, which not only allows the existing antibacterial agents and/or antivirals to be compounded therein at high concentration but also allows it to be persistently remained in a dissolved form and uniformly coated on the surface of a target even if the hardly soluble substance is in a solid form, which therefore makes it fully demonstrate the effect of, for example, the existing antibacterial agents, antivirals and/or disinfectants in addition to the antimicrobial property of the formulation according to present invention. Further, the formulation of the present invention may also offer the prospect of synergistic effects with the existing antibacterial agents, antivirals and/or disinfectants, which enables it to have, for example, antibacterial and/or antiviral properties at a lower concentration.

It is preferred in terms of antimicrobial property that the component (A) be an amino acid having an isoelectric point of 4 or more and 7 or less, and the component (B) have a hydrogen-bonding functional group(s) in the hydrocarbon moiety. It is preferable for the hydrogen-bonding functional group(s) to have a hydroxy group and/or a carboxy group, more prefarablly to have both of the hydroxy and carboxy groups, and particularly preferably to have a hydroxy group and at least two carboxy groups. Moreover, as for the combinations of the components (A) and (B), it is preferred that a ratio of the number of the amino groups to the number of the carboxy groups in the components (A) and (B) (the number of amino groups/the number of carboxy groups) be 1 or less. Among them, it is preferred that the combinations of the components (A) and (B) be the above-listed combinations of 3, 16, 17, 20, 25, 27, 31, 33, 34, 40 and 41.

The formulation according to the present invention is favorable in terms of safety, and particularly the formulation having the components (A) and (B) that are listed in Japanese standards of quasi-drug ingredients (JSQI), Japanese standards of quasi-drug additives, Japanese pharmacopoeia (JP), Japanese pharmaceutical codex (JPC), Japanese pharmaceutical excipients (JPE), Japanese standards of quasi-drug additives, and Japan's specifications and standards for food additives (JSFA) will be superior in terms of safety and have a reduced skin irritancy, which therefore enables the applications for the cosmetics or daily goods.

As for the formulation according to the present invention, the components (A) and (B) are respectively an amino acid and a carboxylic acid, which therefore allows it to be readily degraded in terms of biodegradability. The formulation containing components (A) and (B) of natural origin, in particular, is useful because it is excellent in biodegradability and has a low impact on the environment.

As to the combination with water or polymers, the formulation of the present invention has a hydrogen-bonding functional group(s) in both or either one of the components (A) and (B), which therefore allows it to have a favorable affinity with water or polymers, thereby making is suitable for forming a gel or for enhancing viscosity. The hydrogen-bonding functional group(s) further facilitates polymers to form a gel, which therefore makes it favorable in terms of enhancing viscosity.

The gel composition containing the formulation of the present invention, a polymer and water is useful in terms of exhibiting thixotropy-the property of becoming less viscous when subjected to a shearing stress, thus enabling it to be readily applied to a target, while this viscosity increases when not subjected to a shearing stress, which therefore cause the liquid less likely to be trickled from the application surface.

The formulation of the present invention may be the one containing only of the components (A) and (B), or be a solution of the components (A) and (B) diluted with a solvent, or a mixture with or a composition of a further component.

As for the formulation according to the present invention, the compounding molar ratio of the components (A) and (B) may be, although not particularly limited to the followings, 1:99 to 99:1, preferably 1:9 to 9:1, and more preferably 1:5 to 5:1.

The amount of the components (A) and (B) contained in the formulation according to the present invention may be, although not limited to the followings, for example, 0.01 to 100wt%, 0.1 to 100wt%, or 1 to 95wt% based on the total amount of the formulation.

Examples of the solvent include, but are not particularly limited to, water, methanol, ethanol, propanol, isopropanol, butanol, ethylene glycol, propylene glycol, 1,3-butylene glycol, diethylene glycol, dipropylene glycol, isoprene glycol, hexylene glycol, glycerin, benzyl alcohol, methyl acetate, ethyl acetate, isopropyl acetate, ethyl ether, acetone, toluene, hexane, heptane and acetonitrile; they may be used alone or in combination with two or more species thereof.

Examples of the further component include, but are not particularly limited to, water, surfactants (such as an anionic surfactant, a cationic surfactant, a nonionic surfactant, an amphoteric surfactant), an oil agent, a solvent, an oil agent, a cationic polymer, a water-soluble polymer, a viscosity regulator, a resin, a resin particle, a gloss imparting agent, a higher alcohol, a multivalent alcohol, a higher fatty acid, amidoamines, a hydrocarbon, a wax, esters, a silicone derivative, a physiologically active ingredient, extracts, an antioxidant, a sequestrant, a preservative, an ultraviolet absorber (such as organic or inorganic ones), a perfume, a moisturizer, carbons, metal oxides, minerals, salts, a neutralizer,

a pH adjuster, a refrigerant, an insect repellent, an enzyme, dye compound, an organic colorant, a inorganic colorant, a colorant, a coloring agent, a pearling agent, a pearlizing agent, an anti-inflammatory agent, an antioxidant, a corrosion inhibitor, a rust inhibitor, a metal deactivator, an antifoaming agent, a whitening agent, a wrinkle ameliorating agent, vitamins, amino acids, a hair growing agent, an antibacterial agent, a hormonal agent, a plant extract ingredient, a seaweed extract ingredient, a crude drug ingredient, an activator, a blood circulation promoter, and organic modified clay minerals; they may be used alone or in combination with two or more species thereof.

The formulation of the present invention may be used for imparting water retentivity, moisture absorbency and/or antimicrobial property, as well as for cosmetics. The formulation of the present invention may also be used for a gel composition containing a polymer and water. The formulation of the present invention is excellent in feeling of use (such as the spreadability, moisture retention feeling, non-stickiness and refreshing feeling), adherability to the hair, antimicrobial property, skin irritancy, biodegradability and gel-forming property, which therefore allows the formulation to be suitably used for various applications such as, for example, a water/moisture retention agent, a moisture absorbent, a conductive material, a electrolyte material, a antistatic agent, a solvents for dissolving biomaterial, a dispersion solvent, a preservation solvents and culture media, thickening agents, dissolving or dispersing solvents of organic or inorganic material (including, but not particularly limited to a metal, a metal oxide (including, but not particularly limited to, silica, aluminum oxide (alumina), zirconia, titanium oxide, magnesium oxide, indium tin oxide (ITO), cobalt blue ($CoO-Al_2O_3$), antimony oxide, zinc oxide, cesium oxide, zirconium oxide, yttrium oxide, tungsten oxide, vanadium oxide, cadmium oxide, tantalum oxide, niobium oxide, tin oxide, bismuth oxide, cerium oxide, copper oxide, iron oxide, indium oxide, boron oxide, calcium oxide, barium oxide, thorium oxide, indium tin oxide and ferrite), or carbon material), a surface treatment agent, a beauty product (emulsion, lotion, lipstick, etc.), a cosmetic (fabric softener, detergent, etc.), a sanitary/hygiene product, a pharmaceutical product, a topical product, a transdermal absorbent, a soil conditioner, a soil water retention agent, a soil improvement agent, a waterstop, a road-bedding material, a concrete treatment agent, a water retention agent for greening a material for civil engineering and construction (flame retardants, heat insulators, etc.), a food packaging material, a food additive (freshness preservative, etc.), a sheet, a concrete admixture/modifier, a sealant, a waterproofing agent, a shock absorber, an adsorbent, an extractant, a medical/pharmaceutical material (medical sensor, oral cleanser, etc.), a drug delivery system, a lubricant, a reaction solvent, a heat transfer medium, a refrigerant, a food, an agricultural material (seed coating agent, etc.), an agrichemical, a fertilizer, a pesticide pigment, a dye, a paint, a dye, an ink pigment treatment, an adhesive, a swelling agent, a wetting agent, an antibacterial agent, a paper treatment agent (lotion tissue agent, paper coating liquid, etc.),a textile material (fiber treatment agent, etc.), a flower delivery agent, an electronic material (electronic material cleaning agent, etc.).a biomaterial (artificial skin, etc.),a sanitary material, a deodorant, an air freshener base material, an exothermic material (warmer, etc.),a cold storage agent, a cooling sheet, a pet supply (absorbent sheet),a material for daily necessity (sleepwear, bedding, etc.),a glass material (glass treatment agent (anti-condensation agent), etc.) and a material for industrial product (gas detecting agent, desiccant, adhesive, resin modifier, etc.).

**(Composition)**

The composition of the present invention contains a formulation as explained in the above. The formulation of the present invention may contain an organic salt as explained in the above. These formulations or compositions may be used for imparting water retentivity, moisture absorbency and/or antimicrobial property, as well as for cosmetics. The formulation of the present invention may also be used as a gel composition containing a polymer and water. The composition containing the formulation according to the present invention may be, for example, in a form of, although not particularly limited to the followings, liquid, solid or gel.

As for the preferable combinations of the components (A) and (B) in the composition containing the formulation according to the present invention, the combinations as listed in the above may be referred to depending on their intended applications.

Examples of the further component other than the components (A) and (B) in the composition, having the formulation of the present invention, to be used for imparting antimicrobial property include, but are not particularly limited to, for example, the aforementioned solvents, an agent effective on bacteria or viruses such as an antibacterial agent, an antiviral and/or disinfectant, a surfactant (such as an anionic surfactant, a cationic surfactant, a nonionic surfactant, an amphoteric surfactant), a resin, a UV-absorber (including an organic or inorganic absorber), a perfume, a moisturizer, a metal oxide, a neutralizer, a pH adjuster, a colorant, an antioxidant, a corrosion inhibitor, a rust inhibitor, a metal deactivator and a antifoaming agent; they may be used alone or in combination with two or more species thereof.

Examples of the further component other than the components (A) and (B) in the composition, having the formulation of the present invention, to be used as cosmetics include, but are not particularly limited to, for example, water, a surfactant, an oil agent, a solvent, a cationic polymer, a water-soluble polymer, a viscosity regulator, a resin, resin particles, a gloss imparting agent, a higher alcohol, a multivalent alcohol, a higher fatty acid, amidoamines, a hydrocarbon, a wax, esters, a silicone derivative, a physiologically active ingredient, an extract, an antioxidant, a sequestrant, a preservative, an ultraviolet absorber (organic or inorganic), a perfume, a moisturizer, carbons, metal oxides, minerals, salts, a neutralizer, a pH adjuster, a refrigerant, an insect repellent, an enzyme, dye compound, an organic colorant, an inorganic colorant, a coloring agent, a pearling agent, a pearlizing agent, an anti-inflammatory agent, an antioxidant, a whitening agent, a wrinkle ameliorating agent, vitamins, amino acids, a hair growing agent, an antibacterial agent, a hormonal agent, a plant extract ingredient, a seaweed extract ingredient, a crude drug ingredient, an activator, a blood circulation promoter, and an organic modified clay mineral.

When applied to the skin, the composition of the present invention is capable of imparting a moisture retention feeling, and thus achieving a favorable feeling with no stickiness, due to the effects of the short- or long-term water and moisture retention property and antistatic property (static protection property) owing to the non-volatility of the mixture or organic salt of the components (A) and (B). Further, due to the safety of the formulation and an affinity, permeability, and low-irritating property thereof to the skin or the like, even as a skin care composition, there can be obtained a skin-care composition with a high safety, a favorable skin compatibility, a favorable skin elasticity and being low-irritating to the skin or the like. Furthermore, since the aforementioned formulation is useful as a base material for a skin care composition even in that there can be achieved a high solubility of the active ingredients, and since the formulation is superior in permeability into the skin or the like, the formulation can bring an excellent water /moisture retention effect to the skin or the like, and can thus also be used as a carrier of the active ingredients. Other than skin application, the formulation may also be used in cuticles, nails, and inner regions of oral and nasal cavities where the effects of the present invention are sought. Moreover, the formulation is superior in terms of biodegradability, and therefore may be regarded as a formulation having a low impact on the environment.

The gel composition containing the formulation of the present invention may contain not only the formulation of the components (A) and (B) but also a polymer and water. Examples of the polymer compound include, but are not particularly limited to, a synthetic polymer compound, a semisynthetic polymer compound and a natural polymer compound, among which a polymer having a hydrogen-bonding functional group(s), particularly a polymer compound having at least any one selected from a hydroxy group, a carbonyl group, a carboxy group and a carboxylate group, is preferred because a hydrogen-bonding functional group(s) contained in a polymer compound allows the compound to have an interaction with water, the component (A) and/or the component (B) which in turn enhances the affinity to them. Particularly, in terms of safety, preferred are a semisynthetic polymer compound and a natural polymer compound, of which a natural polymer compound is more preferred. They may be used alone or in combination with two or more species.

Examples of the synthetic polymer include, but are not particularly limited to, polymers based on, for example, polyacrylic acid, vinyl acetate copolymer, maleic anhydride copolymer, polyvinyl alcohol, polyvinylpyrrolidone, polyethylene glycol, polyethylene oxide, polyester, polyacrylonitrile, polyamide, polyimide, polyamideimide, polymaleimide, polyurethane, polycarbonate and polyacrylate.

Examples of the semisynthetic polymer include, but are not particularly limited to, for example, cellulose derivatives (carboxymethylcellulose sodium, hydroxyethylcellulose, methylcellulose, ethylcellulose, nitrocellulose, hydroxypropylcellulose, hydroxypropylmethylcellulose and crystalline cellulose), alginate sodium, an ester gum, and a soluble starch. Examples of the natural polymer include, but are not particularly limited to, for example, a polysaccharide, a cellulose, a nucleic acid or the salt thereof, a ribonucleic acid or the salt thereof, a hydrosoluble protein (such as casein, collagen, gelatin, albumin, fibroin, elastin, keratin and sericin), hyaluronic acid or the salt thereof, and a mucoperiosteum (such as chondroitin sulfate), among which a polysaccharide is preferred.

Examples of the polysaccharide include, but are not particularly limited to, natural polysaccharides such as xanthan gum, carrageenan, tamarind seed gum, gellan gum, guar gum, pectin, gum arabic, karaya gum, locust bean gum, diutan gum, sodium alginate, agarose, hyaluronic acid, polygalacturonic acid; carboxyalkyl polysaccharides such as carboxymethyl pullulan, carboxymethyl chitin, carboxymethyl chitosan, carboxymethyl mannan, carboxymethyl starch, carboxymethyl dextran, carboxyethyl cellulose, carboxymethyl pullulan; oxidized polysaccharides such as oxidized cellulose and oxidized starch; and polysaccharides having sulfate groups such as chondroitin sulfate, dermatan sulfate, heparin and heparan sulfate.

Among them, preferred is a polymer compound having a hydrogen-bonding functional group(s), and more preferred is a hydrosoluble polymer compound. As such polymer compound, preferred are xanthan gum, carrageenan, gellan gum, guar gum, diutan gum and sodium alginate, among which xanthan gum, carrageenan, gellan gum, guar gum, diutan gum are more preferred, and xanthan gum and guar gum are even more preferred.

The component (A), the component (B), polymer compound and water in the gel composition of the present invention may be compounded in any ratio, and the compounding amount thereof is not particularly limited. Nevertheless, in terms of forming a gel or imparting an enhanced viscosity, it is preferred that the total sum of the components (A) and (B) in the composition be 90wt% or less, more preferably 50wt% or less, even more preferably 30wt% or less, and particularly more preferably 10wt% or less. Meanwhile, it is preferable for the polymer to be contained in the composition in an amount of 0.01 wt % or more, more preferably 0.1wt % or more, and even more preferably 1wt % or more.

The embodiments of the present invention have been described above. Nevertheless, the invention shall not be limited to these embodiments, and various modifications may be made within the scope that does not depart from

the gist of the invention. Further, for each of the above preferred examples shown in formula (I), the combination of them with each of the above preferred examples shown in the component (B), and each of the above preferred examples shown in Formula (II), at least one of the combinations of them may be preferred embodiments based on the results of the following working examples. Furthermore, the combination of them with the properties of mixtures of the components (A) and (B) or salts of the components (A) and (B), or even with their respective preferred examples of polymeric compounds, may be more preferred embodiments based on the results of the working examples.

WORKING EXAMPLES

The present invention will be described in more detail below with reference to examples, but the present invention is not limited to these examples.

**1. Preparation of Formulations (Organic** Salts) **1-427**

Formulations (salts) 1-427 as shown in Tables 1A-1D were prepared in a manner to be explained below. The reagents used for the components (A) and (B) are as listed hereinbelow:

L-lysine, γ-aminobutyric acid, hexanoic acid, linoleic acid, fumaric acid, citric acid, benzoic acid, and ascorbic acid manufactured by Tokyo Chemical Industry Co., Ltd;
L-arginine, L-histidine, glycine, L-valine, L-asparagine, L-glutamine, L-cysteine, L-tryptophan, L-aspartic acid, formic acid, acetic acid, propionic acid, gluconic acid, L-malic acid, Tartaric acid and cinnamic acid manufactured by Fujifilm Wako Pure Chemical Corporation;
L-alanine, L-leucine, L-isoleucine, L-serine, L-threonine, L-methionine, L-phenylalanine, and L-glutamic acid manufactured by Peptide Institute. INC;
L-proline made by Sigma-Aldrich Japan;
Isostearic acid and oleic acid manufactured by Miyoshi Oil & Fat Co., Ltd;
Lactic acid, butyric acid, adipic acid, and succinic acid manufactured by Kanto Kagaku Co., Ltd.

**<Working example 48> Formulation** (salt) **48**

L-arginine (11.78 g, 0.10 mol) and isostearic acid (28.45 g, 0.10 mol) in 50 mL of water and 50 mL of ethanol were stirred at room temperature for 3 hours, and then the solvents were evaporated under reduced pressure to obtain a colorless liquid. The obtained liquid was cleaned to obtain a formulation (salt) of colorless liquid of L-arginine and isostearic acid.

**Formulation (salt) 48**

FT-IR (KBr): 3165 $cm^{-1}$, 2925 $cm^{-1}$, 1854 $cm^{-1}$, 1653 $cm^{-1}$, 1406 $cm^{-1}$.

$^1$HNMR (MeOD, 400MHz): 0.86-0.94 (m, 6H, Isostearic acid, $-CH_3$), 1.69-1.79 (m, 2H, Arg, $-CH_2\underline{CH_2}CH_2-$), 1.87-1.94 (m, 2H, Arg, $-\underline{CH_2}CH(NH_2)COOH$), 3.11-3.14 (t, 2H, Arg, - $\underline{CH_2}NH-$), 3.32-3.40 (m, 1H, Isostearic acid, $-\underline{CH}(COOH)-$), 3.58-3.61 (t, 1H, Arg, - $\underline{CH}(NH_2)COOH$).

1.31 (m, 22H), 1.59-1.63 (m, 2H), 2.17-2.21 (t, 2H), 3.21-3.25 (t, 2H): (Isostearic acid, $CH_3(CH_2)_8CH((CH_2)_6CH_3)COOH$).

$^{13}$CNMR (MeOD, 100MHz): 25.8 (Arg, $-CH_2\underline{C}H_2CH_2-$), 27.6 (Arg, $-\underline{C}H_2CH(NH_2)COOH$), 41.9 (Arg, $-\underline{C}H_2NH-$), 55.6 (Arg, $-\underline{C}H(NH_2)COOH$), 56.2 (Isostearic acid, $-\underline{C}H(COOH)-$), 158.8 (Arg, $-\underline{C}(NH)NH_2$), 174.7 (Arg, $CO\underline{O}H$), 182.6 (Isostearic acid, COOH).

29.5, 30.8, 38.8, 48.4, 48.6, 48.8, 49.0, 49.2, 49.5 (Isostearic acid, $\underline{C}H_3(\underline{C}H_2)_8CH((\underline{C}H_2)_6\underline{C}H_3)COOH$).

**<Working examples 1-6, 22-27, 43-48, 64-69, 85-90, 106-111** > **Formulations (salts) 1-6, 22-27, 43-48, 64-69, 85-90, 106-111**

Similarly, the components (A) and (B) were used in the compounding ratio as listed in Table 1A and prepared under the same condition as in Working example 48 to obtain formulations (salts) 1 to 6, 22 to 27, 43 to 48, 64-69, 85-90, 106-111. Spectroscopy data for representative formulations (salts) are listed below.

**Formulation (salt) 69**

FT-IR (KBr): 2925 $cm^{-1}$, 2854 $cm^{-1}$, 1677 $cm^{-1}$, 1542 $cm^{-1}$, 1464 $cm^{-1}$, 1405 $cm^{-1}$.

$^1$HNMR (MeOD, 400MHz): 0.86-0.94 (m, 6H, Isostearic acid, -$CH_3$), 1.69-1.79 (m, 2H, Arg, -$CH_2CH_2CH_2$-), 1.87-1.94 (m, 2H, Arg, -$CH_2CH(NH_2)COOH$), 3.11-3.14 (t, 2H, Arg, - $CH_2$NH-), 3.32-3.40 (m, 1H, Isostearic acid, -CH(COOH)-), 3.58-3.61 (t, 1H, Arg, - $CH(NH_2)COOH$).

1.31 (m, 22H), 1.59-1.63 (m, 2H), 2.17-2.21 (t, 2H), 3.21-3.25 (t, 2H): (Isostearic acid, $CH_3(CH_2)_8CH((CH_2)_6CH_3)COOH$).

$^{13}$CNMR (MeOD, 100MHz): 27.0 (Arg, -$CH_2CH_2CH_2$-), 30.6 (Arg, -$CH_2CH(NH_2)COOH$), 41.8(Arg, -$CH_2$NH-) , 49.7 (Arg, -$CH(NH_2)COOH$), 158.8 (Arg, -$C(NH)NH_2$), 174.3 (Arg, COOH), 180.5 (Isostearic acid, COOH).

29.5, 30.8, 38.8, 48.4, 48.6, 48.8, 49.0, 49.2, 49.5 (Isostearic acid, $CH_3(CH_2)_8CH((CH_2)_6CH_3)COOH$).

**<Working example 49> Formulation (salt) 49**

L-arginine (11.78 g, 0.10 mol) and oleic acid (28.45 g, 0.10 mol) were stirred in 50 mL of water and 50 mL of ethanol at room temperature for 3 hours, and then the solvents were evaporated under reduced pressure to obtain a yellow liquid. The obtained liquid was cleaned to obtain a formulation (salt) of yellow liquid of L-arginine and oleic acid.

**Formulation (salt) 49**

FT-IR (KBr): 3347 $cm^{-1}$, 2925 $cm^{-1}$, 1737 $cm^{-1}$, 1636 $cm^{-1}$, 1404 $cm^{-1}$.

$^1$HNMR (MeOD, 400MHz): 0.90-0.94 (t, 3H, Oleic acid, -$CH_3$), 1.71-1.78 (m, 2H, Arg, - $CH_2CH_2CH_2$-), 1.88-1.94 (m, 2H, Arg, -$CH_2CH(NH_2)COOH$), 3.21-3.25 ((t, 2H, Oleic Acid, -$CH_2$COOH), (t, 2H, Arg, -$CH_2$NH-)), 3.59-3.62 (t, 1H, Arg, -$CH(NH_2)COOH$), 5.35-5.37 (t, 2H, Oleic acid, -CH=CH-).

1.31-1.35 (m, 20H), 1.59-1.62 (m, 2H), 2.01-2.05 (m, 2H), 2.17-2.20 (t, 2H): (Oleic acid, $CH_3(CH_2)_7CH=CH(CH_2)_7COOH$).

$^{13}$CNMR (MeOD, 100MHz): 14.5 (Oleic acid $CH_3$-), 23.8, (Arg, -$CH_2CH_2CH_2$-), 25.7 (Oleic acid), 27.7 (Arg, -$CH_2CH(NH_2)COOH$), 41.9 (-$CH_2$NH-), 55.5 (Arg, -$CH(NH_2)COOH$), 130.8, 130.9 (Oleic acid, -CH=CH-), 158.9 (Arg, -$C(NH)NH_2$), 174.9 (Arg, COOH), 182.9 (Oleic acid, COOH).

25.7, 28.1, 28.2, 29.5, 30.4, 30.47, 30.57, 30.64, 30.8, 30.9, 32.7, 33.1, 39.1 (Oleic acid, CH3 ( CH2 )7CH =CH( CH2 ) 7COOH ) .

**<Working examples 7, 8, 28, 29, 49, 50, 70, 71, 91, 92, 112, 113> Formulation (salt) 7, 8, 28, 29, 49, 50, 70, 71, 91, 92, 112, 113**

Similarly, the components (A) and (B) were used in the compounding ratio as listed in Table 1A and prepared under the same condition as in Working example 49 to obtain formulations (salts) 7, 8, 28, 29, 49, 50, 70, 71, 91, 92, 113 and 114. Spectroscopy data for representative formulations (salts) are listed below.

**Formulation (salt) 50**

FT-IR (KBr): 3346 $cm^{-1}$, 3166 $cm^{-1}$, 2926 $cm^{-1}$, 1645 $cm^{-1}$, 1404 $cm^{-1}$. 1 HNMR (MeOD, 400MHz): 0.90-0.95 (m, 3H, Linoleic acid, -CH3 ) , 1.68-1.80 ( m , 2H, Arg, -CH2CH2CH2- ), 1.84-1.93 ( m , 2H, Arg, -CH2CH ( NH2 )COOH) , 3.11-3.14 (t, 2H, Arg, -CH2NH- ), 3.57-3.60 (t, 1H, Arg, -CH ( NH 2 )COOH), 5.30-5.42 (m, 4H, Linoleic acid, -C H =C H -).

1.30-1.41 (m, 12H), 1.59-1.64 (m, 2H), 2.05-2.11 (q, 4H), 2.78-2.81 (t, 2H), 3.21-3.25 (t, 2H), 3.31-3.34(t, 2H): (Linoleic acid, $CH_3(CH_2)_4(CH=CHCH_2)_2(CH_2)_6COOH$).

$^{13}$CNMR (MeOD, 100MHz): 14.7 (Linoleic acid, -$CH_3$), 23.7 (Arg, -$CH_2CH_2CH_2$-), 27.7 (Arg, -$CH_2CH(NH_2)COOH$), 41.9 (Arg, -$CH_2$NH-), 55.6 (Arg, -$CH(NH_2)COOH$), 56.3 (Linoleic acid, -$CH_2$COOH), 129.7 (Linoleic acid, -CH=CH-), 130.9 (Linoleic acid, - CH=CH-), 158.8 (Arg, -$C(NH)NH_2$), 174.8 (Arg, COOH), 182.7 (Linoleic acid, COOH). 26.6, 28.2, 30.5, 30.8, 38.9, 48.6, 48.8, 49.0, 49.2, 49.5, 49.7 (Linoleic acid, $CH_3(CH_2)_4(CH=CHCH_2)_2(CH_2)_6COOH$).

**Formulation (salt) 70**

FT-IR (KBr): 3411 $cm^{-1}$, 2924 $cm^{-1}$, 1762 $cm^{-1}$, 1635 $cm^{-1}$, 1063 $cm^{-1}$, 963 $cm^{-1}$.

$^1$HNMR (MeOD, 400MHz): 0.80-0.89 (t, 6H, Oleic acid, -$CH_3$), 1.60-1.70 (m, 2H, Arg, - $CH_2CH_2CH_2$-), 1.78-1.84 (m, 2H, Arg, -$CH_2CH(NH_2)COOH$), 3.11-3.14 (t, 2H, Arg, - $CH_2$NH-), 3.50-3.55 (m, 1H, Arg, -$CH(NH_2)COOH$), 5.25-5.27 (t, 4H, Oleic acid,-CH=CH-). 1.21-1.24 (m, 40H) 1.49-1.53 (m, 4H), 1.92-1.99 (m, 8H), 2.12-2.15 (t, 4H): (Oleic acid, $CH_3(CH_2)_7CH=CH(CH_2)_7COOH$).

$^{13}$CNMR (MeOD, 100MHz): 14.5 (Oleic acid -$CH_3$), 23.8 (Arg, -$CH_2CH_2CH_2$-), 27.0 (Arg, -$CH_2CH(NH_2)COOH$), 41.9 (Arg, -$CH_2$NH-), 55.5 (Arg, -$CH(NH_2)COOH$), 130.9, 131.0 (Oleic acid, -CH=CH-), 158.8 (Arg, -$C(NH)NH_2$), 174.4

(Arg, COOH), 180.6 (Oleic acid, COOH).

25.7, 28.1, 28.2, 29.3, 30.3, 30.4, 30.5, 30.6, 30.7, 30.8, 32.7, 33.1, 37.2, 58.0 (Oleic acid, $CH_3(\underline{C}H_2)_7CH=CH(\underline{C}H_2)_7COOH$).

**Formulation (salt) 71**

FT-IR (KBr): 3347 $cm^{-1}$, 2927 $cm^{-1}$, 2855 $cm^{-1}$, 2361 $cm^{-1}$, 1680 $cm^{-1}$, 1405 $cm^{-1}$.

$^1$HNMR (MeOD, 400MHz): 0.91-0.95 (t, 6H, Linoleic acid, $-\underline{C}H_3$), 1.71-1.80 (m, 2H, Arg, $-CH_2CH_2CH_2-$), 1.88-1.97 (m, 2H, Arg, $-CH_2CH(NH_2)COOH$), 3.21-3.26 (t, 2H, Arg, - $C\underline{H_2}NH-$), 3.60-3.63 (t, 1H, Arg, $-C\underline{H}(NH_2)COOH$), 5.31-5.42 (m, 8H, Linoleic acid, - CH=CH-).

1.30-1.42 (m, 24H), 1.60-1.64 (m, 4H), 2.06-2.11 (q, 8H), 2.23-2.30(m, 4H), 3.21-3.25 (t, 4H), 3.31-3.34(t, 4H): (Linoleic acid, $CH_3(CH_2)_4(CH=CHCH_2)_2(CH_2)_6COOH$).

$^{13}$CNMR (MeOD, 100MHz): 14.7 (Linoleic acid, $-\underline{C}H_3$), 23.8 (Arg, $-CH_2\underline{C}H_2CH_2-$), 27.8 (Arg, $-\underline{C}H_2CH(NH_2)COOH$), 41.9 (Arg, $-\underline{C}H_2NH-$), 49.7 (Linoleic acid, $-CH_2COOH$), 55.5 (Arg, $-\underline{C}H(NH_2)COOH$), 129.1, 130.9 (Linoleic acid, -CH=CH-), 158.8 (Arg, $-\underline{C}(NH)NH_2$), 174.3 (Arg, COOH), 180.0 (Linoleic acid, COOH).

26.8, 28.2, 28.6, 29.9, 30.5, 30.8, 31.1, 32.0, 32.7, 36.5, 36.8, 37.1 (Linoleic acid, $CH_3(\underline{C}H_2)_4(CH=CH\underline{C}H_2)_2(CH_2)_6COOH$).

**<Working example 52> Formulation (salt) 52**

L-arginine (11.78 g, 0.10 mol) and 30 wt.% of lactic acid (30.01 g, 0.10 mol) were stirred in 50 mL of water at room temperature for 1 hour, and then the water was evaporated under reduced pressure to obtain a colorless liquid. The obtained liquid was cleaned to obtain a formulation (salt) of colorless liquid of L-arginine and lactic acid.

**Formulation (salt) 52**

FT-IR (KBr): 3366 $cm^{-1}$, 3189 $cm^{-1}$, 1718 $cm^{-1}$, 1141 $cm^{-1}$, 863 $cm^{-1}$.

$^1$HNMR ($D_2O$, 400MHz): 1.20-1.22 (d, 3H, Lactic acid, $-CH_3$), 1.49-1.61 (m, 2H, Arg, - $CH_2CH_2CH_2-$), 1.77-1.83 (m, 2H, Arg, $-CH_2CH(NH_2)COOH$), 3.11-3.14 (t, 2H, Arg, - $C\underline{H_2}NH-$), 3.64-3.67 (t, 1H, Arg, $-C\underline{H}(NH_2)COOH$), 3.97-4.02 (q, 1H, Lactic acid, -CHOH-).

$^{13}$CNMR ($D_2O$, 100MHz): 20.0 (Lactic acid, $-\underline{C}H_3$), 23.8 (Arg, $-CH_2\underline{C}H_2CH_2-$), 27.5 (Arg, $-CH_2\underline{C}H_2CH(NH_2)COOH$), 40.4 (Arg, $-\underline{C}H_2NH-$), 54.3 (Arg, $-\underline{C}H(NH_2)COOH$), 68.4 (Lactic acid, -CHOH-), 156.8 (Arg, $-\underline{C}(NH)NH_2$), 174.3 (Arg, COOH), 182.4 (Lactic acid, -COOH).

**<Working examples 9, 10, 30, 31, 51, 52, 72, 73, 93, 94, 115, 116> Formulations** (salts) **9, 10, 30, 31, 51, 52, 72, 73, 93, 94, 114 and 115**

Similarly, the components (A) and (B) were used in the compounding ratio as listed in Table 1A and prepared under the same condition as in Working example 52 to obtain formulations (salts) 9, 10, 30, 31, 51, 52, 72, 73, 93, 94, 114 and 115. Spectroscopy data for representative formulations (salts) are listed below.

**Formulation (salt) 9**

FT-IR (KBr): 3392 $cm^{-1}$, 2954 $cm^{-1}$, 1720 $cm^{-1}$, 1155 $cm^{-1}$, 903 $cm^{-1}$.

$^1$HNMR ($D_2O$, 400MHz): 1.25-1.44 (m, 2H, Lys, $-CH_2CH_2NH_2$), 1.56-1.63 (m, 2H, Lys, - $CH_2CH_2CH(NH_2)COOH$), 1.75-1.83 (m, 2H, Lys, $-C\underline{H_2}CH(NH_2)COOH$), 2.88-2.92 (t, 2H, Lys, $-C\underline{H_2}NH_2$), 3.52-3.55 (t, 1H, Lys, $-C\underline{H}(NH_2)COOH$).

3.63-3.72 3.52-3.72 (m, 4H), 3.92-3.93 (d, 1H), 4.00-4.02 (d, 1H): (Gluconic acid, $HOCH_2C\underline{H}(OH)C\underline{H}(OH)C\underline{H}(OH)C\underline{H}(OH)COOH$).

$^{13}$CNMR ($D_2O$, 100MHz): 21.4 (Lys, $-\underline{C}H_2CH_2NH_2$), 26.4 (Lys, $-\underline{C}H_2CH_2CH(NH_2)COOH$), 29.8 (Lys, $-\underline{C}H_2NH_2$), 38.6 (Lys, $-\underline{C}H_2CH(NH_2)COOH$), 54.5 (Lys, $-\underline{C}H(NH_2)COOH$), 174.6 (Lys, COOH), 178.5 (gluconic acid, COOH).

62.6, 70.9, 71.1, 72.5, 74.0 (Gluconic acid, $HO\underline{C}H_2\underline{C}H(OH)\underline{C}H(OH)\underline{C}H(OH)\underline{C}H(OH)COOH$).

**Formulation (salt) 30**

FT-IR (KBr): 3394 $cm^{-1}$, 1811 $cm^{-1}$, 1687 $cm^{-1}$, 1590 $cm^{-1}$, 1160 $cm^{-1}$.

$^1$HNMR ($D_2O$, 400MHz): 1.31-1.48 (m, 2H, Lys, $-CH_2CH_2NH_2$), 1.60-1.69 (m, 2H, Lys, - $C\underline{H_2}CH_2CH(NH_2)COOH$), 1.81-1.88 (m, 2H, Lys, $-C\underline{H_2}CH(NH_2)COOH$), 2.93-2.97 (t, 2H, Lys, $-C\underline{H_2}NH_2$), 3.56-3.60 (t,

1H, Lys, -CH($NH_2$)COOH).

3.68-3.81 (m, 8H), 3.98-4.01 (m, 2H), 4.17-4.19 (m, 2H): (Gluconic acid, $HOCH_2CH(OH)CH(OH)CH(OH)CH(OH)COOH$).

$^{13}$CNMR ($D_2O$, 100MHz): 21.3 (Lys, -$CH_2CH_2NH_2$), 26.2 (Lys, -$CH_2CH_2CH(NH_2)COOH$), 29.9 (Lys, -$CH_2NH_2$), 39.1 (Lys, -$CH_2CH(NH_2)COOH$), 54.7 (Lys, -CH($NH_2$)COOH), 174.5 (Lys, COOH), 177.5 (Gluconic acid, COOH).

62.3, 70.4, 71.9, 72.4, 73.7 (Gluconic acid, $HOCH_2CH(OH)CH(OH)CH(OH)CH(OH)COOH$),

**Formulation (salt) 51**

FT-IR (KBr): 3365 $cm^{-1}$, 1720 $cm^{-1}$, 1149 $cm^{-1}$, 836 $cm^{-1}$.

$^{1}$HNMR ($D_2O$, 400MHz): 1.47-1.63 (m, 2H, Arg, -$CH_2CH_2CH_2$-), 1.74-1.80 (m, 2H, Arg, $CH_2CH(NH_2)COOH$), 3.09-3.13 (t, 2H, Arg, -$CH_2$NH-), 3.63-3.66 (m, 1H, Arg, - CH($NH_2$)COOH).

3.51-3.71 (m, 4H), 3.89-3.90 (d, 1H), 3.99-4.00 (d, 1H): (Gluconic acid, $HOCH_2CH(OH)CH(OH)CH(OH)CH(OH)COOH$).

$^{13}$CNMR ($D_2O$, 100MHz): 23.8 (Arg, -$CH_2CH_2CH_2$-), 27.6 (Arg, -$CH_2CH(NH_2)COOH$), 40.4 (Arg, -$CH_2$NH-), 54.2 (Arg, -CH($NH_2$)COOH), 156.7 (Arg, -C(NH)$NH_2$), 174.6 (Arg, COOH), 178.5 (Gluconic acid, COOH).

62.5, 70.9, 71.1, 72.5, 74.0 (Gluconic acid, $HOCH_2CH(OH)CH(OH)CH(OH)CH(OH)COOH$).

**Formulation (salt) 72**

FT-IR (KBr): 3365 $cm^{-1}$,1795 $cm^{-1}$, 1153 $cm^{-1}$, 835 $cm^{-1}$.

$^{1}$HNMR ($D_2O$, 400MHz): 1.45-1.62 (m, 2H, Arg, -$CH_2CH_2CH_2$-), 1.73-1.81 (m, 2H, Arg, - $CH_2CH(NH_2)COOH$), 3.08-3.11 (t, 2H, Arg, -$CH_2$NH-), 3.63-3.66 (m, 1H, Arg, - CH($NH_2$)COOH).

3.49-3.69 (m, 8H) 3.93-3.94 (d, 2H), 4.12-4.13 (d, 2H) :(Gluconic acid, $HOCH_2CH(OH)CH(OH)CH(OH)CH(OH)COOH$).

$^{13}$CNMR ($D_2O$, 100MHz): 23.8 (Arg, -$CH_2CH_2CH_2$-), 27.5 (Arg, -$CH_2CH(NH_2)COOH$), 40.4 (Arg, -$CH_2$NH-), 54.2 (Arg, -CH($NH_2$)COOH), 156.7 (Arg, -C(NH)$NH_2$), 174.3 (Arg, COOH), 177.5 (gluconic acid, COOH).

62.6, 70.8, 71.0, 72.1, 73.3 (Gluconic acid, $HOCH_2CH(OH)CH(OH)CH(OH)CH(OH)COOH$).

**Formulation (salt) 73**

FT-IR (KBr): 3366 $cm^{-1}$, 3203 $cm^{-1}$, 1763 $cm^{-1}$, 1149 $cm^{-1}$, 866 $cm^{-1}$.

$^{1}$HNMR ($D_2O$, 400MHz): 1.23-1.25 (d, 6H, Lactic acid, -$CH_3$), 1.48-1.69 (m, 2H, Arg, - $CH_2CH_2CH_2$-), 1.75-1.81 (m, 2H, Arg, -$CH_2CH(NH_2)COOH$), 3.09-3.13 (t, 2H, Arg, - $CH_2$NH-), 3.63-3.66 (t, 1H, Arg, -CH($NH_2$)COOH), 4.09-4.15 (q, 2H, Lactic acid, -CHOH-). $^{13}$CNMR ($D_2O$, 100MHz): 19.6 (Lactic acid, -$CH_3$), 23.8 (Arg, -$CH_2CH_2CH_2$-), 27.5 (Arg, -$CH_2CH(NH_2)COOH$), 40.4 (Arg, -$CH_2$NH-), 54.2 (Arg, -CH($NH_2$)COOH), 67.4 (Lactic acid, -CHOH-), 156.7 (Arg, -C(NH)$NH_2$), 174.2 (Arg, COOH), 180.5 (Lactic acid, COOH).

**Formulation (salt) 93**

FT-IR (KBr): 3373 $cm^{-1}$, 3159 $cm^{-1}$, 1735 $cm^{-1}$, 1082 $cm^{-1}$.

$^{1}$HNMR ($D_2O$, 400MHz): 3.89 (s, 2H, His, -$CH_2$-), 3.87-3.90 (t, 1H, His, -CH($NH_2$)COOH). 3.20-3.70 (m, 5H), 4.00 (s, 1H): (Gluconic acid, $HOCH_2CH(OH)CH(OH)CH(OH)CH(OH)COOH$).

7.23 (s, 1H), 8.47 (s, 1H): (His, Imidazole).

$^{13}$CNMR ($D_2O$, 100MHz): 25.9 (His, -$CH_2$-), 53.6 (His, -CH($NH_2$)COOH), 172.6 (His, COOH), 178.5 (Gluconic acid, COOH).

62.5, 70.9, 71.1, 72.5, 74.0 (Gluconic acid, $HOCH_2CH(OH)CH(OH)CH(OH)CH(OH)COOH$).

117.6, 127.7, 134.1 (His, Imidazole).

**Formulation (salt) 114**

FT-IR (KBr): 3378 $cm^{-1}$, 2928 $cm^{-1}$, 1677 $cm^{-1}$, 1152 $cm^{-1}$, 894 $cm^{-1}$.

$^{1}$HNMR ($D_2O$, 400MHz): 3.21-3.33 (d, 2H, His, -$CH_2$-), 3.88-3.89 (t, 1H, His, - CH($NH_2$)COOH).

3.51-3.65 (m, 8H), 3.92-3.94 (m, 2H), 4.09-4.10 (m, 2H): (Gluconic acid, $HOCH_2CH(OH)CH(OH)CH(OH)CH(OH)COOH$).

7.26 (s, 1H), 8.54 (s, 1H): (His, Imidazole).

$^{13}$CNMR ($D_2O$, 100MHz): 25.7 (His, -$CH_2$-), 53.5 (His, -CH($NH_2$)COOH), 172.5 (His, COOH), 177.4 (Gluconic acid, COOH).

62.5, 71.8, 72.0, 73.2 (Gluconic acid, $HOCH_2CH(OH)CH(OH)CH(OH)CH(OH)COOH$). 117.6, 127.3, 133.9 (His, Imidazole).

**Formulation (salt) 115**

FT-IR (KBr): 3384 $cm^{-1}$, 3155 $cm^{-1}$, 1775 $cm^{-1}$, 1125 $cm^{-1}$, 875 $cm^{-}$.
$^1$HNMR ($D_2O$, 400MHz): 1.21-1.22 (d, 6H, Lactic acid, $-CH_3$), 3.19-3.21 (d, 2H, His, - $CH_2$-), 3.87-3.90 (t, 1H, His, $-CH(NH_2)COOH$), 4.07-4.11 (q, 2H, Lactic acid, -CHOH-). 7.24 (s, 1H), 8.52 (s, 1H): (His, Imidazole).
$^{13}$CNMR ($D_2O$, 100MHz): 19.6 (Lactic acid, $CH_3$-), 25.7 (His, $-CH_2$-), 53.5 (His, - $CH(NH_2)COOH$), 67.4 (Lactic acid, -CHOH-), 172.5 (His, COOH), 180.5 (Lactic acid, - COOH).
117.6, 127.3, 133.9 (His, Imidazole).

<Working example 53> Formulation (salt) 53

L-arginine (11.78 g, 0.10 mol) and adipic acid (14.61 g, 0.10 mol) were stirred in 50 mL of water at room temperature for 1 hour, and then the water was evaporated under reduced pressure to obtain a white solid formulation (salt) of L-arginine and adipic acid.

Formulation (salt) 53

FT-IR (KBr): 3131 $cm^{-1}$, 2940 $cm^{-1}$, 1752 $cm^{-1}$, 1427 $cm^{-1}$, 857 $cm^{-1}$.
$^1$HNMR ($D_2O$, 400MHz): 1.47-1.66 (m, 4H, Adipic acid, $-CH_2$-), 1.48-1.69 (m, 2H, Arg, - $CH_2CH_2CH_2$-), 1.76-1.82 (m, 2H, Arg, $-CH_2CH(NH_2)COOH$), 2.18-2.20 (t, 4H, Adipic acid, $-CH_2COOH$), 3.10-3.13 (t, 2H, Arg, $-CH_2NH$-), 3.64-3.66 (t, 1H, Arg, $-CH(NH_2)COOH$).
$^{13}$CNMR ($D_2O$, 100MHz): 23.8 (Arg, $-CH_2CH_2CH_2$-), 24.6 (Adipic acid, $-CH_2$-), 27.5 (Arg, $-CH_2CH(NH_2)COOH$), 35.3 (Adipic acid, $-CH_2COOH$), 40.4 (Arg, $-CH_2NH$-), 54.2 (Arg, - $CH(NH_2)COOH$), 156.7 (Arg, $-C(NH)NH_2$), 174.3 (Arg, COOH), 181.1 (Adipic acid, COOH).

**<Working examples 11-13, 32-34, 53-55, 74-76, 95-97, 116-118> Formulation (salt) 11-13, 32-34, 53-55, 74-76, 95-97, 116-118**

Similarly, the components (A) and (B) were used in the compounding ratio as listed in Table 1A and prepared under the same condition as in Working example 53 to obtain formulations (salts) 11 to 13, 32 to 34, 53 to 55, 74-76, 95-97, 116-118.

**<Working example 56> Formulation (salt) 56**

L-arginine (11.78 g, 0.10 mol) and fumaric acid (11.60 g, 0.10 mol) were stirred in 50 mL of water at room temperature for 1 hour, and then the water was evaporated under reduced pressure to obtain a white solid formulation (salt) of L-arginine and adipic acid.

**Formulation (salt) 56**

FT-IR (KBr): 3147 $cm^{-1}$, 1749 $cm^{-1}$, 1632 $cm^{-1}$, 1555 $cm^{-1}$, 1375 $cm^{-1}$.
$^1$HNMR ($D_2O$, 400MHz): 1.47-1.65 (m, 2H, Arg, $-CH_2CH_2CH_2$-), 1.76-1.83 (m, 2H, Arg,-$-CH_2CH(NH_2)COOH$), 3.10-3.13 (t, 2H, Arg, $-CH_2NH$-), 3.65-3.68 (t, 1H, Arg, - $CH(NH_2)COOH$), 6.56 (s, 2H, Fumaric acid, -CH=CH-).
$^{13}$CNMR ($D_2O$, 100MHz): 23.6 (Arg, $-CH_2CH_2CH_2$-), 27.5 (Arg, $-CH_2CH(NH_2)COOH$), 40.4 (Arg, $-CH_2NH$-), 54.2 (Arg, $-CH(NH_2)COOH$), 134.7 (Fumaric acid, -CH=CH-), 156.7 (Arg, $-C(NH)NH_2$), 171.7 (Fumaric acid, COOH), 174.2 (Arg, COOH)

**<Working examples 14, 35, 56, 77, 98, 119> Formulations (salts) 14, 35, 56, 77, 98, 119**

Similarly, the components (A) and (B) were used in the compounding ratio as listed in Table 1A and prepared under the same condition as in Working example 56 to obtain formulations (salts) 14, 35, 56, 77, 98 and 119.

**<Working example 16> Formulation (salt) 16**

L-lysine (14.63 g, 0.10 mol) and citric acid (19.39 g, 0.10 mol) were stirred in 50 mL of water at room temperature

for 3 hour, and then the water was evaporated under reduced pressure to obtain a yellow liquid. The obtained liquid was cleaned to obtain a formulation (salt) of yellow liquid of L-lysine and citric acid.

**Formulation (salt) 16**

FT-IR (KBr): 3432 $cm^{-1}$, 3148 $cm^{-1}$, 1764 $cm^{-1}$, 1217 $cm^{-1}$, 848 $cm^{-1}$.

$^1$HNMR ($D_2O$, 400MHz): 1.26-1.44 (m, 2H, Lys, $-CH_2CH_2NH_2$), 1.55-1.63 (m, 2H, Lys, $-CH_2CH_2CH(NH_2)COOH$), 1.74-1.81 (m, 2H, Lys, $-CH_2CH(NH_2)COOH$), 2.62-2.65 (d, 2H, Citric acid, $-CH_2COOH$), 2.75-2.78 (d, 2H, Citric acid, $-CH_2COOH$), 2.86-2.90 (t, 2H, Lys, $-CH_2NH_2$), 3.62-3.65 (t, 1H, Lys, $-CH(NH_2)COOH$).

$^{13}$CNMR ($D_2O$, 100MHz): 21.4 (Lys, $-CH_2CH_2NH_2$), 26.3 (Lys, $-CH_2CH_2CH(NH_2)COOH$), 29.8 (Lys, $-CH_2NH_2$), 39.0 (Lys, $-CH_2CH(NH_2)COOH$), 43.7 (Citric acid, $-CH_2COOH$), 54.4 (Lys, $-CH(NH_2)COOH$), 73.8 (Citric acid, -C(OH)(COOH)-), 174.4 (Lys, COOH) 174.7 (Citric acid, $-CH_2COOH$), 178.6 (Citric acid, -C(OH)(COOH)-).

**<Working examples 15-17, 36-38, 57-59, 78-80, 99-101, 120-122> Formulation (salt) 15-17, 36-38, 57-59, 78-80, 99- 101, 120-122**

Similarly, the components (A) and (B) were used in the compounding ratio as listed in Table 1A and prepared under the same condition as in Working example 16 to obtain formulations (salts) 15 to 17, 36 to 38, 57 to 59, 78-80, 99-101, 120-122. Spectroscopy data for representative formulations (salts) are listed below.

**Formulation (salt) 37**

FT-IR (KBr): 3400 $cm^{-1}$, 1762 $cm^{-1}$, 1575 $cm^{-1}$, 853 $cm^{-1}$.

$^1$HNMR ($D_2O$, 400MHz): 1.33-1.48 (m, 6H, Lys, $-CH_2CH_2NH_2$), 1.62-1.70 (m, 6H, Lys, $-CH_2CH_2CH(NH_2)COOH$), 1.81-1.88 (m, 6H, Lys, $-CH_2CH(NH_2)COOH$), 2.62-2.78 (d, 8H, Citric acid, $CH_2(COOH)-$), 2.94-2.98 (t, 6H, Lys, $-CH_2NH_2$), 3.71-3.74 (t, 3H, Lys, - $CH(NH_2)COOH$).

$^{13}$CNMR ($D_2O$, 100MHz): 21.4 (Lys, $-CH_2CH_2NH_2$), 26.4 (Lys, $-CH_2CH_2CH(NH_2)COOH$), 29.8 (Lys, $-CH_2NH_2$), 39.1 (Lys, $-CH_2CH(NH_2)COOH$), 43.6 (Citric acid, $CH_2(COOH)-$), 54.6 (Lys, $-CH(NH_2)COOH$), 73.5 (Citric acid, -C(OH)(COOH)-), 174.0 (Lys, COOH), 174.3 (Citric acid, $-CH_2COOH$), 177.8 (Citric acid, -C(OH)(COOH)-).

**Formulation (salt) 57**

FT-IR (KBr): 3368 $cm^{-1}$, 3203 $cm^{-1}$, 1763 $cm^{-1}$, 1398 $cm^{-1}$, 918 $cm^{-1}$.

$^1$HNMR ($D_2O$, 400MHz): 1.47-1.64 (m, 2H, Arg, $-CH_2CH_2CH_2-$), 1.76-1.82 (m, 2H, Arg,$--CH_2CH(NH_2)COOH$), 2.48-2.72 (m, 2H, Malic acid, $-CH_2COOH$), 3.10-3.14 (t, 2H, Arg, - $CH_2NH-$), 3.64-3.67 (t, 1H, Arg, $-CH(NH_2)COOH$), 4.25-4.28 (t, 1H, Malic acid, - CH(OH)COOH).

$^{13}$CNMR ($D_2O$, 100MHz): 23.8 (Arg, $-CH_2CH_2CH_2-$), 27.5 (Arg, $-CH_2CH(NH_2)COOH$), 40.1 (Malic acid, $-CH_2-$), 40.4 (Arg,$-CH_2NH-$), 54.2 (Arg, $-CH(NH_2)COOH$), 68.6 (Malic acid, -CHOH-), 156.7 (Arg, $-C(NH)NH_2$), 174.3 (Arg, COOH), 176.4 (Malic acid, - $CH_2COOH$), 179.1 (Malic acid, -CH(OH)COOH).

**Formulation (salt) 58**

FT-IR (KBr): 3365 $cm^{-1}$, 3207 $cm^{-1}$, 1763 $cm^{-1}$, 1217 $cm^{-1}$, 850 $cm^{-1}$.

$^1$HNMR ($D_2O$, 400MHz): 1.47-1.66 (m, 2H, Arg, $-CH_2CH_2CH_2-$), 1.76-1.81 (m, 2H, Arg, - $CH_2CH(NH_2)COOH$), 2.62-2.79 (d, 4H, Citric acid, $-CH_2COOH$), 3.10-3.13 (t, 2H, Arg, - $CH_2NH-$), 3.64-3.67 (t, 1H, Arg, $-CH(NH_2)COOH$).

$^{13}$CNMR ($D_2O$, 100MHz): 23.8 (Arg, $-CH_2CH_2CH_2-$), 27.5 (Arg, $-CH_2CH(NH_2)COOH$), 40.4 (Arg, $-CH_2NH-$), 43.7 (Citric acid, $-CH_2COOH$), 54.2 (Arg, $-CH(NH_2)COOH$), 73.8 (Citric acid, -C(OH)(COOH)-), 156.7 (Arg, $-C(NH)NH_2$), 174.3 (Arg, COOH), 174.8 (Citric acid, $-CH_2COOH$), 178.7(Citric acid, -C(OH)(COOH)-).

**Formulation (salt) 59**

FT-IR (KBr): 3364 $cm^{-1}$, 3211 $cm^{-1}$, 1764 $cm^{-1}$, 1154 $cm^{-1}$, 854 $cm^{-1}$.

$^1$HNMR ($D_2O$, 400MHz): 1.49-1.66 (m, 2H, Arg, $-CH_2CH_2CH_2-$), 1.77-1.82 (m, 2H, Arg, - $CH_2CH(NH_2)COOH$), 3.11-3.14 (t, 2H, Arg, $-CH_2NH-$), 3.65-3.68 (t, 1H, Arg, - $CH(NH_2)COOH$), 4.40 (s, 2H, Tartaric acid, -CH(OH)COOH).

$^{13}$CNMR ($D_2O$, 100MHz): 23.8 (Arg, $-CH_2CH_2CH_2-$), 27.5 (Arg, $-CH_2CH(NH_2)COOH$), 40.4 (Arg, $-CH_2NH-$), 54.2 (Arg, $-CH(NH_2)COOH$), 72.8 (Tartaric acid, -CH(OH)COOH), 156.7 (Arg, $-C(NH)NH_2$), 174.2(Arg, COOH), 176.5 (Tartaric acid, COOH).

**Formulation (salt) 78**

FT-IR (KBr): 3368 $cm^{-1}$, 3203 $cm^{-1}$, 1763 $cm^{-1}$, 1128 $cm^{-1}$, 918 $cm^{-1}$.

$^{1}$HNMR ($D_2O$, 400MHz): 1.48-1.64 (m, 2H, Arg, $-CH_2CH_2CH_2-$), 1.76-1.82 (m, 2H, Arg, - $CH_2CH(NH_2)COOH-$), 2.60-2.78 (d, 4H, Malic acid, $CH_2COOH-$), 3.10-3.13 (t, 2H, Arg, - $CH_2NH-$), 3.65-3.68 (t, 1H, Arg, $-CH(NH_2)COOH$), 4.35-4.37 (t, 2H, Malic acid, - $CH(OH)COOH$).

$^{13}$CNMR ($D_2O$, 100MHz): 23.8 (Arg, $-CH_2CH_2CH_2-$), 27.4 (Arg, $-CH_2CH(NH_2)COOH$), 39.1 (Malic acid, $-CH_2COOH$), 40.4 (Arg, $-CH_2NH-$), 54.2 (Arg, $-CH(NH_2)COOH$), 67.6 (Malic acid, -CH(OH)COOH), 156.7 (Arg, $-C(NH)NH_2$), 174.2 (Arg, COOH), 175.3 (Malic acid, $CH_2COOH$), 177.9 (Malic acid, CH(OH)COOH).

**Formulation (salt) 79**

FT-IR (KBr): 3365 $cm^{-1}$, 3194 $cm^{-1}$, 1764 $cm^{-1}$, 1213 $cm^{-1}$.

$^{1}$HNMR ($D_2O$, 400MHz): 1.48-1.65 (m, 6H, Arg, $-CH_2CH_2CH_2-$), 1.76-1.82 (m, 6H, Arg, $CH(COOH)(NH_2)CH_2-$), 2.57-2.61 (d, 4H, Citric acid, $-CH_2COOH$), 2.69-2.72 (d, 4H, Citric acid, $-CH_2COOH$), 3.10-3.13 (t, 6H, Arg, $-CH_2NH-$), 3.63-3.67 (t, 3H, Arg, - $CH(NH_2)COOH$).

$^{13}$CNMR ($D_2O$, 100MHz): 23.8 (Arg, $-CH_2CH_2CH_2-$), 27.4 (Arg, $-CH_2CH(NH_2)COOH$), 39.1 (Citric acid, $-CH_2COOH$), 40.4 (Arg, $-CH_2NH-$), 54.2 (Arg, $-CH(NH_2)COOH$), 67.6 (Citric acid, -C(OH)COOH), 156.7 (Arg, $-C(NH)NH_2$), 174.2 (Arg, COOH), 175.3 (Citric acid, $-CH_2COOH$), 177.9 (Citric acid, -C(OH)(COOH)-).

**Formulation (salt) 80**

FT-IR (KBr): 3365 $cm^{-1}$, 3215 $cm^{-1}$, 1693 $cm^{-1}$, 1150 $cm^{-1}$, 725 $cm^{-1}$.

$^{1}$HNMR ($D_2O$, 400MHz): 1.46-1.65 (m, 2H, Arg, $-CH_2CH_2CH_2-$), 1.76-1.82 (m, 2H, Arg, - $CH_2CH(NH_2)COOH$), 3.09-3.12 (t, 2H, Arg, $-CH_2NH-$), 3.66-3.69 (t, 1H, Arg, - $CH(NH_2)COOH$), 4.49 (s, 4H, Tartaric acid, -CH(OH)COOH).

$^{13}$CNMR ($D_2O$, 100MHz): 23.8 (Arg, $-CH_2CH_2CH_2-$), 27.4 (Arg, $-CH_2CH(NH_2)COOH$), 40.4 (Arg, $-CH_2NH-$), 54.0 (Arg, $-CH(NH_2)COOH$), 72.3 (Tartaric acid, -CH(OH)COOH), 156.7 (Arg, $C(NH)NH_2$), 174.0 (Arg, COOH), 175.5 (Tartaric acid, COOH).

**Formulation (salt) 100**

FT-IR (KBr): 3408 $cm^{-1}$, 3149 $cm^{-1}$, 1715 $cm^{-1}$, 1225 $cm^{-1}$, 828 $cm^{-1}$.

$^{1}$HNMR ($D_2O$, 400MHz): 2.62-2.66 (d, 2H, Citric acid, $-CH_2COOH$), 2.74-2.78 (d, 2H, Citric acid, $-CH_2COOH$), 3.21-3.23 (d, 2H, His, $-CH_2-$), 3.89-3.92 (t, 1H, His, - $CH(NH_2)COOH$).

7.23 (s, 1H), 8.54 (s, 1H): (His, Imidazole).

$^{13}$CNMR ($D_2O$, 100MHz): 25.7 (His, $-CH_2-$), 43.6 (Citric acid, $-CH_2COOH$), 53.5 (His, - $CH(NH_2)COOH$), 73.8 (Citric acid, -C(OH)(COOH)-), 172.5 (His, COOH), 174.7 (Citric acid, $-CH_2COOH$), 178.7 (Citric acid, -C(OH)(COOH)-).

117.6, 127.7, 134.1 (His, Imidazole).

**Formulation (salt) 121**

FT-IR (KBr): 3387 $cm^{-1}$, 3249 $cm^{-1}$, 1710 $cm^{-1}$, 924 $cm^{-1}$.

$^{1}$HNMR ($D_2O$, 400MHz): 2.50-2.54 (d, 4H, Citric acid, $-CH_2COOH$), 2.63-2.67 (d, 4H, Citric acid, $-CH_2COOH$), 3.16-3.18 (d, 6H, His, $-CH_2-$), 3.84-3.87 (t, 3H, His, - $CH(NH_2)COOH$).

7.23 (s, 3H), 8.47 (s, 3H): (His, Imidazole).

$^{13}$CNMR ($D_2O$, 100MHz): 25.6 (His, $-CH_2-$), 43.6 (Citric acid, $-CH_2COOH$), 53.5 (His, - $CH(NH_2)COOH$), 73.8 (Citric acid, -C(OH)(COOH)-), 172.6 (His, COOH), 175.8 (Citric acid, $-CH_2COOH$), 179.5 (Citric acid, -C(OH)(COOH)-).

117.6, 127.7, 134.1 (His, Imidazole).

**<Working example 60> Formulation (salt) 60**

L-arginine (11.78 g, 0.10 mol) and benzoic acid (12.21 g, 0.10 mol) were stirred in 50 mL of water at room temperature for 1 hour, and then the water was evaporated under reduced pressure to obtain a colorless liquid. The obtained liquid was cleaned to obtain a formulation (salt) of colorless liquid of L-arginine and benzoic acid.

**Formulation (salt) 60**

FT-IR (KBr): 3366 $cm^{-1}$, 3189 $cm^{-1}$, 1718 $cm^{-1}$, 1191 $cm^{-1}$, 848 $cm^{-1}$, 738 $cm^{-1}$.

$^1$HNMR ($D_2O$, 400MHz): 1.41-1.59 (m, 2H, Arg, $-CH_2CH_2CH_2-$), 1.70-1.76 (m, 2H, Arg,$--CH_2CH(NH_2)COOH$), 2.99-3.03 (t, 2H, Arg, $-CH_2NH-$), 3.59-3.61 (t, 1H, Arg, -CH $(NH_2)COOH$), 7.31-7.35 (t, 2H, Benzoic acid, Ph), 7.38-7.42 (t, 1H, Benzoic acid, Ph), 7.72-7.74 (d, 2H, Benzoic acid, Ph).

$^{13}$CNMR ($D_2O$, 100MHz): 23.8 (Arg, $-CH_2\underline{C}H_2CH_2-$), 27.5 (Arg, $-\underline{C}H_2CH(NH_2)COOH$), 40.4 (Arg, $-NH\underline{C}H_2CH_2-$), 54.3 (Arg, $-\underline{C}H_2NH-$), 128.3 (Benzoic acid, Ph), 128.6 (Benzoic acid, Ph), 131.2 (Benzoic acid, Ph), 136.1 (Benzoic acid, Ph), 156.6 (Arg, $-\underline{C}(NH)NH_2$), 174.3 (Arg, COOH), 175.6 (Benzoic acid, COOH).

**<Working examples 18, 39, 60, 81, 102, 123> Formulation (salt) 18, 39, 60, 81, 102, 123**

Similarly, the components (A) and (B) were used in the compounding ratio as listed in Table 1A and prepared under the same condition as in Working example 60 to obtain formulations (salts) 18, 39, 60, 81, 102 and 123.

**<Working example 61> Formulation (salt) 61**

L-arginine (11.78 g, 0.10 mol) and cinnamic acid (14.81 g, 0.10 mol) were stirred in 50 mL of water at room temperature for 1 hour, and then the water was evaporated under reduced pressure to obtain a white solid formulation (salt) of L-arginine and cinnamic acid.

**Formulation (salt) 61**

FT-IR (KBr): 3088 $cm^{-1}$, 1637 $cm^{-1}$, 1532 $cm^{-1}$, 1381 $cm^{-1}$.

$^1$HNMR ($D_2O$, 400MHz): 1.44-1.63 (m, 2H, Arg, $-CH_2CH_2CH_2-$), 1.74-1.80 (m, 2H, Arg,$--CH_2CH(NH_2)COOH$), 3.05-3.08 (t, 2H, Arg, $-CH_2NH-$), 3.61-3.64 (t, 1H, Arg, -CH $(NH_2)COOH$), 6.37-6.42 (d, 2H, Cinnamic acid, -CH=CH-), 7.25-7.33 (m, 3H, Cinnamic acid, Ph), 7.49-7.51 (d, 2H, Cinnamic acid, Ph).

$^{13}$CNMR ($D_2O$, 100MHz): 23.8 (Arg, $-CH_2\underline{C}H_2CH_2-$), 27.5 (Arg, $-\underline{C}H_2CH(NH_2)COOH$), 40.5 (Arg, $-NH\underline{C}H_2CH_2-$), 54.3 (Arg, $-\underline{C}H_2NH-$), 124.1, 127.7, 129.0, 129.6, 135.1, 140.8 (Cinnamic acid, Ph, $-\underline{C}H=\underline{C}H-$),156.6 (Arg, $-\underline{C}(NH)NH_2$), 174.3 (Arg, COOH), 175.7 (Cinnamic acid, COOH).

**<Working examples 19, 40, 61, 82, 103, 124> Formulations (salts) 19, 40, 61, 82, 103, 124**

Similarly, the components (A) and (B) were used in the compounding ratio as listed in Table 1A and prepared under the same condition as in Working example 61 to obtain formulations (salts) 19, 40, 61, 82, 103 and 124.

**<Working example 62> Formulation (salt) 62**

L-arginine (11.78 g, 0.10 mol) and L-ascorbic acid (17.61 g, 0.10 mol) were stirred in 50 mL of water at room temperature for 1 hour, and then the water was evaporated under reduced pressure to obtain a colorless liquid. The obtained liquid was cleaned to obtain a formulation (salt) of white solid formulation (salt) of L-arginine and L-ascorbic acid.

**Formulation (salt) 62**

FT-IR (KBr): 3161 $cm^{-1}$, 1559 $cm^{-1}$, 1342 $cm^{-1}$, 1108 $cm^{-1}$, 900 $cm^{-1}$.

$^1$HNMR ($D_2O$, 400MHz): 1.50-1.68 (m, 2H, Arg, $-CH_2CH_2CH_2-$), 1.77-1.84 (m, 2H, Arg, - $CH_2CH(NH_2)COOH$), 3.12-3.16 (t, 2H, Arg, $-CH_2NH-$), 3.89-3.94 (t, 1H, Arg, - $C\underline{H}(NH_2)COOH$).

3.61-3.69 (m, 3H), 4.41-4.42 (d, 1H): (Ascorbic acid, $C\underline{H}_{cyclo}$, $-C\underline{H}(OH)CH_2OH$). $^{13}$CNMR ($D_2O$, 100MHz): 23.8 (Arg, $-CH_2\underline{C}H_2CH_2-$), 27.5 (Arg, $-\underline{C}H_2CH(NH_2)COOH$), 40.4 (Arg, $-\underline{C}H_2NH-$), 54.4 (Arg, $-\underline{C}H(NH_2)COOH$), 156.7 (Arg, $-\underline{C}(NH)NH_2$), 174.3 (Arg, COOH).

62.5, 69.1, 78.3, 113.1, 177.8: (Ascorbic acid, $\underline{C}H_{cyclo}$, $-\underline{C}H(OH)\underline{C}H_2OH$).

**<Working examples 20, 21, 41, 42, 62, 63, 83, 84, 104, 105, 125, 126> Formulations (salts) 20, 21, 41, 42, 62, 63, 83, 84, 104, 105, 125, 126**

Similarly, the components (A) and (B) were used in the compounding ratio as listed in Table 1A and prepared under the same condition as in Working example 62 to obtain formulations (salts) 20, 21, 41, 42, 62, 63, 83, 84, 104, 105, 125 and 126.

**<Working example 170> Formulation (salt) 170**

γ-aminobutyric acid (10.31 g, 0.10 mol) and acetic acid (6.01, 0.10 mol) were stirred in 50 mL of water at room temperature for 1 hour, and then the water was evaporated under reduced pressure to obtain a white solid formulation (salt) of γ-aminobutyric acid and acetic acid.

**Formulation (salt) 170**

FT-IR (KBr): 2931 $cm^{-1}$, 2209 $cm^{-1}$, 1653 $cm^{-1}$, 1574 $cm^{-1}$, 1029 $cm^{-1}$, 659 $cm^{-1}$.

$^1$HNMR ($D_2O$, 400MHz): 1.77-1.87 ((m, 3H, Acetic acid, $-CH_3$), (m, 2H, γ-Aminobutyric acid, $-CH_2\underline{CH_2}CH_2-$)), 2.19-2.23 (quin, 2H, γ-Aminobutyric acid, $-CH_2NH_2$), 2.90-2.94 (t, 2H, γ-Aminobutyric acid, $-CH_2COOH$).

$^{13}$CNMR ($D_2O$, 100MHz): 23.4 (γ-Aminobutyric acid, $-CH_2\underline{C}H_2CH_2-$), 34.1 (γ-Aminobutyric acid, $-\underline{C}H_2NH_2$), 39.2 (Acetic acid, $-\underline{C}H_3$), 39.4 (γ-Aminobutyric acid, - $\underline{C}H_2COOH$), 180.2 (γ-Aminobutyric acid, COOH), 181.3 (Acetic acid, COOH).

**<Working examples 127-132, 147-152, 167-172, 187-191, 204-208, 221-225, 238-242, 255-259, 272-276, 289-293, 306-310, 323-327 , 340-345, 360-364, 377-381 > Formulations (salts) 127-132, 147-152, 167-172, 187-191, 204-208, 221-225, 238-242, 255-259, 272-276, 289-293, 306-310, 323-327, 340-345, 360-364, 377-381**

Similarly, the components (A) and (B) were used in the compounding ratio as listed in Tables 1B and 1C and prepared under the same condition as in Working example 170 to obtain formulations (salts) 127 to 132, 147 to 152, 167 to 172, 187-191, 204-208, 221-225, 238-242, 255-259, 272-276, 289-293, 306-310, 323-327, 340-345, 360-364 and 377-381. Spectroscopy data for representative formulations (salts) are listed below.

**Formulation (salt) 345**

FT-IR (KBr): 2925 $cm^{-1}$, 2855 $cm^{-1}$, 1712 $cm^{-1}$, 1616 $cm^{-1}$, 1463 $cm^{-1}$.

$^1$HNMR (MeOD, 400MHz): 0.86-0.94 (m, 6H, Isostearic acid, $-C\underline{H_3}$), 3.32-3.40 (m, 1H, Isostearic acid, -CH(COOH)-).

1.31 (s, 22H), 1.59-1.63 (m, 2H), 2.17-2.21 (t, 2H), 3.21-3.25 (t, 2H): (Isostearic acid, $CH_3(CH_2)_8CH((CH_2)_6CH_3)COOH$).

1.89-2.27 (m, 4H), 3.19-3.34 (m, 2H), 4.00-4.06 (t, 1H): (Pro, $CH_{2\ hetero}$, $CH_{hetero}$). $^{13}$CNMR (MeOD, 100MHz): 56.2 (Isostearic acid, -CH(COOH)-), 174.1 (Pro, COOH), 182.6 (Isostearic acid, COOH).

29.5, 30.8, 38.8, 48.4, 48.8, 49.0, 49.5 (Isostearic acid, $\underline{C}H_3(\underline{C}H_2)_8CH((\underline{C}H_2)_6\underline{C}H_3)COOH$). 23.6, 28.9, 46.0, 61.0: (Pro, $\underline{C}H_{2\ hetero}$, $CH_{hetero}$).

**<Working example 173> Formulation (salt) 173**

γ-aminobutyric acid (10.31 g, 0.10 mol) and oleic acid (28.25 g, 0.10 mol) were stirred in 50 mL of water and 50 mL of ethanol at room temperature for 3 hours, and then the solvents were evaporated under reduced pressure to obtain a colorless liquid. The obtained liquid was cleaned to obtain a formulation (salt) of colorless liquid of γ-aminobutyric acid and oleic acid.

**Formulation (salt) 173**

FT-IR (KBr): 2925 $cm^{-1}$, 2854 $cm^{-1}$, 1711 $cm^{-1}$, 1552 $cm^{-1}$, 1406 $cm^{-1}$, 1244 $cm^{-1}$.

$^1$HNMR (MeOD, 400MHz): 0.90-0.94 (m, 3H, Oleic acid, $-CH_3$), 1.86-1.93 (quin, 2H, γ-Aminobutyric acid, $-CH_2CH_2CH_2-$), 2.35-2.39 (t, 2H, γ-Aminobutyric acid, $-CH_2NH_2$), 2.97-3.00 (t, 2H, γ-Aminobutyric acid, $-C\underline{H_2}COOH$), 3.32-3.34 (m, 2H, Oleic acid, - $CH_2COOH$), 5.35-5.38 (m, 2H, Oleic acid, -CH=CH-).

1.31-1.35 (m, 20H), 1.58-1.66 (m, 2H), 2.03-2.10 (m, 2H), 2.25-2.29 (m, 2H): (Oleic acid, $CH_3(CH_2)_7CH=CH(CH_2)_7COOH$).

$^{13}$CNMR (MeOD, 100MHz): 14.5 (Oleic acid, $-CH_3$), 23.7 (y-Aminobutyric acid, - $CH_2\underline{C}H_2CH_2-$), 32.7 (y-Aminobutyric acid, $-\underline{C}H_2NH_2$), 40.9 (y-Aminobutyric acid, - $\underline{C}H_2COOH$), 130.9 (Oleic acid, -CH=CH-), 178.8 (y-Aminobutyric acid, COOH), 179.8 (Oleic acid, COOH).

24.7, 26.4, 28.2, 30.3, 30.37, 30.39, 30.48, 30.52, 30.6, 30.8, 33.1, 35.4, 35.9 (Oleic acid, $CH_3(\underline{C}H_2)_7CH=CH(\underline{C}H_2)_7COOH$).

**<Working examples 133, 134, 153, 154, 173, 174, 346, 347> Formulations (salts) 133, 134, 153, 154, 173, 174, 346, 347**

Similarly, the components (A) and (B) were used in the compounding ratio as listed in Tables 1B and 1C prepared under the same condition as in Working example 173 to obtain formulations (salts) 133, 134, 153, 154, 173 , 174, 346 and 347. Spectroscopy data for representative formulations (salts) are listed below.

**Formulation (salt) 346**

FT-IR (KBr): 2938 $cm^{-1}$, 2855 $cm^{-1}$, 1711 $cm^{-1}$, 1616 $cm^{-1}$, 1410 $cm^{-1}$, 1291 $cm^{-1}$. $^1$HNMR (MeOD, 400MHz): 0.90-0.94 (t, 3H, Oleic acid, $-CH_3$), 5.32-5.41 (t, 2H, Oleic acid, -CH=CH-).

1.31-1.35 (m, 20H), 1.60-1.64 (t, 2H), 1.96-2.17 (m, 4H), 2.27-2.37 (m, 2H): (Oleic acid, $CH_3(CH_2)_7CH=CH(CH_2)_7COOH$).

1.96-2.37 (m, 4H), 3.23-3.39 (m, 2H), 3.98-4.00 (t, 1H): (Pro, $CH_{2\ hetero}$, $\underline{C}H_{hetero}$). $^{13}$CNMR (MeOD, 100MHz): 14.7 (Oleic acid, $-CH_3$), 63.1 (Oleic acid, $-\underline{C}H_2COOH$), 130.8, 130.9 (Oleic acid, -CH=CH-), 174.1 (Pro, COOH), 177.8 (Oleic acid, COOH).

25.2, 26.2, 27.7, 29.7, 30.3, 30.5, 30.7, 30.8, 33.1, 34.8, 35.1 (Oleic acid, $CH_3(\underline{C}H_2)_7CH=CH(\underline{C}H_2)_7COOH$).

23.8, 28.2, 48.4, 62.2: (Pro, $\underline{C}H_{2\ hetero}$, $CH_{hetero}$).

**Formulation (salt) 347**

FT-IR (KBr): 2927 $cm^{-1}$, 2856 $cm^{-1}$, 1711 $cm^{-1}$, 1617 $cm^{-1}$, 1410 $cm^{-1}$, 1252 $cm^{-1}$. $^1$HNMR (MeOD, 400MHz): 0.91-0.95 (t, 3H, Linoleic acid, $-CH_3$), 5.31-5.42 (m, 4H, Linoleic acid, -CH=CH-).

1.31-1.41 (m, 12H), 1.59-1.64 (m, 2H), 1.95-2.18 (q, 4H), 2.78-2.81 (t, 2H), 3.23-3.29 (t, 2H), 3.30-3.33 (t, 2H): (Linoleic acid, $CH_3(CH_2)_4(CH=CHCH_2)_2(CH_2)_6COOH$).

1.89-2.38 (m, 4H), 3.23-3.39 (m, 2H), 3.98-4.06 (t, 1H): (Pro, $CH_{2\ hetero}$, $\underline{C}H_{hetero}$). $^{13}$CNMR (MeOD, 100MHz): 14.7 (Linoleic acid, $-\underline{C}H_3$), 129.1, 130.9(Linoleic acid, - CH=CH-), 174.0 (Pro, COOH), 177.8 (Linoleic acid, COOH).

26.6, 27.8, 28.6, 29.6, 30.1, 30.3, 30.5, 30.7, 32.7, 34.8, 35.1, 35.3, 47.1 (Linoleic acid, $CH_3(\underline{C}H_2)_4(CH=CH\underline{C}H_2)_2(\underline{C}H_2)_6COOH$).

26.2, 28.2, 48.4, 62.2: (Pro, $\underline{C}H_{2\ hetero}$, $CH_{hetero}$).

**<Working example 176> Formulation (salt) 176**

L-aminobutyric acid (10.31.78 g, 0.10 mol) and 30 wt.% of lactic acid (30.01 g, 0.10 mol) were stirred in 50 mL of water at room temperature for 3 hour, and then the water was evaporated under reduced pressure to obtain a colorless liquid. The obtained liquid was cleaned to obtain a formulation (salt) of colorless liquid of γ-aminobutyric acid and lactic acid.

**Formulation (salt) 176**

FT-IR (KBr): 3406 $cm^{-1}$, 2984 $cm^{-1}$, 1764 $cm^{-1}$, 878 $cm^{-1}$.

$^1$HNMR ($D_2O$, 400MHz): 1.22-1.24 (d, 3H, Lactic acid, $-CH_3$), 1.76-1.84 (quin, 2H, γ-Aminobutyric acid, $-CH_2CH_2CH_2-$), 2.28-2.34 (t, 2H, γ-Aminobutyric acid, $-C\underline{H}_2NH_2$), 2.88-2.91 (t, 2H, γ-Aminobutyric acid, $-C\underline{H}_2COOH$), 4.06-4.11 (q, 1H, Lactic acid, - CHOH-).

$^{13}$CNMR ($D_2O$, 100MHz): 19.7 (Lactic acid, $-\underline{C}H_3$), 22.6 (y-Aminobutyric acid, - $CH_2\underline{C}H_2CH_2-$), 32.0 (y-Aminobutyric acid, $-\underline{C}H_2NH_2$), 38.9 (y-Aminobutyric acid, - $\underline{C}H_2COOH$), 67.6 (Lactic acid, -CHOH-), 178.7 (y-Aminobutyric acid, COOH), 180.9 (Lactic acid, COOH).

**<Working examples 135, 136, 155, 156, 175, 176, 192, 193, 209, 210, 226, 227, 243, 244, 260, 261, 277, 278, 294, 295, 311, 312, 328, 329 , 348, 349, 365, 366, 382, 383 > Formulations (salts) 135, 136, 155, 156, 175, 176, 192, 193, 209, 210, 226, 227, 243, 244, 260, 261, 277, 278, 294, 295, 311, 312, 328, 329, 348, 349, 365, 366, 382, 383**

Similarly, the components (A) and (B) were used in the compounding ratio as listed in Tables 1B and 1C prepared under the same condition as in Working example 176 to obtain formulations (salts) 135, 136, 155, 156, 175 , 176, 192, 193, 209, 210, 226, 227, 243, 244, 260, 261, 277, 278, 294, 295, 311, 312, 328, 329, 348, 349, 365, 366, 382 and 383. Spectroscopy data for representative formulations (salts) are listed below.

**Formulation (salt) 175**

FT-IR (KBr): 3378 $cm^{-1}$, 2971 $cm^{-1}$, 1672 $cm^{-1}$, 1157 $cm^{-1}$, 904 $cm^{-1}$.

$^1$HNMR ($D_2O$, 400MHz): 1.75-1.85 (quin, 2H, γ-Aminobutyric acid, $-CH_2CH_2CH_2-$), 2.27-2.35 (t, 2H, γ-Aminobutyric acid, $-CH_2NH_2$), 2.90-2.94 (t, 2H, γ-Aminobutyric acid, - $CH_2COOH$).

3.20-3.70 (m, 5H), 4.00 (s, 1H): (Gluconic acid,

$^{13}$CNMR ($D_2O$, 100MHz): 22.6 (y-Aminobutyric acid, $-CH_2CH_2CH_2-$), 32.1 (y-Aminobutyric acid, $-CH_2NH_2$), 38.9 (y-Aminobutyric acid, $-CH_2COOH$), 177.7 (gluconic acid, COOH), 178.8 (y-Aminobutyric acid, COOH).

62.6, 70.8, 71.1, 72.2, 73.5 (Gluconic acid, $HOCH_2CH(OH)CH(OH)CH(OH)CH(OH)COOH$).

**Formulation (salt) 243**

FT-IR (KBr): 3385 $cm^{-1}$, 2958 $cm^{-1}$, 1684 $cm^{-1}$, 1162 $cm^{-1}$, 833 $cm^{-1}$.

$^1$HNMR ($D_2O$, 400MHz): 3.47-3.52 (qd, 2H, Ser, $-CH_2OH$), 3.93-3.95 (t, 1H, Ser, - $CH(NH_2)COOH$).

3.59-3.81 (m, 5H), 4.22 (s, 1H): (Gluconic acid, $HOCH_2CH(OH)CH(OH)CH(OH)CH(OH)COOH$).

$^{13}$CNMR ($D_2O$, 100MHz): 56.0 (Ser, $-CH_2OH$), 59.9 (Ser, $-CH(NH_2)COOH$), 172.0 (Ser, COOH), 176.4 (Gluconic acid, COOH).

60.0, 62.5, 70.6, 71.0, 72.5 (Gluconic acid, $HOCH_2CH(OH)CH(OH)CH(OH)CH(OH)COOH$).

**Formulation (salt) 348**

FT-IR (KBr): 3404 $cm^{-1}$, 1685 $cm^{-1}$, 1156 $cm^{-1}$, 899 $cm^{-1}$.

$^1$HNMR ($D_2O$, 400MHz): 3.53-3.68 (m, 4H), 4.00-4.06 (d, 2H), 4.22 (s, 1H): (Gluconic acid, $HOCH_2CH(OH)CH(OH)CH(OH)CH(OH)COOH$).

1.89-2.27 (m, 4H), 3.19-3.34 (m, 2H) 3.98-4.06 (t, 1H): (Pro, $CH_{2\ hetero}$, $CH_{\ hetero}$). $^{13}$CNMR ($D_2O$, 100MHz): 174.4 (Pro, COOH), 176.2 (Gluconic acid, COOH).

62.6, 70.6, 70.8, 71.0, 72.5 (Gluconic acid, $HOCH_2CH(OH)CH(OH)CH(OH)CH(OH)COOH$).

23.6, 28.9, 46.0, 61.0: (Pro, $CH_{2\ hetero}$, $CH_{\ hetero}$).

**Formulation (salt) 349**

FT-IR (KBr): 3404 $cm^{-1}$, 1685 $cm^{-1}$, 1156 $cm^{-1}$, 899 $cm^{-1}$.

$^1$HNMR ($D_2O$, 400MHz): 1.22-1.24 (d, 3H, Lactic acid, $-CH_3$), 4.06-4.11 (q, 1H, Lactic acid, -CHOH-).

1.89-2.27 (m, 4H), 3.19-3.34 (m, 2H), 4.00-4.06 (t, 1H): (Pro, $CH_{2\ hetero}$, $CH_{\ hetero}$). $^{13}$CNMR ($D_2O$, 100MHz): 19.7 (Lactic acid, $-CH_3$), 67.6 (Lactic acid, -CHOH-),174.4 (Pro, COOH), 180.9 (Lactic acid, COOH).

23.6, 28.9, 46.0, 61.0: (Pro, $CH_{2\ hetero}$, $CH_{\ hetero}$).

**<Working example 157> Formulation (salt) 157**

L-alanine (8.91 g, 0.10 mol) and adipic acid (14.61 g, 0.10 mol) were stirred in 50 mL of water at room temperature for 1 hour, and then the water was evaporated under reduced pressure to obtain a white solid formulation (salt) of arginine and adipic acid.

**Formulation (salt) 157**

FT-IR (KBr): 3076 $cm^{-1}$, 2692 $cm^{-1}$, 1735 $cm^{-1}$, 1693 $cm^{-1}$, 1475 $cm^{-1}$, 1025 $cm^{-1}$.

$^1$HNMR ($D_2O$, 400MHz): 1.35-1.37 (m, 4H, Adipic acid, $-CH_2-$), 1.50-1.53 (d, 3H, Ala, - $CH_3$), 2.27-2.30 (m, 4H, Adipic acid, $-CH_2COOH$), 3.64-3.71 (q, 1H, Ala, $-CH(NH_2)COOH$). $^{13}$CNMR ($D_2O$, 100MHz): 16.0 (Ala, $-CH_3$), 23.7 (Adipic acid, $-CH_2-$), 33.6 (Adipic acid, - $CH_2COOH$), 50.4 (Ala, $-CH(NH_2)COOH$), 175.6 (Ala, COOH), 178.9 (Adipic acid, COOH).

**<Working examples 137-139, 157-159, 177-179, 194-196, 211-213, 228-230, 245-247, 262-264, 279-281, 296-298, 313-315, 330-332, 350-352, 367-369, 384-386> Formulations (salts) 137-139, 157-159, 177-179, 194-196, 211-213, 228-230, 245-247, 262-264, 279-281, 296-298, 313-315, 330-332, 350-352, 367-369, 384-386**

Similarly, the components (A) and (B) were used in the compounding ratio as listed in Tables 1B and 1C and prepared under the same condition as in Working example 157 to obtain formulations (salts) 137 to 139, 157 to 159, 177 to 179, 194-196, 211-213, 228-230, 245-247, 262-264, 279-281, 296-298, 313-315, 330-332, 350-352, 367-369,

384-386.

**<Working example 160>> Formulation (salt) 157**

L-alanine (8.91 g, 0.10 mol) and fumaric acid (11.60 g, 0.10 mol) were stirred in 50 mL of water at room temperature for 1 hour, and then the water was evaporated under reduced pressure to obtain a white solid formulation (salt) of arginine and adipic acid.

**Formulation (salt) 160**

FT-IR (KBr): 3081 $cm^{-1}$, 1735 $cm^{-1}$, 1588 $cm^{-1}$, 1025 $cm^{-1}$, 658 $cm^{-1}$.
$^1$HNMR ($D_2O$, 400MHz): 1.39-1.43 (d, 3H, Ala, $-CH_3$), 3.76-3.82 (q, 1H, Ala, $CH(NH_2)COOH$), 6.71 (s, 2H, Fumaric acid, -CH=CH-).
$^{13}$CNMR ($D_2O$, 100MHz): 15.5 (Ala, $-CH_3$), 50.3 (Ala, $-CH(NH_2)COOH$), 134.6 (Fumaric acid, -CH=CH-), 169.7 (Fumaric acid, COOH), 174.8 (Ala, COOH).

**<Working examples 140, 160, 180, 197, 214, 231, 248, 265, 282, 299, 316, 333, 353, 370, 387> Formulations (salts) 140, 160, 180, 197, 214, 231, 248, 265, 282, 299, 316, 333, 353, 370, 387**

Similarly, the components (A) and (B) were used in the compounding ratio as listed in Tables 1B and 1C prepared under the same condition as in Working example 160 to obtain formulations (salts) 140, 160, 180, 197, 214, 231, 248, 265, 282, 299, 316, 333, 353, 370, 387.

**<Working example 182>> Formulation (salt) 182>**

L-aminobutyric acid (10.31 g, 0.10 mol) and citric acid (19.21 g, 0.10 mol) were stirred in 50 mL of water at room temperature for 3 hour, and then the water was evaporated under reduced pressure to obtain a colorless liquid. The obtained liquid was cleaned to obtain a formulation (salt) of colorless liquid of γ-aminobutyric acid and citric acid.

**Formulation (salt) 182**

FT-IR (KBr): 3441 $cm^{-1}$, 3207 $cm^{-1}$, 1708 $cm^{-1}$, 1213 $cm^{-1}$, 844 $cm^{-1}$.
$^1$HNMR ($D_2O$, 400MHz): 1.72-1.85 (quin, 2H, γ-Aminobutyric acid, $-CH_2CH_2CH_2-$), 2.32-2.36 (t, 2H, γ-Aminobutyric acid, $-CH_2NH_2$), 2.63-2.66 (d, 2H, Citric acid, - $CH_2COOH$), 2.77-2.80 (d, 2H, Citric acid, $-CH_2COOH$), 2.89-2.92 (t, 2H, γ-Aminobutyric acid, $-CH_2COOH$).
$^{13}$CNMR ($D_2O$, 100MHz): 22.2 (y-Aminobutyric acid, $-CH_2CH_2CH_2-$), 31.2 (y-Aminobutyric acid, $-CH_2NH_2$), 38.8 (y-Aminobutyric acid, $-CH_2COOH$), 43.5 (Citric acid, $-CH_2COOH$), 73.7 (Citric acid, -C(OH)COOH), 174.4 (Citric acid, $-CH_2COOH$), 177.7 (y-Aminobutyric acid, COOH), 178.3 (Citric acid, -C(OH)(COOH)-).

**<Working examples 141-143, 161-163, 181-183, 198-200, 215-217, 232-234, 249-251, 266-268, 283-285, 300-302, 317-319, 334-336 , 354-256, 371-373, 388-390> Formulations (salts) 141-143, 161-163, 181-183, 198-200, 215-217, 232-234, 249-251, 266-268, 283-285, 300-302, 317-319, 334-336, 354-256, 371-373, 388-390**

Similarly, the components (A) and (B) were used in the compounding ratio as listed in Tables 1B and 1C and prepared under the same condition as in Working example 182 to obtain formulations (salts) 141 to 143, 161 to 163, 181 to 183, 198-200, 215-217, 232-234, 249-251, 266-268, 283-285, 300-302, 317-319, 334-336, 354-256, 371-373, 388-390. Spectroscopy data for representative formulations (salts) are listed below.

**Formulation (salt) 142**

FT-IR (KBr): 3450 $cm^{-1}$, 3171 $cm^{-1}$, 1713 $cm^{-1}$, 1215 $cm^{-1}$, 950 $cm^{-1}$.
$^1$HNMR ($D_2O$, 400MHz): 2.66-2.70 (d, 2H, Citric acid, $-CH_2COOH$), 2.82-2.86 (d, 2H, Citric acid, $-CH_2COOH$), 3.52 (s, 2H, Gly, $CH_2(NH_2)COOH$).
$^{13}$CNMR ($D_2O$, 100MHz): 40.9 (Gly, $CH_2(NH_2)COOH$), 43.3 (Citric acid, $-CH_2COOH$), 73.4 (Citric acid, -C(OH)COOH), 171.5 (Gly, $CH_2(NH_2)COOH$), 173.7 (Citric acid, - $CH_2COOH$), 177.3 (Citric acid, -C(OH)(COOH)-).

**Formulation (salt) 249**

FT-IR (KBr): 3423 $cm^{-1}$, 3228 $cm^{-1}$, 1715 $cm^{-1}$, 1236 $cm^{-1}$, 854 $cm^{-1}$.

$^1$HNMR ($D_2O$, 400MHz): 2.68-2.81 (qd, 2H, Ser, -$CH_2OH$), 3.78-3.80 (t, 1H, Ser, - CH($NH_2$)COOH), 3.85-3.86 (d, 2H, Malic acid, -$CH_2COOH$), 4.43-4.46 (t, 1H, Malic acid, -CH(OH)COOH).

$^{13}$CNMR ($D_2O$, 100MHz): 38.6 (Malic acid, -$CH_2COOH$), 56.0 (Ser, -$CH_2OH$), 59.9 (Ser, - CH($NH_2$)COOH), 67.0 (Malic acid, -CH(OH)COOH), 171.8 (Ser, COOH), 174.7 (Malic acid, $CH_2COOH$), 177.0 (Malic acid, -CH(OH)COOH).

**Formulation (salt) 250**

FT-IR (KBr): 3477 $cm^{-1}$, 3211 $cm^{-1}$, 1717 $cm^{-1}$, 1217 $cm^{-1}$, 845 $cm^{-1}$.

$^1$HNMR ($D_2O$, 400MHz): 2.71-2.74 (d, 2H, Citric acid, -$CH_2COOH$), 2.87-2.91 (d, 2H, Citric acid, -$CH_2COOH$), 3.82-3.83 (t, 1H, Ser, -CH($NH_2$)COOH), 3.66-3.80 (d, 2H, Ser, - $CH_2OH$).

$^{13}$CNMR ($D_2O$, 100MHz): 43.3 (Citric acid, -$CH_2COOH$), 55.8 (Ser, -$CH_2OH$), 59.9 (Ser, -CH($NH_2$)COOH), 73.4 (Citric acid, -C(OH)(COOH)-), 171.7 (Ser, COOH), 173.4 (Citric acid, -$CH_2COOH$), 177.2 (Citric acid, -C(OH)(COOH)-).

**Formulation (salt) 354**

FT-IR (KBr): 3424 $cm^{-1}$, 3203 $cm^{-1}$, 1775 $cm^{-1}$, 1126 $cm^{-1}$, 807 $cm^{-1}$.

$^1$HNMR ($D_2O$, 400MHz): 2.68-2.82 (m, 2H, Malic acid, -$CH_2COOH$), 4.43-4.46 (t, 1H, Malic acid, -CH(OH)COOH).

1.87-2.28 (m, 4H), 3.18-3.31 (m, 2H), 4.03-4.07 (t, 1H): (Pro, $CH_{2\ hetero}$, $CH_{hetero}$). $^{13}$CNMR ($D_2O$, 100MHz): 38.6 (Malic acid, -$CH_2$-), 67.0 (Malic acid, -CHOH-), 174.1(Malic acid, -$CH_2COOH$), 174.6 (Pro, COOH), 176.8 (Malic acid, -CH(OH)COOH). 23.6, 28.9, 46.0, 61.0: (Pro, $CH_{2\ hetero}$, $CH_{hetero}$).

**Formulation (salt) 355**

FT-IR (KBr): 3449 $cm^{-1}$, 3203 $cm^{-1}$, 1716 $cm^{-1}$, 1218 $cm^{-1}$, 845 $cm^{-1}$.

$^1$HNMR ($D_2O$, 400MHz): 2.71-2.75 (d, 2H, Citric acid, -$CH_2COOH$), 2.88-2.92 (d, 2H, Citric acid, -$CH_2COOH$).

1.86-2.29 (m, 4H), 3.19-3.34 (m, 2H), 4.05-4.09 (t, 1H): (Pro, $CH_{2\ hetero}$, $CH_{hetero}$). $^{13}$CNMR ($D_2O$, 100MHz): 43.3 (Citric acid, -$CH_2$-), 73.4 (Citric acid, -C(OH)(COOH)-), 173.6 (Citric acid, -$CH_2COOH$), 174.0 (Pro, COOH), 177.1(Citric acid, -C(OH)(COOH)-). 23.6, 28.9, 46.0, 61.0: (Pro, $CH_{2\ hetero}$, $CH_{hetero}$).

**<Working example 184> Formulation (salt) 184**

γ-aminobutyric acid (10.31 g, 0.10 mol) and benzoic acid (12.12.01, 0.10 mol) were stirred in 50 mL of water at room temperature for 3 hour, and then the water was evaporated under reduced pressure to obtain a white solid formulation (salt) of γ-aminobutyric acid and benzoic acid.

**Formulation (salt) 184**

FT-IR (KBr): 2953 $cm^{-1}$, 1752 $cm^{-1}$, 1518 $cm^{-1}$, 1388 $cm^{-1}$, 1282 $cm^{-1}$.

$^1$HNMR ($D_2O$, 400MHz): 1.76-1.83 (quin, 2H, γ-Aminobutyric acid, -$CH_2CH_2CH_2$-), 2.23-2.27 (t, 2H, γ-Aminobutyric acid, -$CH_2NH_2$), 2.89-2.93 (d, 2H, citric acid, -$CH_2COOH$), 7.37-7.41 (d, 2H, Benzoic acid, Ph), 7.47-7.50 (t, 1H, Benzoic acid, Ph), 7.81-7.83 (t, 2H, Benzoic acid, Ph).

$^{13}$CNMR ($D_2O$, 100MHz): 23.1 (γ-Aminobutyric acid, -$CH_2CH_2CH_2$-), 33.2 (γ-Aminobutyric acid, -$CH_2NH_2$), 39.1 (γ-Aminobutyric acid, -$CH_2COOH$), 128.9, 129.1, 131.8, 134.1 (Benzoic acid, Ph), 174.2 (γ-Aminobutyric acid, COOH), 180.2 (Benzoic acid, COOH).

**<Working examples 144, 164, 184, 201, 218, 235, 252, 269, 286, 303, 320, 337, 357, 374, 391> Formulations (salts) 144, 164, 184, 201, 218, 235, 252, 269, 286, 303, 320, 337, 357, 374, 391**

Similarly, the components (A) and (B) were used in the compounding ratio as listed in Tables 1B and 1C prepared under the same condition as in Working example 184 to obtain formulations (salts) 144, 164, 184, 201, 218 , 235, 252, 269, 286, 303, 320, 337, 357, 374 and 391.

**<Working example 165> Formulation (salt) 165**

L-alanine (8.91 g, 0.10 mol) and L-ascorbic acid (17.61 g, 0.10 mol) were stirred in 50 mL of water at room temperature for 1 hour, and then the water was evaporated under reduced pressure to obtain a white solid formulation (salt) of L-alanine and L-ascorbic acid.

**Formulation (salt) 165**

FT-IR (KBr): 3157 $cm^{-1}$, 1791 $cm^{-1}$, 1591 $cm^{-1}$, 1110 $cm^{-1}$.
$^1$HNMR ($D_2O$, 400MHz): 1.37-1.39 (d, 3H, Ala, $-CH_3$), 3.95-3.99 (q, 1H, Ala, - $C\underline{H}(NH_2)COOH$).
3.64-3.74 (m, 3H), 4.81 (s, 1H): (Ascorbic acid, $C\underline{H}_{arom}$, $-C\underline{H}(OH)CH_2OH$).
$^{13}$CNMR ($D_2O$, 100MHz): 15.6 (Ala, $-CH_3$), 50.5 (Ala, $-\underline{C}H(NH_2)COOH$), 175.4 (Ala, COOH).
62.3, 68.5, 76.1, 117.2, 157.8, 173.8: (Ascorbic acid, $\underline{C}H_{cyclo}$, $-\underline{C}H(OH)\underline{C}H_2OH$).

**<Working examples 145, 146, 165, 166, 185, 186, 202, 203, 219, 220, 236, 237, 253, 254, 270, 271, 287, 288, 304, 305, 321, 322, 338, 339 , 358, 359, 375, 376, 392, 393 > Formulations (salts) 145, 146, 165, 166, 185, 186, 202, 203, 219, 220, 236, 237, 253, 254, 270, 271, 287, 288, 304, 305, 321, 322, 338, 339, 358, 359, 375, 376, 392, 393**

Similarly, the components (A) and (B) were used in the compounding ratio as listed in Tables 1B and 1C prepared under the same condition as in Working example 165 to obtain formulations (salts) 145, 146, 165, 166, 185, 186, 202, 203, 219, 220, 236, 237, 253, 254, 270, 271, 287, 288, 304, 305, 321, 322, 338, 339, 358, 359, 375, 376, 392 and 393.

**<Working example 414> Formulation (salt) 414**

L-glutamic acid (14.71 g, 0.10 mol) and hexanoic acid (11.61 g, 0.10 mol) were stirred in 50 mL of water at room temperature for 1 hour, and then the water was evaporated under reduced pressure to obtain a white solid formulation (salt) of L-glutamic acid and hexanoic acid.

**Formulation (salt) 414**

FT-IR (KBr): 3197 $cm^{-1}$, 1685 $cm^{-1}$, 1509 $cm^{-1}$, 1271 $cm^{-1}$, 817 $cm^{-1}$.
$^1$HNMR ($D_2O$, 400MHz): 0.75-0.78 (t, 3H, Hexanoic acid, $-CH_3$), 1.18-1.22 (m, 4H, Hexanoic acid, $-CH_2-$), 1.48-1.52 (m, 2H, Hexanoic acid, $-CH_2-$), 2.01-2.08 (m, 2H, Glu, - $CH_2-$), 2.25-2.29 (t, 2H, Hexanoic acid, $-C\underline{H}_2COOH$), 2.44-2.48 (m, 2H, Glu, $-CH_2COOH$), 3.69-3.72 (t, 1H, Glu, $-C\underline{H}(NH_2)COOH$).
$^{13}$CNMR ($D_2O$, 100MHz): 21.6 (Hexanoic acid), 24.0 (Hexanoic acid), 25.5 (Glu, $-\underline{C}H_2-$), 29.9 (Glu, $-\underline{C}H_2COOH$), 31.1, 33.8, (Hexanoic acid), 54.0 (Glu, $\underline{C}H(NH_2)COOH$), 173.7 (Glu, $-CH(NH_2)\underline{C}OOH$), 177.0 (Glu, COOH), 179.5 (Hexanoic acid, COOH).

**<Working examples 394-398, 411-415> Formulations (salts) 394-398, 411-415**

Similarly, the components (A) and (B) were used in the compounding ratio as listed in Table 1D and prepared under the same condition as in Working example 414 to obtain formulations (salts) 394-398 and 411-415.

**<Working example 416> Formulation (salt) 416**

L-glutamic acid (14.71 g, 0.10 mol) and 50wt.% of gluconic acid (38.32 g, 0.10 mol) were stirred in 50 mL of water at room temperature for 1 hour, and then the water was evaporated under reduced pressure to obtain a white solid formulation (salt) of L-glutamic acid and gluconic acid.

**Formulation (salt) 416**

FT-IR (KBr): 3411 $cm^{-1}$, 3065 $cm^{-1}$, 1734 $cm^{-1}$, 1514 $cm^{-1}$, 1353 $cm^{-1}$, 1127 $cm^{-1}$. $^1$HNMR ($D_2O$, 400MHz): 2.01-2.14 (m, 2H, Glu, $-CH_2-$), 2.47-2.52 (m, 2H, Glu, - $CH_2COOH$), 3.69-3.80 (t, 1H, Glu, $-C\underline{H}(NH_2)COOH$).
3.53-3.68 (m, 4H), 4.03-4.05 (d, 1H), 4.31-4.34 (s, 1H): (Gluconic acid, $HOCH_2C\underline{H}(OH)C\underline{H}(OH)C\underline{H}(OH)C\underline{H}(OH)COOH$).
$^{13}$CNMR ($D_2O$, 100MHz): 25.4 (Glu, $-\underline{C}H_2-$), 29.8 (Glu, $-\underline{C}H_2COOH$), 53.7 (Glu, $\underline{C}H(NH_2)COOH$), 173.6 (Glu, $CH(NH_2)\underline{C}OOH$), 176.3 (Gluconic acid, COOH), 176.9 (Glu, COOH).

62.3, 70.6, 71.0, 72.5, 79.6 (Gluconic acid, $HOCH_2CH(OH)CH(OH)CH(OH)CH(OH)COOH$).

**<Working examples 399, 400, 416, 417> Formulations (salts) 399, 400, 416, 417**

Similarly, the components (A) and (B) were used in the compounding ratio as listed in Table 1D and prepared under the same condition as in Working example 416 to obtain formulations (salts) 399, 400, 416, and 417.

**<Working example 418> Formulation (salt) 418**

L-glutamic acid (14.71 g, 0.10 mol) and adipic acid (14.61 g, 0.10 mol) were stirred in 50 mL of water at room temperature for 1 hour, and then the water was evaporated under reduced pressure to obtain a white solid formulation (salt) of L-glutamic acid and adipic acid.

**Formulation (salt) 418**

FT-IR (KBr): 3031 $cm^{-1}$, 2951 $cm^{-1}$, 1756 $cm^{-1}$, 1692 $cm^{-1}$, 1508 $cm^{-1}$, 1256 $cm^{-1}$. $^1$HNMR ($D_2O$, 400MHz): 1.47-1.50 (m, 4H, Adipic acid, $-CH_2-$), 1.97-2.11 (m, 2H, Glu, - $CH_2$-), 2.27-2.31 (t, 4H, Adipic acid, $-CH_2COOH$), 2.42-2.47 (m, 2H, Glu, $-CH_2COOH$), 3.68-3.72 (t, 1H, Glu, $-CH(NH_2)COOH$).

$^{13}$CNMR ($D_2O$, 100MHz): 23.6 (Adipic acid, $-CH_2-$), 25.5 (Glu, $-CH_2-$), 29.9 (Glu, $CH_2COOH$), 33.4 (Adipic acid, $-CH_2COOH$), 53.8 (Glu, $-CH(NH_2)COOH$), 173.7 (Glu, - $CH(NH_2)COOH$), 177.0 (Glu, COOH), 178.7 (Adipic acid, COOH).

**<Working examples 401-403, 418-420> Formulations (salts) 401-403, 418-420**

Similarly, the components (A) and (B) were used in the compounding ratio as listed in Table 1D and prepared under the same condition as in Working example 418 to obtain formulations (salts) 401-403 and 418-420.

**<Working example 421> Formulation (salt) 421**

L-glutamic acid (14.71 g, 0.10 mol) and fumaric acid (11.61 g, 0.10 mol) were stirred in 50 mL of water at room temperature for 1 hour, and then the water was evaporated under reduced pressure to obtain a white solid formulation (salt) of L-glutamic acid and fumaric acid.

**Formulation (salt) 421**

FT-IR (KBr): 3040 $cm^{-1}$, 1748 $cm^{-1}$, 1510 $cm^{-1}$, 1444 $cm^{-1}$, 727 $cm^{-1}$.

$^1$HNMR ($D_2O$, 400MHz): 1.98-2.12 (m, 2H, Glu, $-CH_2-$), 2.44-2.49 (m, 2H, Glu, - $CH_2COOH$), 3.73-3.77 (t, 1H, Glu, $-CH(NH_2)COOH$), 6.69 (s, 2H, Fumaric acid, -CH=CH-).

$^{13}$CNMR ($D_2O$, 100MHz): 25.3 (Glu, $-CH_2-$), 29.7 (Glu, $-CH_2COOH$), 53.5 (Glu, - $CH(NH_2)COOH$), 134.2 (Fumaric acid, -CH=CH-), 169.6 (Fumaric acid, COOH), 173.4 (Glu, $-CH(NH_2)COOH$), 176.7 (Glu, COOH).

**<Working examples 404 and 421> Formulations (salts) 404, 421**

Similarly, the components (A) and (B) were used in the compounding ratio as listed in Table 1D and prepared under the same condition as in Working example 421 to obtain formulations (salts) 404 and 421.

**<Working example 422> Formulation (salt) 422**

L-glutamic acid (14.71 g, 0.10 mol) and L-malic acid (13.41 g, 0.10 mol) were stirred in 50 mL of water at room temperature for 1 hour, and then the water was evaporated under reduced pressure to obtain a white solid formulation (salt) of L-glutamic acid and L-malic acid.

**Formulation (salt) 422**

FT-IR (KBr): 3041 $cm^{-1}$, 1722 $cm^{-1}$, 1511 $cm^{-1}$, 1257 $cm^{-1}$, 1102 $cm^{-1}$.

$^1$HNMR ($D_2O$, 400MHz): 2.10-2.16 (m, 2H, Glu, $-CH_2-$), 2.48-2.52 (m, 2H, Glu, - $CH_2COOH$), 2.72-2.86 (m, 2H, Malic acid, $-CH_2COOH$), 3.77-3.80 (t, 1H, Glu, - $CH(NH_2)COOH$), 4.47-4.50 (t, 1H, Malic acid, -CH(OH)COOH).

$^{13}$CNMR ($D_2O$, 100MHz): 25.3 (Glu, $-CH_2-$), 29.8 (Glu, $-CH_2COOH$), 38.7 (Malic acid, - $CH_2COOH$), 53.9 (Glu, $-CH(NH_2)COOH$), 67.1 (Malic acid, -CH(OH)COOH), 173.4 (Glu, $-CH(NH_2)COOH$), 174.7 (Malic acid $-CH_2COOH$),

176.6 (Glu, COOH), 176.9 (Malic acid, -CH(OH)COOH).

<Working examples 405-407 and 422-424> Formulations (salts) 405-407 and 422-424

Similarly, the components (A) and (B) were used in the compounding ratio as listed in Table 1D and prepared under the same condition as in Working example 422 to obtain formulations (salts) 405-407 and 422-424.

**<Working example 425> Formulation (salt) 425**

L-glutamic acid (14.71 g, 0.10 mol) and benzoic acid (12.12 g, 0.10 mol) were stirred in 50 mL of water at room temperature for 1 hour, and then the water was evaporated under reduced pressure to obtain a white solid formulation (salt) of L-glutamic acid and benzoic acid.

**Formulation (salt) 425**

FT-IR (KBr): 3030 $cm^{-1}$, 1637$cm^{-1}$, 1509 $cm^{-1}$, 1258 $cm^{-1}$.
$^1$HNMR ($D_2O$, 400MHz): 2.04-2.14 (m, 2H, Glu, -$CH_2$-), 2.47-2.51 (m, 2H, Glu, - $CH_2$COOH), 3.72-3.75 (t, 1H, Glu, -CH($NH_2$)COOH), 7.46-7.50 (t, 2H, Benzoic acid, Ph), 7.60-7.62 (t, 1H, Benzoic acid, Ph), 7.95-7.97 (d, 2H, Benzoic acid, Ph).
$^{13}$CNMR ($D_2O$, 100MHz): 25.5 (Glu, -$CH_2$-), 30.1 (Glu, -$CH_2$COOH), 53.6 (Glu, - CH($NH_2$)COOH), 128.1, 128.9, 129.5, 129.7 (Benzoic acid, Ph), 173.8 (Glu, - CH($NH_2$)COOH), 175.6 (Benzoic acid, COOH), 177.1 (Glu, COOH).

**<Working examples 408, 425> Formulations (salts) 408, 425**

Similarly, the components (A) and (B) were used in the compounding ratio as listed in Table 1D and prepared under the same condition as in Working example 425 to obtain formulations (salts) 408 and 425.

**<Working example 426> Formulation (salt) 426**

L-glutamic acid (14.71 g, 0.10 mol) and L-ascorbic acid (17.61 g, 0.10 mol) were stirred in 50 mL of water at room temperature for 1 hour, and then the water was evaporated under reduced pressure to obtain a white solid formulation (salt) of L-glutamic acid and L-ascorbic acid.

**Formulation (salt) 426**

FT-IR (KBr): 3055 $cm^{-1}$, 1805 $cm^{-1}$, 1509 $cm^{-1}$, 1268 $cm^{-1}$, 815 $cm^{-1}$.
$^1$HNMR ($D_2O$, 400MHz): 1.96-2.10 (m, 2H, Glu, -$CH_2$-), 2.42-2.47 (m, 2H, Glu, - $CH_2$COOH), 3.72-3.75(m, 1H, Glu, -CH($NH_2$)COOH).
3.62-3.97 (m, 3H), 4.82 (s, 1H): (Ascorbic acid, $CH_{cyclo}$, -CH(OH)$CH_2$OH).
$^{13}$CNMR ($D_2O$, 100MHz): 25.4 (Glu, -$CH_2$-), 29.9 (Glu, -$CH_2$COOH), 53.7 (Glu, - CH($NH_2$)COOH), 173.7 (Glu, -CH($NH_2$)COOH), 176.9 (Glu, COOH).
62.0, 68.9, 76.3, 117.4, 173.6: (Ascorbic acid, $CH_{cyclo}$, -CH(OH)$CH_2$OH).

**<Working examples 409, 410, 426 and 427> Formulations (organic salts) 409, 410, 426 and 427**

Similarly, the components (A) and (B) were used in the compounding ratio as listed in Table 1D and prepared under the same condition as in Working example 426 to obtain formulations (salts) 409, 410, 426 and 427.

**2. Appearance of formulation (organic salt)**

Formulations 1 to 427 shown in Tables 1A to 1D were prepared by the above-described method using the components (A) and (B) at the compounding molar ratios shown in the tables, and their states at 25°C were observed.

[Table 1A-1]

| Component (A) / Component (B) | | Amino acids (values in parentheses are isoelectric points) | | | | | |
|---|---|---|---|---|---|---|---|
| | | L-lysine (9.75) | | | | | |
| | | Compounding molar ratio (Component (A) : Component (B)) | | | | | |
| | | 1:1 | | 1:2 | | 3:2 | |
| Saturated aliphatic monocarboxylic acid | Formic acid | Working example 1 | Formulation 1 : Solid | Working example 22 | Formulation 22 : Solid | — | — |
| | Acetic acid | Working example 2 | Formulation 2 : Solid | Working example 23 | Formulation 23 : Solid | — | — |
| | Propionic acid | Working example 3 | Formulation 3 : Solid | Working example 24 | Formulation 24 : Solid | — | — |
| | Hexanoic acid | Working example 4 | Formulation 4 : Solid | Working example 25 | Formulation 25 : Solid | — | — |
| | Butyric acid | Working example 5 | Formulation 5 : Solid | Working example 26 | Formulation 26 : Solid | — | — |
| | Isostearic acid | Working example 6 | Formulation 6 : Solid | Working example 27 | Formulation 27 : Solid | — | — |
| Unsaturated aliphatic carboxylic acid | Oleic acid | Working example 7 | Formulation 7 : Solid | Working example 28 | Formulation 28 : Solid | — | — |
| | Linoleic acid | Working example 8 | Formulation 8 : Solid | Working example 29 | Formulation 29 : Solid | — | — |
| Saturated hydroxy monocarboxylic acid | Gluconic acid | Working example 9 | Formulation 9 : Liquid | Working example 30 | Formulation 30 : Liquid | — | — |
| | Lactic acid | Working example 10 | Formulation 10 : Solid | Working example 31 | Formulation 31 : Solid | — | — |
| Saturated aliphatic dicarboxylic acid | Adipic acid | Working example 11 | Formulation 11 : Solid | Working example 32 | Formulation 32 : Solid | — | — |
| | succinic acid | Working example 12 | Formulation 12 : Solid | Working example 33 | Formulation 33 : Solid | — | — |
| | L-glutamic acid | Working example 13 | Formulation 13 : Solid | Working example 34 | Formulation 34 : Solid | — | — |
| Unsaturated aliphatic dicarboxylic acid | Fumaric acid | Working example 14 | Formulation 14 : Liquid | Working example 35 | Formulation 35 : Solid | — | — |
| Saturated hydroxy di- or tricarboxylic acid | L-malic acid | Working example 15 | Formulation 15 : Solid | Working example 36 | Formulation 36 : Solid | — | — |
| | Citric acid | Working example 16 | Formulation 16 : Liquid | — | — | Working example 37 | Formulation 37 : Liquid |
| | Tartaric acid | Working example 17 | Formulation 17 : Solid | Working example 38 | Formulation 38 : Solid | — | — |
| Aromatic carboxylic acid | Benzoic acid | Working example 18 | Formulation 18 : Solid | Working example 39 | Formulation 39 : Solid | — | — |
| | Cinnamic acid | Working example 19 | Formulation 19 : Solid | Working example 40 | Formulation 40 : Solid | — | — |
| Hydroxy group-containing cyclic lactone | L-ascorbic acid | Working example 20 | Formulation 20 : Solid | Working example 41 | Formulation 41 : Solid | — | — |
| | Erythorbic acid | Working example 21 | Formulation 21 : Liquid | Working example 42 | Formulation 42 : Solid | — | — |

[Table 1A-2]

| Component (B) \ Component (A) | | Amino acids (values in parentheses are isoelectric points) | | | | | |
|---|---|---|---|---|---|---|---|
| | | L-arginine (10.76) | | | | | |
| | | Compounding molar ratio (Component (A) : Component (B)) | | | | | |
| | | 1:1 | | 1:2 | | 3:2 | |
| Saturated aliphatic monocarboxylic acid | Formic acid | Working example 43 | Formulation 43 : Solid | Working example 64 | Formulation 64 : Solid | — | — |
| | Acetic acid | Working example 44 | Formulation 44 : Solid | Working example 65 | Formulation 65 : Solid | — | — |
| | Propionic acid | Working example 45 | Formulation 45 : Solid | Working example 66 | Formulation 66 : Solid | — | — |
| | Hexanoic acid | Working example 46 | Formulation 46 : Solid | Working example 67 | Formulation 67 : Solid | — | — |
| | Butyric acid | Working example 47 | Formulation 47 : Solid | Working example 68 | Formulation 68 : Solid | — | — |
| | Isostearic acid | Working example 48 | Formulation 48 : Liquid | Working example 69 | Formulation 69 : Liquid | — | — |
| Unsaturated aliphatic carboxylic acid | Oleic acid | Working example 49 | Formulation 49 : Liquid | Working example 70 | Formulation 70 : Liquid | — | — |
| | Linoleic acid | Working example 50 | Formulation 50 : Liquid | Working example 71 | Formulation 71 : Liquid | — | — |
| Saturated hydroxy monocarboxylic acid | Gluconic acid | Working example 51 | Formulation 51 : Liquid | Working example 72 | Formulation 72 : Liquid | — | — |
| | Lactic acid | Working example 52 | Formulation 52 : Liquid | Working example 73 | Formulation 73 : Liquid | — | — |
| Saturated aliphatic dicarboxylic acid | Adipic acid | Working example 53 | Formulation 53 : Solid | Working example 74 | Formulation 74 : Solid | — | — |
| | succinic acid | Working example 54 | Formulation 54 : Solid | Working example 75 | Formulation 75 : Solid | — | — |
| | L-glutamic acid | Working example 55 | Formulation 55 : Solid | Working example 76 | Formulation 76 : Solid | — | — |
| Unsaturated aliphatic dicarboxylic acid | Fumaric acid | Working example 56 | Formulation 56 : Solid | Working example 77 | Formulation 77 : Solid | — | — |
| Saturated hydroxy di- or tricarboxylic acid | L-malic acid | Working example 57 | Formulation 57 : Liquid | Working example 78 | Formulation 78 : Liquid | — | — |
| | Citric acid | Working example 58 | Formulation 58 : Liquid | — | — | Working example 79 | Formulation 79 : Liquid |
| | Tartaric acid | Working example 59 | Formulation 59 : Liquid | Working example 80 | Formulation 80 : Liquid | — | — |
| Aromatic carboxylic acid | Benzoic acid | Working example 60 | Formulation 60 : Liquid | Working example 81 | Formulation 81 : Solid | — | — |
| | Cinnamic acid | Working example 61 | Formulation 61 : Solid | Working example 82 | Formulation 82 : Solid | — | — |
| Hydroxy group-containing cyclic lactone | L-ascorbic acid | Working example 62 | Formulation 62 : Solid | Working example 83 | Formulation 83 : Solid | — | — |
| | Erythorbic acid | Working example 63 | Formulation 63 : Solid | Working example 84 | Formulation 84 : Solid | — | — |

[Table 1A-3]

| Component (A) / Component (B) | | Amino acids (values in parentheses are isoelectric points) | | | | | |
|---|---|---|---|---|---|---|---|
| | | L-histidine (7.59) | | | | | |
| | | Compounding molar ratio (Component (A) : Component (B)) | | | | | |
| | | 1:1 | | 1:2 | | 3:2 | |
| Saturated aliphatic monocarboxylic acid | Formic acid | Working example 85 | Formulation 85 : Solid | Working example106 | Formulation106 : Solid | — | — |
| | Acetic acid | Working example 86 | Formulation 86 : Solid | Working example107 | Formulation107 : Solid | — | — |
| | Propionic acid | Working example 87 | Formulation 87 : Solid | Working example108 | Formulation108 : Solid | — | — |
| | Hexanoic acid | Working example 88 | Formulation 88 : Solid | Working example109 | Formulation109 : Solid | — | — |
| | Butyric acid | Working example 89 | Formulation 89 : Solid | Working example110 | Formulation110 : Solid | — | — |
| | Isostearic acid | Working example 90 | Formulation 90 : Solid | Working example111 | Formulation111 : Solid | — | — |
| Unsaturated aliphatic carboxylic acid | Oleic acid | Working example 91 | Formulation 91 : Solid | Working example112 | Formulation112 : Solid | — | — |
| | Linoleic acid | Working example 92 | Formulation 92 : Solid | Working example113 | Formulation113 : Solid | — | — |
| Saturated hydroxy monocarboxylic acid | Gluconic acid | Working example93 | Formulation 93 : Liquid | Working example114 | Formulation114 : Liquid | — | — |
| | Lactic acid | Working example94 | Formulation 94 : Solid | Working example115 | Formulation115 : Liquid | — | — |
| Saturated aliphatic dicarboxylic acid | Adipic acid | Working example 95 | Formulation 95 : Solid | Working example116 | Formulation116 : Solid | — | — |
| | succinic acid | Working example 96 | Formulation 96 : Solid | Working example117 | Formulation107 : Solid | — | — |
| | L-glutamic acid | Working example 97 | Formulation 97 : Solid | Working example118 | Formulation108 : Solid | — | — |
| Unsaturated aliphatic dicarboxylic acid | Fumaric acid | Working example 98 | Formulation 98 : Solid | Working example119 | Formulation109 : Solid | — | — |
| Saturated hydroxy di- or tricarboxylic acid | L-malic acid | Working example 99 | Formulation 99 : Solid | Working example120 | Formulation120 : Solid | — | — |
| | Citric acid | Working example 100 | Formulation100 : Liquid | — | — | Working example121 | Formulation121 : Liquid |
| | Tartaric acid | Working example 101 | Formulation101 : Solid | Working example122 | Formulation122 : Solid | — | — |
| Aromatic carboxylic acid | Benzoic acid | Working example 102 | Formulation102 : Solid | Working example123 | Formulation123 : Solid | — | — |
| | Cinnamic acid | Working example 103 | Formulation103 : Solid | Working example124 | Formulation124 : Solid | — | — |
| Hydroxy group-containing cyclic lactone | L-ascorbic acid | Working example 104 | Formulation104 : Solid | Working example125 | Formulation125 : Solid | — | — |
| | Erythorbic acid | Working example 105 | Formulation105 : Solid | Working example126 | Formulation126 : Solid | — | — |

[Table 1B-1]

| Component (A) / Component (B) | | Amino acids (values in parentheses are isoelectric points) | | | | | |
|---|---|---|---|---|---|---|---|
| | | Glycine (5.97) | | L-alanine (6.00) | | γ-aminobutyric acid (7.85) | |
| | | Compounding molar ratio (Component (A) : Component (B)) | | | | | |
| | | 1:1 | | 1:1 | | 1:1 | |
| Saturated aliphatic monocarboxylic acid | Formic acid | Working example127 | | Working example147 | Formulation 147 : Solid | Working example167 | Formulation 167 : Solid |
| | Acetic acid | Working example128 | Formulation 128 : Solid | Working example148 | Formulation 148 : Solid | Working example168 | Formulation 168 : Solid |
| | Propionic acid | Working example129 | Formulation 129 : Solid | Working example149 | Formulation 149 : Solid | Working example169 | Formulation 169 : Solid |
| | Hexanoic acid | Working example130 | Formulation 130 : Solid | Working example150 | Formulation 150 : Solid | Working example170 | Formulation 170 : Solid |
| | Butyric acid | Working example131 | Formulation 131 : Solid | Working example151 | Formulation 151 : Solid | Working example171 | Formulation 171 : Solid |
| | Isostearic acid | Working example132 | Formulation 132 : Solid | Working example152 | Formulation 152 : Solid | Working example172 | Formulation 172 : Solid |
| Unsaturated aliphatic carboxylic acid | Oleic acid | Working example133 | Formulation 133 : Solid | Working example153 | Formulation 153 : Solid | Working example173 | Formulation173 : Liquid |
| | Linoleic acid | Working example134 | Formulation 134 : Solid | Working example154 | Formulation 154 : Solid | Working example174 | Formulation 174 : Solid |
| Saturated hydroxy monocarboxylic acid | Gluconic acid | Working example135 | Formulation 135 : Solid | Working example155 | Formulation 155 : Solid | Working example175 | Formulation175 : Liquid |
| | Lactic acid | Working example136 | Formulation 136 : Solid | Working example156 | Formulation 156 : Solid | Working example176 | Formulation176 : Liquid |
| Saturated aliphatic dicarboxylic acid | Adipic acid | Working example137 | Formulation 137 : Solid | Working example157 | Formulation 157 : Solid | Working example177 | Formulation 177 : Solid |
| | succinic acid | Working example138 | Formulation 138 : Solid | Working example158 | Formulation 158 : Solid | Working example178 | Formulation 178 : Solid |
| | L-glutamic acid | Working example139 | Formulation 139 : Solid | Working example159 | Formulation 159 : Solid | Working example179 | Formulation 179 : Solid |
| Unsaturated aliphatic dicarboxylic acid | Fumaric acid | Working example140 | Formulation 140 : Solid | Working example160 | Formulation 160 : Solid | Working example180 | Formulation 180 : Solid |
| Saturated hydroxy di- or tricarboxylic acid | L-malic acid | Working example141 | Formulation 141 : Solid | Working example161 | Formulation 161 : Solid | Working example181 | Formulation 181 : Solid |
| | Citric acid | Working example142 | Formulation142 : Liquid | Working example162 | Formulation 162 : Solid | Working example182 | Formulation182 : Liquid |
| | Tartaric acid | Working example143 | Formulation 143 : Solid | Working example163 | Formulation 163 : Solid | Working example183 | Formulation 183 : Solid |
| Aromatic carboxylic acid | Benzoic acid | Working example144 | Formulation 144 : Solid | Working example164 | Formulation 164 : Solid | Working example184 | Formulation 184 : Solid |
| | Cinnamic acid | — | — | — | — | — | — |
| Hydroxy group-containing cyclic lactone | L-ascorbic acid | Working example145 | Formulation 145 : Solid | Working example165 | Formulation 165 : Solid | Working example185 | Formulation 185 : Solid |
| | Erythorbic acid | Working example146 | Formulation 146 : Solid | Working example166 | Formulation 166 : Solid | Working example186 | Formulation 186 : Solid |

[Table 1B-2]

| Component (B) \ Component (A) | | Amino acids (values in parentheses are isoelectric points) | | | | | |
|---|---|---|---|---|---|---|---|
| | | L-valine (5.96) | | L-leucine (5.98) | | L-isoleucine (6.02) | |
| | | Compounding molar ratio (Component (A) : Component (B)) | | | | | |
| | | 1:1 | | 1:1 | | 1:1 | |
| Saturated aliphatic monocarboxylic acid | Formic acid | Working example187 | Formulation187 : Solid | Working example204 | Formulation204 : Solid | Working example221 | Formulation221 : Solid |
| | Acetic acid | Working example188 | Formulation188 : Solid | Working example205 | Formulation205 : Solid | Working example222 | Formulation222 : Solid |
| | Propionic acid | Working example189 | Formulation189 : Solid | Working example206 | Formulation206 : Solid | Working example223 | Formulation223 : Solid |
| | Hexanoic acid | Working example190 | Formulation190 : Solid | Working example207 | Formulation207 : Solid | Working example224 | Formulation224 : Solid |
| | Butyric acid | Working example191 | Formulation191 : Solid | Working example208 | Formulation208 : Solid | Working example225 | Formulation225 : Solid |
| | Isostearic acid | — | — | — | — | — | — |
| Unsaturated aliphatic carboxylic acid | Oleic acid | — | — | — | — | — | — |
| | Linoleic acid | — | — | — | — | — | — |
| Saturated hydroxy monocarboxylic acid | Gluconic acid | Working example192 | Formulation192 : Solid | Working example209 | Formulation209 : Solid | Working example226 | Formulation226 : Solid |
| | Lactic acid | Working example193 | Formulation193 : Solid | Working example210 | Formulation210 : Solid | Working example227 | Formulation227 : Solid |
| Saturated aliphatic dicarboxylic acid | Adipic acid | Working example194 | Formulation194 : Solid | Working example211 | Formulation211 : Solid | Working example228 | Formulation228 : Solid |
| | succinic acid | Working example195 | Formulation195 : Solid | Working example212 | Formulation212 : Solid | Working example229 | Formulation229 : Solid |
| | L-glutamic acid | Working example196 | Formulation196 : Solid | Working example213 | Formulation213 : Solid | Working example230 | Formulation230 : Solid |
| Unsaturated aliphatic dicarboxylic acid | Fumaric acid | Working example197 | Formulation197 : Solid | Working example214 | Formulation214 : Solid | Working example231 | Formulation231 : Solid |
| Saturated hydroxy di- or tricarboxylic acid | L-malic acid | Working example198 | Formulation198 : Solid | Working example215 | Formulation215 : Solid | Working example232 | Formulation232 : Solid |
| | Citric acid | Working example199 | Formulation199 : Solid | Working example216 | Formulation216 : Solid | Working example233 | Formulation233 : Solid |
| | Tartaric acid | Working example200 | Formulation200 : Solid | Working example217 | Formulation217 : Solid | Working example234 | Formulation234 : Solid |
| Aromatic carboxylic acid | Benzoic acid | Working example201 | Formulation201 : Solid | Working example218 | Formulation218 : Solid | Working example235 | Formulation235 : Solid |
| | Cinnamic acid | — | — | — | — | — | — |
| Hydroxy group-containing cyclic lactone | L-ascorbic acid | Working example202 | Formulation202 : Solid | Working example219 | Formulation219 : Solid | Working example236 | Formulation236 : Solid |
| | Erythorbic acid | Working example203 | Formulation203 : Solid | Working example220 | Formulation220 : Solid | Working example237 | Formulation237 : Solid |

[Table 1B-3]

| Component(B) \ Component(A) | | Amino acids (values in parentheses are isoelectric points) L-serine (5.68) | | L-threonine (6.16) | |
|---|---|---|---|---|---|
| | | Compounding molar ratio (Component (A) : Component (B)) 1:1 | | 1:1 | |
| Saturated aliphatic monocarboxylic acid | Formic acid | Working example238 | Formulation 238 : Solid | Working example255 | Formulation 255 : Solid |
| | Acetic acid | Working example239 | Formulation 239 : Solid | Working example256 | Formulation 256 : Solid |
| | Propionic acid | Working example240 | Formulation 240 : Solid | Working example257 | Formulation 257 : Solid |
| | Hexanoic acid | Working example241 | Formulation 241 : Solid | Working example258 | Formulation 258 : Solid |
| | Butyric acid | Working example242 | Formulation 242 : Solid | Working example259 | Formulation 259 : Solid |
| | Isostearic acid | — | — | — | — |
| Unsaturated aliphatic carboxylic acid | Oleic acid | — | — | — | — |
| | Linoleic acid | — | — | — | — |
| Saturated hydroxy monocarboxylic acid | Gluconic acid | Working example243 | Formulation 243 : Liquid | Working example260 | Formulation 260 : Solid |
| | Lactic acid | Working example244 | Formulation 244 : Solid | Working example261 | Formulation 261 : Solid |
| Saturated aliphatic dicarboxylic acid | Adipic acid | Working example245 | Formulation 245 : Solid | Working example262 | Formulation 262 : Solid |
| | succinic acid | Working example246 | Formulation 246 : Solid | Working example263 | Formulation 263 : Solid |
| | L-glutamic acid | Working example247 | Formulation 247 : Solid | Working example264 | Formulation 264 : Solid |
| Unsaturated aliphatic dicarboxylic acid | Fumaric acid | Working example248 | Formulation 248 : Solid | Working example265 | Formulation 265 : Solid |
| Saturated hydroxy di- or tricarboxylic acid | L-malic acid | Working example249 | Formulation 249 : Liquid | Working example266 | Formulation 266 : Solid |
| | Citric acid | Working example250 | Formulation 250 : Liquid | Working example267 | Formulation 267 : Solid |
| | Tartaric acid | Working example251 | Formulation 251 : Solid | Working example268 | Formulation 268 : Solid |
| Aromatic carboxylic acid | Benzoic acid | Working example252 | Formulation 252 : Solid | Working example269 | Formulation 269 : Solid |
| | Cinnamic acid | — | — | — | — |
| Hydroxy group-containing cyclic lactone | L-ascorbic acid | Working example253 | Formulation 253 : Solid | Working example270 | Formulation 270 : Solid |
| | Erythorbic acid | Working example254 | Formulation 254 : Solid | Working example271 | Formulation 271 : Solid |

[Table 1C-1]

| Component(A) / Component(B) | | Amino acids (values in parentheses are isoelectric points) | | | | | |
|---|---|---|---|---|---|---|---|
| | | L-aspartic acid (5.41) | | L-glutamic acid (5.65) | | L-cysteine (5.07) | |
| | | Compounding molar ratio (Component (A) : Component (B)) | | | | | |
| | | 1:1 | | 1:1 | | 1:1 | |
| Saturated aliphatic monocarboxylic acid | Formic acid | Working example272 | Formulation 272 : Solid | Working example 289 | Formulation 289 : Solid | Working example 306 | Formulation 306 : Solid |
| | Acetic acid | Working example273 | Formulation 273 : Solid | Working example 290 | Formulation 290 : Solid | Working example 307 | Formulation 307 : Solid |
| | Propionic acid | Working example274 | Formulation 274 : Solid | Working example 291 | Formulation 291 : Solid | Working example 308 | Formulation 308 : Solid |
| | Hexanoic acid | Working example275 | Formulation 275 : Solid | Working example292 | Formulation 292 : Solid | Working example 309 | Formulation 309 : Solid |
| | Butyric acid | Working example276 | Formulation 276 : Solid | Working example 293 | Formulation 293 : Solid | Working example 310 | Formulation 310 : Solid |
| | Isostearic acid | — | — | — | — | — | — |
| Unsaturated aliphatic carboxylic acid | Oleic acid | — | — | — | — | — | — |
| | Linoleic acid | — | — | — | — | — | — |
| Saturated hydroxy monocarboxylic acid | Gluconic acid | Working example277 | Formulation 277 : Solid | Working example 294 | Formulation 294 : Solid | Working example 311 | Formulation 311 : Solid |
| | Lactic acid | Working example 278 | Formulation 278 Solid | Working example 295 | Formulation 295 : Solid | Working example 312 | Formulation 312 : Solid |
| Saturated aliphatic dicarboxylic acid | Adipic acid | Working example279 | Formulation 279 : Solid | Working example 296 | Formulation 296 : Solid | Working example 313 | Formulation 313 : Solid |
| | succinic acid | Working example280 | Formulation 280 : Solid | Working example 297 | Formulation 297 : Solid | Working example 314 | Formulation 314 : Solid |
| | L-glutamic acid | Working example281 | Formulation 281 : Solid | Working example 298 | Formulation 298 : Solid | Working example 315 | Formulation 315 : Solid |
| Unsaturated aliphatic dicarboxylic acid | Fumaric acid | Working example282 | Formulation 282 : Solid | Working example 299 | Formulation 299 : Solid | Working example 316 | Formulation 316 : Solid |
| Saturated hydroxy di- or tricarboxylic acid | L-malic acid | Working example283 | Formulation 283 : Solid | Working example 300 | Formulation 300 : Solid | Working example 317 | Formulation 317 : Solid |
| | Citric acid | Working example284 | Formulation 284 : Solid | Working example 301 | Formulation 301 : Solid | Working example 318 | Formulation 318 : Solid |
| | Tartaric acid | Working example285 | Formulation 285 : Solid | Working example 302 | Formulation 302 : Solid | Working example 319 | Formulation 319 : Solid |
| Aromatic carboxylic acid | Benzoic acid | Working example286 | Formulation 286 : Solid | Working example 303 | Formulation 303 : Solid | Working example 320 | Formulation 320 : Solid |
| | Cinnamic acid | — | — | — | — | — | — |
| Hydroxy group-containing cyclic lactone | L-ascorbic acid | Working example287 | Formulation 287 : Solid | Working example 304 | Formulation 304 : Solid | Working example 321 | Formulation 321 : Solid |
| | Erythorbic acid | Working example288 | Formulation 288 : Solid | Working example 305 | Formulation 305 : Solid | Working example 322 | Formulation 322 : Solid |

[Table 1C-2]

| Component(A) / Component(B) | | Amino acids (values in parentheses are isoelectric points) | | | | | |
|---|---|---|---|---|---|---|---|
| | | L-methionine (5.74) | | L-proline (6.30) | | L-phenylalanine (5.48) | |
| | | Compounding molar ratio (Component (A) : Component (B)) | | | | | |
| | | 1:1 | | 1:1 | | 1:1 | |
| Saturated aliphatic monocarboxylic acid | Formic acid | Working example323 | Formulation 323 : Solid | Working example340 | Formulation 340 : Solid | Working example360 | Formulation 360 : Solid |
| | Acetic acid | Working example324 | Formulation 324 : Solid | Working example341 | Formulation 34 : Solid | Working example361 | Formulation 361 : Solid |
| | Propionic acid | Working example325 | Formulation 325 : Solid | Working example342 | Formulation 342 : Solid | Working example362 | Formulation 362 : Solid |
| | Hexanoic acid | Working example326 | Formulation 326 : Solid | Working example343 | Formulation 343 : Solid | Working example363 | Formulation 363 : Solid |
| | Butyric acid | Working example327 | Formulation 327 : Solid | Working example344 | Formulation 344 : Solid | Working example364 | Formulation 364 : Solid |
| | Isostearic acid | — | — | Working example345 | Formulation 345 : Liquid | — | — |
| Unsaturated aliphatic carboxylic acid | Oleic acid | — | — | Working example346 | Formulation 346 : Liquid | — | — |
| | Linoleic acid | — | — | Working example347 | Formulation 347 : Liquid | — | — |
| Saturated hydroxy monocarboxylic acid | Gluconic acid | Working example328 | Formulation 328 : Solid | Working example348 | Formulation 348 : Liquid | Working example365 | Formulation 365 : Solid |
| | Lactic acid | Working example329 | Formulation 329 : Solid | Working example349 | Formulation 349 : Liquid | Working example366 | Formulation 366 : Solid |
| Saturated aliphatic dicarboxylic acid | Adipic acid | Working example330 | Formulation 330 : Solid | Working example350 | Formulation 350 : Solid | Working example367 | Formulation 367 : Solid |
| | succinic acid | Working example331 | Formulation 331 : Solid | Working example351 | Formulation 351 : Solid | Working example368 | Formulation 368 : Solid |
| | L-glutamic acid | Working example332 | Formulation 332 : Solid | Working example352 | Formulation 352 : Solid | Working example369 | Formulation 369 : Solid |
| Unsaturated aliphatic dicarboxylic acid | Fumaric acid | Working example333 | Formulation 333 : Solid | Working example353 | Formulation 353 : Solid | Working example370 | Formulation 370 : Solid |
| Saturated hydroxy di- or tricarboxylic acid | L-malic acid | Working example334 | Formulation 334 : Solid | Working example354 | Formulation 354 : Liquid | Working example371 | Formulation 371 : Solid |
| | Citric acid | Working example335 | Formulation 335 : Solid | Working example355 | Formulation 355 : Liquid | Working example372 | Formulation 372 : Solid |
| | Tartaric acid | Working example336 | Formulation 336 : Solid | Working example356 | Formulation 356 : Solid | Working example373 | Formulation 373 : Solid |
| Aromatic carboxylic acid | Benzoic acid | Working example337 | Formulation 337 : Solid | Working example357 | Formulation 357 : Solid | Working example374 | Formulation 374 : Solid |
| | Cinnamic acid | — | — | — | — | — | — |
| Hydroxy group-containing cyclic lactone | L-ascorbic acid | Working example338 | Formulation 338 : Solid | Working example358 | Formulation 358 : Solid | Working example375 | Formulation 375 : Solid |
| | Erythorbic acid | Working example339 | Formulation 339 : Solid | Working example359 | Formulation 359 : Solid | Working example376 | Formulation 376 : Solid |

[Table 1C-3]

| Component (B) \ Component (A) | | Amino acids (values in parentheses are isoelectric points) | |
|---|---|---|---|
| | | L-tryptophan (5.89) | |
| | | Compounding molar ratio (Component (A) : Component (B)) | |
| | | 1:1 | |
| Saturated aliphatic monocarboxylic acid | Formic acid | Working example377 | Formulation 377 : Solid |
| | Acetic acid | Working example378 | Formulation 378 : Solid |
| | Propionic acid | Working example379 | Formulation 379 : Solid |
| | Hexanoic acid | Working example380 | Formulation 380 : Solid |
| | Butyric acid | Working example381 | Formulation 381 : Solid |
| | Isostearic acid | — | — |
| Unsaturated aliphatic carboxylic acid | Oleic acid | — | — |
| | Linoleic acid | — | — |
| Saturated hydroxy monocarboxylic acid | Gluconic acid | Working example382 | Formulation 382 : Solid |
| | Lactic acid | Working example383 | Formulation 383 : Solid |
| Saturated aliphatic dicarboxylic acid | Adipic acid | Working example384 | Formulation 384 : Solid |
| | succinic acid | Working example385 | Formulation 385 : Solid |
| | L-glutamic acid | Working example386 | Formulation 386 : Solid |
| Unsaturated aliphatic dicarboxylic acid | Fumaric acid | Working example387 | Formulation 387 : Solid |
| Saturated hydroxy di- or tricarboxylic acid | L-malic acid | Working example388 | Formulation 388 : Solid |
| | Citric acid | Working example389 | Formulation 389 : Solid |
| | Tartaric acid | Working example390 | Formulation 390 : Solid |
| Aromatic carboxylic acid | Benzoic acid | Working example391 | Formulation 391 : Solid |
| | Cinnamic acid | — | — |
| Hydroxy group-containing cyclic lactone | L-ascorbic acid | Working example392 | Formulation 392: Solid |
| | Erythorbic acid | Working example393 | Formulation 393 : Solid |

[Table 1D]

| Component (B) \ Component (A) | | Amino acids (values in parentheses are isoelectric points) | | | |
|---|---|---|---|---|---|
| | | L-aspartic acid (2.77) | | L-glutamic acid (3.22) | |
| | | Compounding molar ratio (Component (A) : Component (B)) | | | |
| | | 1:1 | | 1:1 | |
| Saturated aliphatic monocarboxylic acid | Formic acid | Working example394 | Formulation 394 : Solid | Working example411 | Formulation 411 : Solid |
| | Acetic acid | Working example395 | Formulation 395 : Solid | Working example412 | Formulation 412 : Solid |
| | Propionic acid | Working example396 | Formulation 396 : Solid | Working example413 | Formulation 413 : Solid |
| | Hexanoic acid | Working example397 | Formulation 397 : Solid | Working example414 | Formulation 414 : Solid |
| | Butyric acid | Working example398 | Formulation 398 : Solid | Working example415 | Formulation 415 : Solid |
| | Isostearic acid | — | — | — | — |
| Unsaturated aliphatic carboxylic acid | Oleic acid | — | — | — | — |
| | Linoleic acid | — | — | — | — |
| Saturated hydroxy monocarboxylic acid | Gluconic acid | Working example399 | Formulation 399 : Solid | Working example416 | Formulation 416 : Solid |
| | Lactic acid | Working example400 | Formulation 400 : Solid | Working example417 | Formulation 417 : Solid |
| Saturated aliphatic dicarboxylic acid | Adipic acid | Working example401 | Formulation 401 : Solid | Working example418 | Formulation 418 : Solid |
| | succinic acid | Working example402 | Formulation 402 : Solid | Working example419 | Formulation 419 : Solid |
| | L-glutamic acid | Working example403 | Formulation 403 : Solid | Working example420 | Formulation 420 : Solid |
| Unsaturated aliphatic dicarboxylic acid | Fumaric acid | Working example404 | Formulation 404 : Solid | Working example421 | Formulation 421 : Solid |
| Saturated hydroxy di- or tricarboxylic acid | L-malic acid | Working example405 | Formulation 405 : Solid | Working example422 | Formulation 422 : Solid |
| | Citric acid | Working example406 | Formulation 406 : Solid | Working example423 | Formulation 423 : Solid |
| | Tartaric acid | Working example407 | Formulation 407 : Solid | Working example424 | Formulation 424 : Solid |
| Aromatic carboxylic acid | Benzoic acid | Working example408 | Formulation 408 : Solid | Working example425 | Formulation 425 : Solid |
| | Cinnamic acid | — | — | — | — |
| Hydroxy group-containing cyclic lactone | L-ascorbic acid | Working example409 | Formulation 409 : Solid | Working example426 | Formulation 426 : Solid |
| | Erythorbic acid | Working example410 | Formulation 410 : Solid | Working example427 | Formulation 427 : Solid |

From the results in Tables 1A-1D, those of the working examples 9, 16, 30, 37, 48-52, 57-60, 69-73, 78-80, 93, 100, 114, 115, 121, 142, 173, 175, 176, 182, 243, 249, 250, 345-349, 354 and 355 were confirmed to be liquid at 25°C.

This suggests that combinations of A to O as indicated in the description tend to be liquid.

### 3. Water retention evaluation

80wt.% aqueous solutions of the formulations (organic salts) of the working examples 428 to 459 and comparative examples 1 to 5 in Tables 2A and 2B were prepared, and the water contents were measured using a Karl Fischer moisture meter (CA-200 manufactured by Mitsubishi Chemical Analytech Co., Ltd.). It was confirmed that the water contents of them were 20.0wt.% (moisture content before test: A). Each of the 1.0 g samples was added to a screw tube, and left uncovered to stand still for 24 hours in a constant temperature and humidity bath (KCL-2000W manufactured by Tokyo Rikakikai Co., Ltd.) which was set at 35° C and 25% RH. Moisture contents after being left for 24 hours were measured again (moisture content after test: B) to evaluate their water retention property by using the following formula for calculating the moisture reduction rates. After that, it was left to stand still until the moisture content remained unchanged, and determinations of the hydrate and the number of hydrated water were made based on the final moisture contents.

Moisture content before test: A (%)
Moisture content after test: B (%)
Moisture reduction rate (%) = [(A (%) - B (%)) / A (%)] × 100

[Table 2A]

| | Formulation | Component (A) | Component (B) | Compounding molar ratio (Component (A) :Component (B)) | State at 25°C | Water retention test of 80wt.% aqueous solution | | | Presence or absence of hydrates | n-hydrate |
|---|---|---|---|---|---|---|---|---|---|---|
| | | | | | | Moisture content before test (A) | Moisture content before test (B) | Moisture eduction rate | | |
| Working example 428 | Formulation 9 | L-lysine | Gluconic acid | 1:1 | Liquid | 20.0% | 15.4% | 22.9% | Present | Dihydrate |
| Working example 429 | Formulation 16 | | Citric acid | 1:1 | Liquid | 20.0% | 16.8% | 16.0% | Present | Monohydrate |
| Working example 430 | Formulation 30 | | Gluconic acid | 1:2 | Liquid | 20.0% | 11.5% | 42.7% | Present | 1.5-hydrate |
| Working example 431 | Formulation 37 | | Citric acid | 3:2 | Liquid | 20.0% | 15.5% | 22.4% | Present | Dihydrate |

(continued)

| | Formulation | Component (A) | Component (B) | Compounding molar ratio (Component (A) :Component (B)) | State at 25°C | Water retention test of 80wt.% aqueous solution | | | Presence or absence of hydrates | n-hydrate |
|---|---|---|---|---|---|---|---|---|---|---|
| | | | | | | Moisture content before test (A) | Moisture content before test (B) | Moisture eduction rate | | |
| Working example 432 | Formulation 51 | L-arginine | Gluconic acid | 1:1 | Liquid | 20.0% | 15.3% | 23.7% | Present | Monohydrate |
| Working example 433 | Formulation 52 | | Lactic acid | 1:1 | Liquid | 20.0% | 15.1% | 24.3% | Present | Monohydrate |
| Working example 434 | Formulation 57 | | L-malic acid | 1:1 | Liquid | 20.0% | 15.4% | 22.8% | Present | Monohydrate |
| Working example 435 | Formulation 58 | | Citric acid | 1:1 | Liquid | 20.0% | 15.6% | 21.8% | Present | 1.5-hydrate |
| Working example 436 | Formulation 59 | | Tartaric acid | 1:1 | Liquid | 20.0% | 13.9% | 30.5% | Present | 1.5-hydrate |
| Working example 437 | Formulation 60 | | Benzoic acid | 1:1 | Liquid | 20.0% | 8.2% | 58.9% | Present | Dihydrate |
| Working example 438 | Formulation 72 | | Gluconic acid | 1:2 | Liquid | 20.0% | 13.6% | 31.9% | Present | 1.5-hydrate |
| Working example 439 | Formulation 73 | | Lactic acid | 1:2 | Liquid | 20.0% | 10.2% | 49.3% | Present | Monohydrate |
| Working example 440 | Formulation 78 | | L-malic acid | 1:2 | Liquid | 20.0% | 13.7% | 31.5% | Present | Monohydrate |
| Working example 441 | Formulation 79 | | Citric acid | 3:2 | Liquid | 20.0% | 15.1% | 24.3% | Present | Dihydrate |
| Working example 442 | Formulation 80 | | Tartaric acid | 1:2 | Liquid | 20.0% | 12.4% | 37.9% | Present | Monohydrate |

(continued)

| | Formulation | Component (A) | Component (B) | Compounding molar ratio (Component (A) :Component (B)) | State at 25°C | Water retention test of 80wt.% aqueous solution | | | Presence or absence of hydrates | n-hydrate |
|---|---|---|---|---|---|---|---|---|---|---|
| | | | | | | Moisture content before test (A) | Moisture content before test (B) | Moisture eduction rate | | |
| Working example 443 | Formulation 93 | L-histidine | Gluconic acid | 1:1 | Liquid | 20.0% | 15.9% | 20.4% | Present | 1.5-hydrate |
| Working example 444 | Formulation 100 | | Citric acid | 1:1 | Liquid | 20.0% | 16.9% | 15.4% | Present | Dihydrate |
| Working example 445 | Formulation 114 | | Gluconic acid | 1:2 | Liquid | 20.0% | 12.8% | 35.9% | Present | Monohydrate |
| Working example 446 | Formulation 115 | | Lactic acid | 1:2 | Liquid | 20.0% | 9.3% | 53.4% | Present | Monohydrate |
| Working example 447 | Formulation 121 | | Citric acid | 3:2 | Liquid | 20.0% | 13.8% | 30.9% | Present | Monohydrate |
| Working example 448 | Formulation 4 | L-lysine | Hexanoic acid | 1:1 | Solid | 20.0% | 8.0% | 60.2% | Absent | - |
| Comparative example 1 | Formulation 428 | Potassium | Lactic acid | 1:1 | Solid | 20.0% | 2.5% | 87.5% | Absent | - |
| Comparative example 2 | Formulation 429 | L-lysine | Chloride | 1:1 | Solid | 20.0% | 6.4% | 68.2% | Absent | - |
| Comparative example 3 | Formulation 430 | Tetrabutylammonium bromide | | - | Solid | 20.0% | 6.0% | 70.0% | Absent | - |
| Comparative example 4 | Formulation 431 | 1-butyl-3-methylimidazolium tetrafluoroborate | | - | Liquid | 20.0% | 5.7% | 71.5% | Absent | - |
| Comparative example 5 | Formulation 432 | Glycerin | | - | Liquid | 20.0% | 2.1% | 89.7% | Absent | - |

[Table 2B]

| | Formulation | Component (A) | Component (B) | Compounding molar ratio (Component (A) : Component (B)) | State at 25°C | Water retention test of 80wt.% aqueous solution | | | Presence or absence of hydrates | n-hydrate |
|---|---|---|---|---|---|---|---|---|---|---|
| | | | | | | Moisture content before test (A) | Moisture content before test (B) | Moisture eduction rate | | |
| Working example 449 | Formulation 142 | Glycine | Citric acid | 1:1 | Liquid | 20.0% | 13.6% | 32.2% | Present | Hemihydrate |
| Working example 450 | Formulation 175 | γ-aminobutyric acid | Gluconic acid | 1:1 | Liquid | 20.0% | 12.7% | 36.7% | Present | Monohydrate |
| Working example 451 | Formulation 176 | | Lactic acid | 1:1 | Liquid | 20.0% | 8.0% | 60.0% | Present | Monohydrate |
| Working example 452 | Formulation 182 | | Citric acid | 1:1 | Liquid | 20.0% | 14.2% | 29.0% | Present | Monohydrate |
| Working example 453 | Formulation 243 | L-serine | Gluconic acid | 1:1 | Liquid | 20.0% | 11.7% | 41.6% | Present | Monohydrate |
| Working example 454 | Formulation 249 | | L-malic acid | 1:1 | Liquid | 20.0% | 11.5% | 42.5% | Present | Monohydrate |
| Working example 455 | Formulation 250 | | Citric acid | 1:1 | Liquid | 20.0% | 12.2% | 39.2% | Present | 1.5-hydrate |
| Working example 456 | Formulation 348 | L-proline | Gluconic acid | 1:1 | Liquid | 20.0% | 10.6% | 46.8% | Present | Monohydrate |
| Working example 457 | Formulation 349 | | Lactic acid | 1:1 | Liquid | 20.0% | 7.0% | 64.9% | Present | Hemihydrate |
| Working example 458 | Formulation 354 | | L-malic acid | 1:1 | Liquid | 20.0% | 7.7% | 61.6% | Present | Hemihydrate |
| Working example 459 | Formulation 355 | | Citric acid | 1:1 | Liquid | 20.0% | 10.2% | 48.9% | Present | Monohydrate |
| Working example 448 | Formulation 4 | L-lysine | Hexanoic acid | 1:1 | Solid | 20.0% | 8.0% | 60.2% | Absent | - |

(continued)

| | Formulation | Component (A) | Component (B) | Compounding molar ratio (Component (A) : Component (B)) | State at 25°C | Water retention test of 80wt.% aqueous solution | | | Presence or absence of hydrates | n-hydrate |
|---|---|---|---|---|---|---|---|---|---|---|
| | | | | | | Moisture content before test (A) | Moisture content before test (B) | Moisture eduction rate | | |
| Comparative example 1 | Formulation 428 | Potassium | Lactic acid | 1:1 | Solid | 20.0% | 2.5% | 87.5% | Absent | - |
| Comparative example 2 | Formulation 429 | L-lysine | Chloride | 1:1 | Solid | 20.0% | 6.4% | 68.2% | Absent | - |
| Comparative example 3 | Formulation 430 | Tetrabutylammonium bromide | | - | Solid | 20.0% | 6.0% | 70.0% | Absent | - |
| Comparative example 4 | Formulation 431 | 1-butyl-3-methylimidazolium tetrafluoroborate | | - | Liquid | 20.0% | 5.7% | 71.5% | Absent | - |
| Comparative example 5 | Formulation 432 | Glycerin | | - | Liquid | 20.0% | 2.1% | 89.7% | Absent | - |

As shown from the results in Tables 2A and 2B, the formulations (organic salts) of the working examples 428 to 459 of 80wt.% aqueous solutions had moisture reduction rates smaller than Comparative examples 1 to 5, and therefore they were excellent in water retention property.

Comparison of the working examples 433, 439, and 446 with the comparative example 1 in which all of them employ lactic acid as the component (B) of the formulation (organic salt) shows that the formulations (organic salts) of the working examples have higher water retention properties, which therefore manifest superiority of the components (A).

A comparison of the working examples 428 to 431 with the comparative example 2 in which all of them employ L-lysine as the component (A) of the formulation (organic salt) manifests superiority of carboxylic acid as the component (B).

Comparison of the working examples 428 to 459 with the comparative example 5a-a conventional water retention agent-shows that they are excellent in water retention property, which therefore suggests contributions of the combinations of components (A) and (B) to the water retention property.

Comparison of the working examples 428 to 459 with comparative examples 3 and 4 of salt structures showed that they are excellent in water retention property, which therefore suggests usability of the combination of components (A) and (B) which can form salt structures.

From the comparison among the formulation with a common the component (B), it was confirmed that the formulations having components (A) of amino acids with isoelectric points of more than 7 (working examples 428, 429, 432, 435, 443, 444, 450 and 452) are excellent in water retention property in comparison to the formulations having amino acids with isoelectric points of 4 or more and 7 or less (working examples 449, 453, 455, 456 and 459).

It was suggested that among amino acids having isoelectric points of more than 7, amino acids having two or more nitrogen atoms are excellent in water retention property.

As can be seen from comparison of the formulations (organic salts) of the working examples 428 to 431 with the formulation (organic salt) of the working example 448 in which all of them employ L-lysine as the component (A), the formulations having carboxylic acids as the component (B) that have a hydrogen-bonding functional group (hydroxy group and/or carboxy group) were shown to have superiorities. Further, as can be seen from comparison among the formulations with components (A) of amino acids having isoelectric points of more than 7, it was confirmed that the formulations having citric acid -a hydroxytricarboxylic acid-as components (B) in the working examples 429, 431, 435, 441, 444a and 447 and 452 were shown to be excellent in water retention property in comparison to the formulations in the working examples 428, 430, 432 to 434, 436 to 440, 442, 443, 445, 446, 450 and 451 having carboxylic acids as components (B) which are not citric acid.

This result suggests that the formulation of the present invention is suitable as a water retention agent used in, for example, cosmetics because of its excellent water retention property.

### 4. Hygroscopicity evaluation

The working examples 460 to 470 and the comparative example 6 in Table 3 were completely dried in a vacuum dryer at 60°C for 18 hours, and then left to stand still at room temperature for 1 month whose moisture contents were then measured using a Karl Fischer moisture meter (CA-200 manufactured by Mitsubishi Chemical Analytech Co., Ltd.) to evaluate their hygroscopicity.

[Table 3]

| | Formulation | Component (A) | Component (B) | Compounding molar ratio (Component (A) : Component (B)) | State at 25°C | Hygroscopicity test | Presence or absence of hydrates | n-hydrate |
|---|---|---|---|---|---|---|---|---|
| | | | | | | Moisture content after test | | |
| Working example460 | Formulation 7 | L-lysine | Oleic acid | 1:1 | Solid | 2.1% | Present | Multihydrate |
| Working example 461 | Formulation 48 | L-arginine | Isostearic acid | 1:1 | Liquid | 6.5% | Present | Multihydrate |
| Working example 462 | Formulation 49 | | Oleic acid | 1:1 | Liquid | 9.1% | Present | Multihydrate |
| Working example 463 | Formulation 50 | | Linoleic acid | 1:1 | Liquid | 4.5% | Present | Multihydrate |
| Working example 464 | Formulation 69 | | Isostearic acid | 2:1 | Liquid | 0.8% | Present | Multihydrate |
| Working example 465 | Formulation 70 | | Oleic acid | 2:1 | Liquid | 9.4% | Present | Multihydrate |
| Working example 466 | Formulation 71 | | Linoleic acid | 2:1 | Liquid | 0.9% | Present | Multihydrate |
| Working example 467 | Formulation 173 | γ-aminobutyric acid | Oleic acid | 1:1 | Liquid | 1.4% | Present | Multihydrate |
| Working example 468 | Formulation 345 | L-proline | Isostearic acid | 1:1 | Liquid | 0.2% | Present | Multihydrate |
| Working example 469 | Formulation 346 | | Oleic acid | 1:1 | Liquid | 0.3% | Present | Multihydrate |
| Working example 470 | Formulation 347 | | Linoleic acid | 1:1 | Liquid | 0.8% | Present | Multihydrate |
| Comparative example 6 | Formulation 433 | Sodium | Oleic acid | - | Solid | 0.0% | - | - |

From the results in Table 3, examples 460 to 470 had a higher moisture content in a saturated state the comparative example 6 and were excellent in hygroscopicity.

When comparing the working examples 460, 462, 465, 467 and 469 (organic salt) with the comparative example 2 in which all of them employ oleic acids as the component (B) of the formulation (organic salt), it was confirmed that the formulations employing an amino acid as the component (A) were excellent, and therefore suitable as hygroscopic agents.

Among them, when comparing the component (A) among those having the same component (B) (i.e.; comparing examples 460, 462, 465 and 467 having amino acids of isoelectric points of more than 7 with examples 469 having an amino acid of an isoelectric point of 4 or more and 7 or less; comparing examples 461 and 464 having amino acids with isoelectric points of more than 7 with example 468 having an amino acid of an isoelectric point of 4 or more and 7 or less; comparing examples 463 and 466 having amino acids with isoelectric points of more than 7 with example 470 having an amino acid of isoelectric point of 4 or more and 7 or less), amino acids with isoelectric points of more than 7 were excellent in hygroscopicity. Furthermore, it was confirmed that L-arginine is superior among amino acids having isoelectric points of more than 7.

From this, it was suggested that the one containing an amino acid having an isoelectric point of more than 7 as the component (A) is excellent in hygroscopicity. It will therefore be suggested that the formulation of the present invention is suitable as a hygroscopic agent used in, for example, cosmetics because of its excellent hygroscopicity.

**5. Sensory evaluation 1**

Each formulation (organic salt) described in examples 471-502 and comparative examples 7-9 in Tables 4A and 4B was diluted to a concentration of 20wt.%, placed in a spray bottle, and was sprayed by a constant amount on the skin to evaluate their spreadability, moisture retention feeling, and non-stickiness when it was applied. Five panel members were randomly selected regardless of age and sex, and the average values were calculated and used as the evaluation values.

As for the spreadability after application, an aqueous solution of each formulation (organic salt) was applied to the skin, and a feeling of the skin when spreading the formulation was then evaluated on a scale of 1 to 5, in which 5 was given to examples where a spreadability was felt significantly, 3 was given to examples where a spreadability was felt, and 1 was given to examples where no spreadability was felt.

As for the moisture retention feeling after application, an aqueous solution of each formulation (organic salt) was applied to the skin, and a feeling of the skin when spreading the formulation was then evaluated on a scale of 1 to 5, in which 5 was given to examples where a moisture retention feeling was felt significantly, 3 was given to examples where a moisture retention feeling was felt, and 1 was given to examples where no moisture retention feeling was felt.

As for the non-stickiness after application, an aqueous solution of each formulation (organic salt) was applied to the skin, and a feeling of the skin when spreading the formulation was then evaluated on a scale of 1 to 5, in which 5 was given to examples where a non-stickiness was felt significantly, 3 was given to examples where a non-stickiness was felt, and 1 was given to examples where no non-stickiness was felt.

[Table 4A]

| | Formulation | Component (A) | Component (B) | Compounding molar ratio (Component (A) : Component (B)) | Sensory evaluation 1 | | |
|---|---|---|---|---|---|---|---|
| | | | | | Spreadability | Moisture retention feeling | Non stickiness |
| Working example 471 | Formulation 9 | L-lysine | Gluconic acid | 1:1 | 4.1 | 4.8 | 3.5 |
| Working example 472 | Formulation 16 | | Citric acid | 1:1 | 4.7 | 4.0 | 4.7 |
| Working example 473 | Formulation 30 | | Gluconic acid | 1:2 | 4.2 | 4.5 | 3.8 |
| Working example 474 | Formulation 37 | | Citric acid | 3:2 | 4.8 | 4.3 | 4.5 |

(continued)

| | Formulation | Component (A) | Component (B) | Compounding molar ratio (Component (A) : Component (B)) | Sensory evaluation 1 | | |
|---|---|---|---|---|---|---|---|
| | | | | | Spreadability | Moisture retention feeling | Non stickiness |
| Working example 475 | Formulation 51 | L-arginine | Gluconic acid | 1:1 | 3.5 | 4.8 | 3.5 |
| Working example 476 | Formulation 52 | | Lactic acid | 1:1 | 4.0 | 4.0 | 4.0 |
| Working example 477 | Formulation 57 | | L-malic acid | 1:1 | 4.0 | 4.0 | 4.5 |
| Working example 478 | Formulation 58 | | Citric acid | 1:1 | 4.7 | 4.0 | 4.8 |
| Working example 479 | Formulation 59 | | Tartaric acid | 1:1 | 3.0 | 4.0 | 3.5 |
| Working example 480 | Formulation 60 | | Benzoic acid | 1:1 | 3.0 | 4.5 | 3.0 |
| Working example 481 | Formulation 72 | | Gluconic acid | 1:2 | 3.5 | 4.5 | 4.0 |
| Working example 482 | Formulation 73 | | Lactic acid | 1:2 | 4.5 | 4.5 | 4.8 |
| Working example 483 | Formulation 78 | | L-malic acid | 1:2 | 4.0 | 4.5 | 4.0 |
| Working example 484 | Formulation 79 | | Citric acid | 3:2 | 4.7 | 4.0 | 4.8 |
| Working example 485 | Formulation 80 | | Tartaric acid | 1:2 | 3.0 | 4.0 | 3.5 |

(continued)

| | Formulation | Component (A) | Component (B) | Compounding molar ratio (Component (A) : Component (B)) | Sensory evaluation 1 | | |
|---|---|---|---|---|---|---|---|
| | | | | | Spreadability | Moisture retention feeling | Non stickiness |
| Working example 486 | Formulation 93 | L-histidine | Gluconic acid | 1:1 | 3.0 | 4.5 | 3.0 |
| Working example 487 | Formulation 100 | | Citric acid | 1:1 | 5.0 | 4.8 | 4.8 |
| Working example 488 | Formulation 114 | | Gluconic acid | 1:2 | 4.0 | 4.0 | 4.0 |
| Working example 489 | Formulation 115 | | Lactic acid | 1:2 | 4.5 | 4.0 | 4.5 |
| Working example 490 | Formulation 121 | | Citric acid | 3:2 | 4.5 | 4.7 | 4.5 |
| Working example 491 | Formulation 4 | L-lysine | Hexanoic acid | 1:1 | 3.2 | 3.5 | 3.2 |
| Comparative example7 | Formulation 428 | Potassium | Lactic acid | 1:1 | 1.2 | 1.4 | 2.4 |
| Comparative example8 | Formulation 429 | L-lysine | Chloride | 1:1 | 1.4 | 1.5 | 2.2 |
| Comparative example9 | Formulation 432 | Glycerin | | - | 2.8 | 3.3 | 2.1 |

[Table 4B]

| | Formulation | Component (A) | Component (B) | Compounding molar ratio (Component (A) : Component (B)) | Sensory evaluation 1 | | |
|---|---|---|---|---|---|---|---|
| | | | | | Spreadability | Moisture retention feeling | Non stickiness |
| Working example 492 | Formulation 142 | Glycine | Citric acid | 1:1 | 4.8 | 4.5 | 4.8 |
| Working example 493 | Formulation 175 | γ-aminobutyric acid | Gluconic acid | 1:1 | 3.3 | 4.2 | 3.2 |
| Working example 494 | Formulation 176 | | Lactic acid | 1:1 | 4.7 | 4.2 | 3.6 |
| Working example 495 | Formulation 182 | | Citric acid | 1:1 | 4.6 | 4.5 | 4.5 |
| Working example 496 | Formulation 243 | L-serine | Gluconic acid | 1:1 | 4.1 | 4.4 | 3.3 |
| Working example 497 | Formulation 249 | | L-malic acid | 1:1 | 4.5 | 4.6 | 4.6 |
| Working example 498 | Formulation 250 | | Citric acid | 1:1 | 4.7 | 4.7 | 4.6 |
| Working example 499 | Formulation 348 | L-proline | Gluconic acid | 1:1 | 4.3 | 4.1 | 4.1 |
| Working example 500 | Formulation 349 | | Lactic acid | 1:1 | 4.7 | 3.7 | 4.5 |
| Working example 501 | Formulation 354 | | L-malic acid | 1:1 | 4.6 | 4.5 | 4.5 |
| Working example 502 | Formulation 355 | | Citric acid | 1:1 | 4.8 | 4.5 | 4.8 |
| Working example 491 | Formulation 4 | L-lysine | Hexanoic acid | 1:1 | 3.2 | 3.5 | 3.2 |
| Comparative example 7 | Formulation 428 | Potassium | Lactic acid | 1:1 | 1.2 | 1.4 | 2.4 |
| Comparative example 8 | Formulation 429 | L-lysine | Chloride | 1:1 | 1.4 | 1.5 | 2.2 |

(continued)

| | Formulation | Component (A) | Component (B) | Compounding molar ratio (Component (A) : Component (B)) | Sensory evaluation 1 | | |
|---|---|---|---|---|---|---|---|
| | | | | | Spreadability | Moisture retention feeling | Non stickiness |
| Comparative example 9 | Formulation 432 | Glycerin | | - | 2.8 | 3.3 | 2.1 |

As can be seen from Tables 4A and 4B, it was confirmed that the working examples 471 to 502 using the formulation (organic ammonium salt(s)) of the present invention were superior to the comparative examples 7 to 9, in any of the feelings of use including spreadability, moisture retention feeling and non-stickiness.
When comparing the working examples 476, 482, 489, 494 and 500 (organic salt) with the comparative example 7 in which all of them employ lactic acid as the component (B) of the formulation (organic salt), it was confirmed that the formulations employing an amino acid as the component (A) were excellent.
A comparison of the working examples 471 to 474 with the comparative example 8 in which all of them employ L-lysine as the component (A) of the formulation (organic salt) shows that formulations having carboxylic acid as the component (B) exhibited favorable feeling of use.
Furthermore, the formulations (organic salts) of examples 471 to 502 were also compared with the comparative example 9-a general water retention agent-indicating structural superiority of the formulation (organic salt) of the component (A) having a cationic residue of the invention and the component (B) having an anionic residue of the invention.
As can be seen from comparison of the formulations (organic salts) of the working examples 471 to 474 with the formulation (organic salt) of the working example 491 in which all of them employ L-lysine as the component (A) of the formulation, the formulations having carboxylic acids as the component (B) that have a hydrogen-bonding functional group (hydroxy group and/or carboxy group) were shown to have superiorities.
Furthermore, the formulations of the working examples 472, 474, 477, 478, 483 484, 487, 490, 492, 495, 497, 498, 501 and 502 had favorable feelings of use, where they each contains: as the component (A) an amino acid having an isoelectric point of more than 7 or an amino acid having an isoelectric point of 4 or more and 7 or less; and as the component (B) L-malic acid or citric acid.
It will therefore be suggested that the formulation of the present invention is suitable as, for example, a cosmetic because of its excellent feelings of use when applied to the skin

### 6. Sensory evaluation 2

For each formulation (organic salt) described in the working examples 503 to 514 and comparative examples 10 and 11 in Table 5, the formulations (organic salt) obtained in "4. Hygroscopicity evaluation" (they were left to stand still at room temperature for 1 month) were used as evaluation samples. The evaluation concentration was calculated from the moisture content obtained in the above-described test. As the evaluation method, the evaluation samples were each applied to the skin by a constant amount to evaluate their spreadability, refreshing feeling and non-stickiness when applied to the skin. Five panel members were randomly selected regardless of age and sex, and the average values were calculated and used as the evaluation values.
As for the spreadability after application, each of the evaluation materials was applied to the skin, and a feeling of the skin when spreading the formulation was then evaluated on a scale of 1 to 5, in which 5 was given to examples where a spreadability was felt significantly, 3 was given to examples where a spreadability was felt, and 1 was given to examples where no spreadability was felt.
As for the refreshing feeling after application, each of the evaluation materials was applied to the skin, and a feeling of the skin when spreading the material was then evaluated on a scale of 1 to 5, in which 5 was given to examples where a refreshing feeling was felt significantly, 3 was given to examples where a refreshing feeling was felt, and 1 was given to examples where no refreshing feeling was felt.
As for the non-stickiness after application, each of the evaluation materials was applied to the skin, and a feeling of the skin when spreading the material was then evaluated on a scale of 1 to 5, in which 5 was given to examples where no stickiness was felt, 3 was given to examples where a subtle stickiness was felt, and 1 was given to examples with stickiness.

[Table 5]

| | Formulation | Component (A) | Component (B) | Compounding molar ratio (Component (A) : Component (B)) | Concentration (wt.%) | Sensory evaluation 2 | | |
|---|---|---|---|---|---|---|---|---|
| | | | | | | Spreadability | Refreshing feeling | Non stickiness |
| Working example503 | Formulation 7 | L-lysine | Oleic acid | 1:1 | 98 | 4.1 | 4.8 | 4.4 |
| Working example 504 | Formulation 48 | L-arginine | Isostearic acid | 1:1 | 94 | 3.1 | 4.4 | 4.1 |
| Working example 505 | Formulation 49 | | Oleic acid | 1:1 | 91 | 4.6 | 4.7 | 4.5 |
| Working example 506 | Formulation 50 | | Linoleic acid | 1:1 | 96 | 3.1 | 3.1 | 3.2 |
| Working example 507 | Formulation 69 | | Isostearic acid | 1:2 | 99 | 4.5 | 4.5 | 4.5 |
| Working example508 | Formulation 70 | | Oleic acid | 1:2 | 91 | 4.8 | 4.9 | 4.7 |
| Working example509 | Formulation 71 | | Linoleic acid | 1:2 | 99 | 3.1 | 3.1 | 3.2 |
| Working example510 | Formulation 172 | γ-aminobutyric acid | Isostearic acid | 1:1 | 99 | 3.3 | 3.5 | 3.2 |
| Working example511 | Formulation 173 | | Oleic acid | 1:1 | 99 | 3.5 | 4.1 | 3.1 |
| Working example512 | Formulation 345 | L-proline | Isostearic acid | 1:1 | 99 | 4.5 | 4.8 | 4.6 |
| Working example513 | Formulation 346 | | Oleic acid | 1:1 | 99 | 4.8 | 4.7 | 4.8 |
| Working example514 | Formulation 347 | | Linoleic acid | 1:1 | 99 | 4.8 | 4.6 | 4.8 |
| Comparative example 10 | Formulation 433 | Sodium | Oleic acid | - | 99 | 1.0 | 1.0 | 1.0 |
| Comparative example 11 | Formulation 434 | Petrolatum | | - | 99 | 2.6 | 2.8 | 2.1 |

As can be seen from Table 5, it was confirmed that the working examples 503 to 514 using the formulation (organic ammonium salt(s)) of the present invention were superior to the comparative examples 10 and 11 which are used as conventional cosmetic oil-based base materials in any of the feelings of use including spreadability, refreshing feeling and non-stickiness when applied to the skin.

When comparing the working examples 503, 505, 508, 511 and 513 (organic salt) with the comparative example 10 in which all of them employ oleic acid as the component (B) of the formulation (organic salt), it was confirmed that formulations employing amino acids as the component (A) were excellent.

The formulations of the working examples 507, 508, and 512-514 particularly had favorable feelings of use. Furthermore, the comparisons between the working examples 504 and 507 and between the working examples 505 and 508 showed superior feelings of use for the formulations having components (A) and (B) in the compounding ration of 1:2.

It will therefore be suggested that the formulation (organic salt) of the present invention is suitable as a cosmetic because of its excellent feelings of use when applied to the skin.

**7. Adhesiveness to hair 1**

80wt.% aqueous solutions of the formulations (organic salts) of the working examples 515 to 544 and comparative examples 12 to 16 in Tables 6A and 6B were prepared, and the water contents were measured using a Karl Fischer moisture meter (KF-200 manufactured by Mitsubishi Chemical Analytech Co., Ltd.). It was confirmed that the water contents of them were 20.0wt.%. As the healthy hair, chemically untreated healthy hair (manufactured by Beaulax Co.,Ltd., black human hair) was used.

0.05 g of hairs (hair weight A before test) were respectively immersed for 60 minutes in 3.0 g of 80 wt.% aqueous solutions of the working examples 515 to 544 and comparative examples 12 to 16. After the immersion, the hairs were each taken out and the composition (organic salt) was wiped off with a Kimwipe until no change in weight had been observed, after which their weight were measured (hair weight B after the test).

The adhesion rate of the formulation (organic salt) to hair was calculated using the following formula.

$$\text{Adhesion rate (\%) of formulation (organic salt)} = [(\text{hair weight B (g) after test} - \text{hair weight A (g) before test}) / \text{hair weight A (g) before test}] \times 100$$

[Table 6A]

| | Formulation | Component (A) | Component (B) | Compounding molar ratio (Component (A) : Component (B)) | Adhesion rate of formulation |
|---|---|---|---|---|---|
| Working example 515 | Formulation 9 | L-lysine | Gluconic acid | 1:1 | 1.7% |
| Working example 516 | Formulation 16 | | Citric acid | 1:1 | 1.6% |

(continued)

| | Formulation | Component (A) | Component (B) | Compounding molar ratio (Component (A) : Component (B)) | Adhesion rate of formulation |
|---|---|---|---|---|---|
| Working example 517 | Formulation 51 | L-arginine | Gluconic acid | 1:1 | 1.7% |
| Working example518 | Formulation 52 | | Lactic acid | 1:1 | 1.5% |
| Working example519 | Formulation 57 | | L-malic acid | 1:1 | 1.9% |
| Working example 520 | Formulation 58 | | Citric acid | 1:1 | 2.0% |
| Working example 521 | Formulation 59 | | Tartaric acid | 1:1 | 1.4% |
| Working example 522 | Formulation 60 | | Benzoic acid | 1:1 | 1.2% |
| Working example 523 | Formulation 72 | | Gluconic acid | 1:2 | 1.7% |
| Working example 524 | Formulation 73 | | Lactic acid | 1:2 | 1.4% |
| Working example 525 | Formulation 78 | | L-malic acid | 1:2 | 1.8% |
| Working example 526 | Formulation 79 | | Citric acid | 3:2 | 1.9% |
| Working example 527 | Formulation 80 | | Tartaric acid | 1:2 | 1.8% |
| Working example 528 | Formulation 93 | L-histidine | Gluconic acid | 1:1 | 2.1% |
| Working example 529 | Formulation 100 | | Citric acid | 1:1 | 2.1% |
| Working example 530 | Formulation 114 | | Gluconic acid | 1:2 | 1.5% |
| Working example 531 | Formulation 115 | | Lactic acid | 1:2 | 1.3% |
| Working example 532 | Formulation 121 | | Citric acid | 3:2 | 1.6% |
| Working example 533 | Formulation 4 | L-lysine | Hexanoic acid | 1:1 | 1.3% |
| Comparative example12 | Formulation 428 | Potassium | Lactic acid | 1:1 | 0.4% |
| Comparative example13 | Formulation 429 | L-lysine | Chloride | 1:1 | 0.6% |
| Comparative example14 | Formulation 430 | Tetrabutylammonium bromide | | - | 1.0% |
| Comparative example15 | Formulation 431 | 1-butyl-3-methylimidazolium tetrafluoroborate | | - | 0.6% |

(continued)

| | Formulation | Component (A) | Component (B) | Compounding molar ratio (Component (A) : Component (B)) | Adhesion rate of formulation |
|---|---|---|---|---|---|
| Comparative example16 | Formulation 432 | Glycerin | | - | 1.1% |

[Table 6B]

| | Formulation | Component (A) | Component (B) | Compounding molar ratio (Component (A) : Component (B)) | Adhesion rate of formulation |
|---|---|---|---|---|---|
| Working example534 | Formulation 142 | Glycine | Citric acid | 1:1 | 2.1% |
| Working example535 | Formulation 175 | γ-aminobutyric acid | Gluconic acid | 1:1 | 2.5% |
| Working example536 | Formulation 176 | | Lactic acid | 1:1 | 1.5% |
| Working example537 | Formulation 182 | | Citric acid | 1:1 | 2.3% |
| Working example538 | Formulation 243 | L-serine | Gluconic acid | 1:1 | 1.7% |
| Working example539 | Formulation 249 | | L-malic acid | 1:1 | 1.7% |
| Working example540 | Formulation 250 | | Citric acid | 1:1 | 1.8% |
| Working example541 | Formulation 348 | L-proline | Gluconic acid | 1:1 | 1.7% |
| Working example542 | Formulation 349 | | Lactic acid | 1:1 | 1.4% |
| Working example543 | Formulation 354 | | L-malic acid | 1:1 | 2.0% |
| Working example544 | Formulation 355 | | Citric acid | 1:1 | 2.2% |
| Working example533 | Formulation 4 | L-lysine | Hexanoic acid | 1:1 | 1.3% |
| Comparative example12 | Formulation 428 | Potassium | Lactic acid | 1:1 | 0.4% |
| Comparative example13 | Formulation 429 | L-lysine | Chloride | 1:1 | 0.6% |
| Comparative example14 | Formulation 430 | Tetrabutylammonium bromide | | - | 1.0% |
| Comparative example15 | Formulation 431 | 1-butyl-3-methylimidazolium tetrafluoroborate | | - | 0.6% |
| Comparative example16 | Formulation 432 | Glycerin | | - | 1.1% |

As shown from the results in Tables 6A and 6B, the formulations (organic salts) of the working examples 515 to 544 had adhesions to the healthy hairs which were superior to those of comparative examples 12 to 16.

When comparing the working examples 518, 524, 531, 536 and 542 (organic salt) with the comparative example 12 in which all of them employ lactic acid as the component (B) of the formulation (organic salt), it was confirmed that the formulations employing an amino acid as the component (A) were excellent.

A comparison of the working examples 515 to 516 with the comparative example 13 in which all of them employ L-lysine as the component (A) of the formulation (organic salt) manifests superiority of carboxylic acid as the component (B).

Comparison of the working examples 515 to 544 with the comparative example 16 shows that they are excellent in adhesion to the hair, which therefore suggests contributions of the combinations of components (A) and (B) to the adhesiveness to the hair.

Comparison of the working examples 515 to 544 with comparative examples 14 and 15 of salt structures shows that they are excellent in the adhesiveness to the hair, which therefore suggests that the combinations of components (A) and (B), potentially forming salt structures, are useful in terms of adhesiveness to the hair.

As can be seen from comparison of the formulations (organic salts) of the working examples 515 and 516 with the formulation (organic salt) of the working example 533 in which all of them employ L-lysine as the component (A) of the formulation, the formulations having carboxylic acids as the component (B) that have a hydrogen-bonding functional group (hydroxy group and/or carboxy group) were shown to have superiorities.

Further, as can be seen from comparisons of the formulations (organic salts) between the working examples 518 and 523 and between the working examples 523 and 524 in which all of them employ L-lysine as the component (A) of the formulation, it was suggested that a carboxylic acid having a plurality of hydroxy groups in the hydrocarbon moiety carboxylic acids as the component (B) is shown to have superiority.

As can be seen from comparison of the formulations (organic salts) among the working examples 518 to 520 and 524 to 526 in which all of them employ L-arginine as the component (A) of the formulation, it was suggested that citric acid having a plurality of carboxy groups in the hydrocarbon moiety as the component (B) is shown to have superiority.

This result suggests excellency in adhesiveness of a formulation (organic salt) having functional group(s) (hydroxy group, carboxyl group, etc.) interacting with the hydrogen-bonding functional groups of proteins on the surface of the hair.

### 8. Adhesion to hair 2

For the working examples 545 to 555 and comparative examples 17 and 18 in Table 7, the formulations (organic salts) as prepared according to "4. Hygroscopicity evaluation" were used in the same manner as explained in "7. Adhesiveness to hair 1" to evaluate their adhesiveness.

[Table 7]

| | Formulation | Component (A) | Component (B) | Compounding molar ratio (Component (A) : Component (B)) | Adhesion rate of formulation |
|---|---|---|---|---|---|
| Working example 545 | Formulation 7 | L-lysine | Oleic acid | 1:1 | 1.4% |
| Working example 546 | Formulation 48 | L-arginine | Isostearic acid | 1:1 | 1.6% |
| Working example 7 | Formulation 49 | | Oleic acid | 1:1 | 1.7% |
| Working example 548 | Formulation 50 | | Linoleic acid | 1:1 | 1.7% |
| Working example 549 | Formulation 69 | | Isostearic acid | 1:2 | 1.3% |
| Working example 550 | Formulation 70 | | Oleic acid | 1:2 | 1.2% |
| Working example 551 | Formulation 71 | | Linoleic acid | 1:2 | 1.3% |

(continued)

| | Formulation | Component (A) | Component (B) | Compounding molar ratio (Component (A) : Component (B)) | Adhesion rate of formulation |
|---|---|---|---|---|---|
| Working example 2 | Formulation 173 | γ-aminobutyric acid | Oleic acid | 1:1 | 1.8% |
| Working example 553 | Formulation 345 | L-proline | Isostearic acid | 1:1 | 1.5% |
| Working example 4 | Formulation 346 | | Oleic acid | 1:1 | 1.6% |
| Working example 555 | Formulation 347 | | Linoleic acid | 1:1 | 1.7% |
| Comparative example17 | Formulation 433 | Sodium | Oleic acid | - | 0.9% |
| Comparative example 18 | Formulation 435 | Liquid paraffin | | - | 0.9% |

As shown from the results in Table 7, the formulations (organic salts) of the working examples 545 to 555 had adhesions to the healthy hairs which were superior to those of comparative examples 17 and 18 which are used as conventional cosmetic oil-based base materials.

When comparing the working examples 545, 547, 550 and 552 (organic salt) with the comparative example 17 in which all of them employ oleic acid as the component (B) of the formulation (organic salt), it was confirmed that formulations employing amino acids as the component (A) were excellent.

**9. Antibacterial property**

The following reagents, etc,, were used for minimum inhibitory concentration (MIC) measurement and halo test.

Staphylococcus aureus: strain of NBRC15035, NITE
Escherichia coli: strain of NBRC 15035, NITE
Potassium lactate manufactured by Fujifilm Wako Pure Chemical
Medium of Muller-Hinton broth: Becton, Dickinson and Company
Media for revival of L-dried specimens "DAIGO" manufactured by Fujifilm Wako Pure Chemical
Calcium chloride manufactured by Fuji Film Wako Pure Chemical
Magnesium chloride manufactured by Fuji Film Wako Pure Chemical
Skim milk: Megmilk Snow Brand
Meat extract: from Solabia Biokar Diagnostics
Peptone: from Solabia Biokar Diagnostics
Sodium chloride manufactured by Fuji Film Wako Pure Chemical
Agar manufactured by Fuji Film Wako Pure Chemical

**9-1. Antibacterial property evaluation 1 (Minimum inhibitory concentration) for formulation (organic salt)**

Working examples 556 to 583 and the comparative example 19 shown in Tables 8A and 8B were subjected to the minimum inhibitory concentration (MIC) test against Escherichia coli and Staphylococcus aureus by the broth microdilution method (as described in the standard method of the Japanese Society of Chemotherapy) to study antibacterial activities of the formulations (organic salt). Formulation (organic salt) concentrations at 250, 200, 150, 100, 50, 25, 12.5, 8, 4, 2, 0.8, 0.4, 0.2, 0.1, 0.05 mg/mL were evaluated, and the MICs are shown in Tables 8A and 8B.

[Table 8A]

| | Formulation | Component (A) | Component (B) | Compounding molar ratio (Component (A) : Component (B)) | Ratio of the total number of amino groups to the total number of carboxy groups (Total number of amino groups/ Total number of carboxy groups) in components (A) and (B) | State at 25°C | MIC (mg/mL) | |
|---|---|---|---|---|---|---|---|---|
| | | | | | | | Staphylococcus aureus | Escherichia coli |
| Working example556 | Formulation 16 | L-lysine | Citric acid | 1:1 | 0.50 | Liquid (hydrate) | 8 | 8 |
| Working example557 | Formulation 30 | | Gluconic acid | 1:2 | 0.67 | Liquid (hydrate) | 8 | 25 |
| Working example 558 | Formulation 37 | | Citric acid | 3:2 | 0.86 | Liquid (hydrate) | 12.5 | 25 |
| Working example559 | Formulation 57 | L-arginine | L-malic acid | 1:1 | 0.67 | Liquid (hydrate) | 25 | 25 |
| Working example 560 | Formulation 58 | | Citric acid | 1:1 | 0.50 | Liquid (hydrate) | 8 | 12.5 |
| Working example 561 | Formulation 59 | | Tartaric acid | 1:1 | 0.67 | Liquid (hydrate) | 8 | 8 |
| Working example 562 | Formulation 60 | | Benzoic acid | 1:1 | 1.00 | Liquid (hydrate) | 25 | 25 |
| Working example 563 | Formulation 72 | | Gluconic acid | 1:2 | 0.67 | Liquid (hydrate) | 12.5 | 25 |
| Working example 564 | Formulation 73 | | Lactic acid | 1:2 | 0.67 | Liquid (hydrate) | 4 | 8 |
| Working example 565 | Formulation 78 | | L-malic acid | 1:2 | 0.40 | Liquid (hydrate) | 4 | 8 |
| Working example 566 | Formulation 79 | | Citric acid | 3:2 | 0.86 | Liquid (hydrate) | 25 | 50 |
| Working example 567 | Formulation 80 | | Tartaric acid | 1:2 | 0.40 | Liquid (hydrate) | 4 | 4 |

(continued)

| | Formulation | Component (A) | Component (B) | Compounding molar ratio (Component (A) : Component (B)) | Ratio of the total number of amino groups to the total number of carboxy groups (Total number of amino groups/ Total number of carboxy groups) in components (A) and (B) | State at 25°C | MIC (mg/mL) | |
|---|---|---|---|---|---|---|---|---|
| | | | | | | | Staphylococcus aureus | Escherichia coli |
| Working example 568 | Formulation 100 | L-histidine | Citric acid | 1:1 | 0.50 | Liquid (hydrate) | 8 | 8 |
| Working example 569 | Formulation 114 | | Gluconic acid | 1:2 | 0.67 | Liquid (hydrate) | 12.5 | 25 |
| Working example 570 | Formulation 115 | | Lactic acid | 1:2 | 0.67 | Liquid (hydrate) | 4 | 8 |
| Working example 571 | Formulation 121 | | Citric acid | 3:2 | 0.86 | Liquid (hydrate) | 25 | 25 |
| Comparative example 19 | Formulation 428 | Potassium | Lactic acid | - | - | Solid | 150 | 100 |

[Table 8B]

| | Formulation | Component (A) | Component (B) | Compounding molar ratio (Component(A) : Component (B)) | Ratio of the total number of amino groups to the total number of carboxy groups (Total number of amino groups/ Total number of carboxy groups) in components (A) and (B) | State at 25°C | MIC (mg/mL) | |
|---|---|---|---|---|---|---|---|---|
| | | | | | | | Staphylococcus aureus | Escherichia coli |
| Working example572 | Formulation 142 | Glycine | Citric acid | 1:1 | 0.25 | Liquid (hydrate) | 2 | 4 |
| Working example573 | Formulation 176 | γ-aminobutyric acid | Lactic acid | 1:1 | 0.50 | Liquid (hydrate) | 4 | 8 |
| Working example574 | Formulation 182 | | Citric acid | 1:1 | 0.25 | Liquid (hydrate) | 4 | 4 |
| Working example575 | Formulation 243 | L-serine | Gluconic acid | 1:1 | 0.50 | Liquid (hydrate) | 4 | 8 |
| Working example576 | Formulation 249 | | L-malic acid | 1:1 | 0.33 | Liquid (hydrate) | 2 | 4 |
| Working example577 | Formulation 250 | | Citric acid | 1:1 | 0.25 | Liquid (hydrate) | 4 | 4 |
| Working example578 | Formulation 348 | L-proline | Gluconic acid | 1:1 | 0.50 | Liquid (hydrate) | 8 | 8 |
| Working example579 | Formulation 349 | | Lactic acid | 1:1 | 0.50 | Liquid (hydrate) | 4 | 4 |
| Working example580 | Formulation 354 | | L-malic acid | 1:1 | 0.33 | Liquid (hydrate) | 4 | 4 |
| Working example581 | Formulation 355 | | Citric acid | 1:1 | 0.25 | Liquid (hydrate) | 4 | 4 |
| Working example582 | Formulation 358 | | L-ascorbic acid | 1:1 | 0.50 | Solid | 8 | 8 |
| Working example583 | Formulation 359 | | Erythorbic acid | 1:1 | 0.50 | Solid | 8 | 8 |
| Comparative example19 | Formulation 428 | Potassium | Lactic acid | - | - | Solid | 150 | 100 |

From the results in Tables 8A and 8B, examples 556 to 583 showed antimicrobial activities that were higher than that of the comparative example 19, and their MICs were 2 mg/mL to 50 mg/mL.

When comparing the working examples 564, 570, 573, 579 (organic salt) with the comparative example 19 in which all of them employ lactic acid as the component (B) of the formulation (organic salt), it was confirmed that the formulations employing an amino acid as the component (A) were excellent.

As can be seen from comparison of the formulations (organic salts) among the working examples 556, 558, 560, 566, 568, 571, 572, 574, 577 and 581 in which all of them employ citric acid as the component (B) of the formulation, it was shown that the component (A) of amino acids having isoelectric points of 4 or more and 7 or less tend to have superior antibacterial activity to those having isoelectric points of more than 7.

It was conformed that a formulation having a value of 1 or less with respect to a ratio of the total number of amino groups to the number of carboxy groups (the total number of amino groups/ the number of carboxy groups) in the components (A) and (B) (Tables 8A and 8B) has an excellent antibacterial activity.

**9-2. Antibacterial property evaluation 2 of formulation (organic salt) (Halo test 1)**

A halo test with Staphylococcus aureus was performed based on JISL1902, 21φm/m test strips of filter paper for KIRIYAMA ROHTO (Manufactured by Kiriyama glass.CO.) were each used and put on a culture media containing the microbials to which a 100μL aqueous solution of the formulation (organic salt) (working examples 584 and 585 and comparative examples 20 and 21) was dropped at a concentration as shown in Table 9. After that, culture was performed, and the presence or absence of halos was visually confirmed, which is as shown in Table 9.

**9-3. Adhesiveness evaluation and Antibacterial property evaluation 3 of formulation (organic salt) (Halo test 2)**

**9-3-1. Adhesiveness evaluation**

Into 21φm/m filter papers for KIRIYAMA ROHTO (Manufactured by Kiriyama glass.CO.) were each dropped a 100μL aqueous solution of the formulation (organic salt) (working examples 584 and 585) at an concentration as shown in Table 9, and subjected to vacuum dehydration for 8 hours at 60° C. The adhesion rates of the samples were each calculated from the weight change of the filter paper (Table 9). As a blank test, 100 μL of water was dropped on the filter paper, and this filter paper was dried in a vacuum. The change in weight was measured and used as the correction value.

A: Weight of adhered sample = Weight of filter paper after drying (mg) - Weight of filter paper before test (mg) + correction value (1.15 mg)
B: Weight of applied sample = Weight of 100 μL aqueous solution (mg) × concentration (wt.%)/100

$$\text{Adhesion rate (\%)} = 100 \text{ x A/B}$$

**9-3-2. Halo test 2**

A halo test with Staphylococcus aureus was performed based on JISL1902 using filter papers as test strips which were each subjected to vacuum dehydration for 8 hours at 60° C after dropping the respective aqueous solution of the formulation (organic salt) according to 9-3-1. After the test, culture was performed, and the presence or absence of halos was visually confirmed, which is as shown in Table 9.

[Table 9]

| | Formulation | Component (A) | Component (B) | Compounding molar ratio (Component (A): Component (B)) | State at 25°C | Concentration (Wt.%) | Halo test 1 | Halo test 2 | |
|---|---|---|---|---|---|---|---|---|---|
| | | | | | | | Presence or absence of halo | Adhesion rate (%) | Presence or absence of halo |
| Working example584 | Formulation 249 | L-serine | L-malic acid | 1:1 | Liquid (hydrate) | 10 | Present | 94.3 | Present |
| Working example585 | Formulation 249 | | | | | 50 | Present | 92.8 | Present |
| Comparative example20 | Formulation 436 | Ethanol | | - | Liquid | 10 | Absent | - | - |
| Comparative example21 | Formulation 436 | | | | | 50 | Absent | - | - |

The ethanol, which is widely known for its bactericidal and antibacterial effects, was volatilized during the culture and could not exhibit an antibacterial effect, and no halo was observed. In contrast, halos were observed in the formulation of the working examples. It will therefore be suggested that the formulations of the present invention are not volatile, and remain involatile on the filter paper which in turn allows the formulations to have long-lasting antibacterial properties.

It was conformed that the ethanol was volatilized in the halo test 2, and did not adhere to the filter paper, whereas the formulations of the working examples adhered well to the filter paper having a hydrogen-bonding functional group since the formulations of the working examples each has a hydrogen-bonding functional group, and the formulations were non-volatile after subjecting them to heat vacuum dehydration, and exhibited long-lasing antibacterial activity for a long period of time.

**9-4. Stability evaluation and Solubility evaluations of poorly-soluble substances to the**

**formulations.**

Into 20 wt.% aqueous solutions of the formulations (organic salts) shown in Table 10 are dissolved methylparaben (manufactured by Tokyo Kasei, hereinafter, paraben) at 1.0 wt.%, 0.5 wt.% and 0.25 wt.% at 80°C to prepare the respective antibacterial compositions. The composition was then left to stand still at 25° C. for 24 hours under closed conditions, and the appearance of each antibacterial composition was visually observed to evaluate the solubility according to the following criteria (Table 10).

◎: 1.0 wt.% or more; no crystal precipitation was observed

◦: 0.5 wt.% or more and less than 1.0 wt.%; no crystal precipitation was observed

△: 0.25 wt.% or more and less than 0.5 wt.%; no crystal precipitation was observed

×: less than 0.25 wt.%; no crystal precipitation was observed

Polyoxyethylene alkyl ether (hereinafter referred to as AE) which is an antiviral substance recognized to be effective against viruses was also subjected to the similar procedure to evaluate the solubility based on the above-mentioned criteria. As the AE, Peletex 2465, manufactured by Miyoshi Oil & Fats Co., Ltd., was used. (Table 10)

[Table 10]

| | Formulation | Component (A) | Component (B) | State at 25°C | Compounding molar ratio (Component (A) : Component (B)) | Solubility into 20 wt. % aqueous solution of the formulations | |
|---|---|---|---|---|---|---|---|
| | | | | | | Paraben | AE |
| Working example586 | Formulation 60 | L-arginine | Benzoic acid | Liquid | 1:1 | ◎ | ◎ |
| Working example587 | Formulation 72 | L-arginine | Gluconic acid | Liquid | 2:1 | ○ | ◎ |
| Working example588 | Formulation 100 | L-histidine | Citric acid | Liquid | 1:1 | ○ | ◎ |
| Working example589 | Formulation 114 | L-histidine | Gluconic acid | Liquid | 2:1 | ○ | ◎ |
| Working example590 | Formulation 115 | L-histidine | Lactic acid | Liquid | 2:1 | ◎ | ◎ |
| Working example591 | Formulation 182 | γ-aminobutyric acid | Citric acid | Liquid | 1:1 | ◎ | ◎ |
| Working example592 | Formulation 249 | L-serine | L-malic acid | Liquid | 1:1 | ○ | ◎ |

(continued)

| | Formulation | Component (A) | Component (B) | State at 25°C | Compounding molar ratio (Component (A) : Component (B)) | Solubility into 20 wt. % aqueous solution of the formulations | |
|---|---|---|---|---|---|---|---|
| | | | | | | Paraben | AE |
| Working example593 | Formulation 250 | L-serine | Citric acid | Liquid | 1:1 | ○ | ◎ |
| Working example594 | Formulation 354 | L-proline | L-malic acid | Liquid | 1:1 | ◎ | ◎ |
| Working example595 | Formulation 355 | L-proline | Citric acid | Liquid | 1:1 | ◎ | ◎ |
| Working example596 | Formulation 358 | L-proline | Ascorbic acid | Solid | 1:1 | ○ | ◎ |
| Comparative example22 | Formulation 428 | Potassium | Lactic acid | Solid | 1:1 | × | ◎ |
| Comparative example23 | Formulation 437 | L-proline | Chloride | Solid | 1:1 | × | ◎ |
| Comparative example24 | Formulation 430 | Tetrabutylammonium bromide | | Solid | - | × | ◎ |
| Comparative example25 | Formulation 431 | 1-butyl-3-methylimidazolium tetrafluoroborate | | Solid | - | × | ◎ |
| Comparative example26 | Formulation 438 | Water | | Liquid | - | Δ | ◎ |

It was observed that the formulations (organic salts) of the present invention favorably dissolve poorly soluble antibacterial agents and other antiviral substances, and no crystals precipitate was observed after being left to stand still at 25°C for 24 hours, thereby providing a stable solution. Accordingly, the formulation of the present invention imparted antibacterial or antiviral property by adding, for example, an antibacterial agent. It is suggested that an antibacterial composition can be obtained thereby.

When comparing the working example 590 (organic salt) with the comparative example 22 in which both of them employ lactic acid as the component (B) of the formulation (organic salt), it was observed that the formulations employing amino acid as the component (A) tend to have a high solubility to paraben.

A comparison of the working examples 594 to 596 with the comparative example 23 in which all of them employ L-proline as the component (A) of the formulation (organic salt) shows superiority of carboxylic acid as the component (B).

A comparison of the working examples 586 to 596 with the comparative examples 24 and 25 of salt structures shows that they are excellent in solubility of poorly-soluble substances, which therefore suggests that the combinations of components (A) and (B), potentially forming salt structures, are useful in terms of solubility of poorly-soluble substances.

### 9-5. Antibacterial evaluation of antibacterial composition (Minimum inhibitory concentration)

Antibacterial compositions prepared to have the composition having the components (A) and (B) and paraben in an equal amount (working examples 597 to 612, wherein 1.0mg/mL of paraben and 1.0mg/mL of the respective working examples 597 to 612 are combined to be 2.0 mg/mL of the composition); the comparative example 27 containing only of water and paraben (at the concentration of 1.0mg/mL of paraben); and reference example 1 containing L-lysine and citric acid (at the concentration of 1.0mg/mL) were each subjected to a test performed by a method similar to and as explained in the section of 9-1 based on the presence or absence of the bacterial growth to evaluate the presence or absence of the antibacterial activity.

[Table 11]

| | Formulation | Component (A) | Component (B) | Compounding molar ratio (Component (A) : Component (B)) | Total concentration of antibacterial agents (mg/mL) (concentration of the formulation (mg/mL) + Concentration of parabe (mg/mL)) | Antibacterial activity is |
|---|---|---|---|---|---|---|
| Working example597 | Formulation 16 | L-lysine | Citric acid | 1:1 | 2 (1+1) | present |
| Working example598 | Formulation 57 | L-arginine | L-malic acid | 1:1 | 2 (1+1) | present |
| Working example599 | Formulation 58 | | Citric acid | 1:1 | 2 (1+1) | present |
| Working example600 | Formulation 59 | | Tartaric acid | 1:1 | 2 (1+1) | present |
| Working example601 | Formulation 60 | | Benzoic acid | 1:1 | 2 (1+1) | present |
| Working example602 | Formulation 78 | | L-malic acid | 2:1 | 2 (1+1) | present |
| Working example603 | Formulation 79 | | Citric acid | 3:2 | 2 (1+1) | present |
| Working example604 | Formulation 80 | | Tartaric acid | 2:1 | 2 (1+1) | present |
| Working example605 | Formulation 100 | L-histidine | Citric acid | 1:1 | 2 (1+1) | present |
| Working example606 | Formulation 121 | | Citric acid | 3:2 | 2 (1+1) | present |
| Working example607 | Formulation 142 | Glycine | Citric acid | 1:1 | 2 (1+1) | present |
| Working example608 | Formulation 182 | γ-aminobutyric acid | Citric acid | 1:1 | 2 (1+1) | present |
| Working example609 | Formulation 249 | L-serine | L-malic acid | 1:1 | 2 (1+1) | present |
| Working example610 | Formulation 250 | L-serine | Citric acid | 1:1 | 2 (1+1) | present |
| Working example611 | Formulation 354 | L-proline | L-malic acid | 1:1 | 2 (1+1) | present |
| Working example612 | Formulation 355 | L-proline | Citric acid | 2:1 | 2 (1+1) | present |
| Comparative example27 | Formulation 438 | Water | | - | 1 (0+1) | absent |
| Reference example 1 | Formulation 16 | L-lysine | Citric acid | 1:1 | 1 (1+0) | absent |

Although 1 mg/mL of paraben of the comparative example 27 and reference example 1 or the formulation (organic salt) alone does not exhibit any antibacterial activity, the working examples 597 to 612 in which 1mg/mL of paraben and 1mg/mL of the formulation (organic salt) are mixed with each other to make in total 2mg/mL of antibacterial composition provided antibacterial activity of paraben on top of the antibacterial properties of the formulations (organic salts), which thereby exhibited antibacterial properties as compositions.

That is, the formulation (organic salt) of the present invention may be added with a further additive to impart an advantage of the additive to a composition containing the formulation. For example, a composition obtained by adding (dissolving) an antiviral substance as explained in the section of 9-4 impart not only an antiviral property but also an antibacterial activity.

### 9-6. Stability evaluation of antibacterial composition

Into 50wt% of formulations 597 to 612 were respectively added paraben of 20wt.% water solution which were put into sample vials with the open top and left to stand still for 8 hours at 50°. The appearance of each antibacterial composition was visually observed.

Water was evaporated and crystals of paraben were deposited for a case where only water is used. Meanwhile, as for 50wt.% aqueous solutions of the formulations 597 to 612, the liquids were not completely evaporated and no crystal precipitation was observed, which indicates that the compositions of the present invention exhibit favorably stability and maintain solubility of an additive due to the non-volatility of the compounds (organic salts) of the present invention, thus exhibiting the effect(s) of the additive in an efficient manner for a long period of time.

### 10. Evaluation of skin irritation

The skin irritation tests of the working examples 613 to 619 in Table 12 were carried out based on a skin irritation test method using LabCyte EPI-MODEL24 (3-D Cultured Human Epidermis Model) manufactured by Japan Tissue Engineering Co., Ltd. (J-TEC). Note that the measurement concentration was determined to be 50wt.%. The irritancy was determined from the obtained cell viability based on the following criteria.

Viability: ≤50% Irritating
Viability: >50% non-irritating

[Table 12]

| | Formulation | Component (A) | Component (B) | Compounding molar ratio (Component (A) : Component (B)) | Skin irritation |
|---|---|---|---|---|---|
| Working example613 | Formulation 100 | L-histidine | Citric acid | 1:1 | Non-irritating |
| Working example614 | Formulation 115 | | Lactic acid | 1:2 | Non-irritating |
| Working example615 | Formulation 121 | | Citric acid | 3:2 | Non-irritating |
| Working example616 | Formulation 182 | γ-aminobutyric acid | Citric acid | 1:1 | Non-irritating |
| Working example617 | Formulation 249 | L-serine | L-malic acid | 1:1 | Non-irritating |
| Working example618 | Formulation 250 | | Citric acid | 1:1 | Non-irritating |
| Working example619 | Formulation 355 | L-proline | Citric acid | 1:1 | Non-irritating |

As shown in Table 12, the skin irritation test shows that the working examples 613 to 619 were non-irritating, suggesting that the formulations of the present invention are safe and secure for the skin.

Therefore, it is therefore suggested that the formulation of the present invention is suitable for use in cosmetics and daily necessities because of its safety when applied to the skin.

## 11. Biodegradability assessment

The biodegradability test of the working examples 620-623 in Table 13 was conducted according to the OECD test guidelines of 301C test. In this test, a general activated sludge was used as a microorganism source, and into 300 ml of the prepared standard test culture solution was added a microorganism source and a test substance such that they reach to the concentrations of 30 mg / L and 100mg/L in the test period of 28 days using aniline as a standard material. Regarding the degradation rate, a BOD sensor by Actac.LTD was used for measuring the biochemical oxygen demand (BOD), and the degradation rate was calculated from the calculated theoretical degradation rate. Specifically, when the BOD degradations for 28 days were 60% or more, they were determined as being readily degradable (herein denoted as O), and when the BOD degradations were less than 60%, they were determined as being not readily degradable (herein denoted as x).

[Table 13]

| | Formulation | Component (A) | Component (B) | Compounding molar ratio (Component (A) : Component (B)) | Biodegradability |
|---|---|---|---|---|---|
| Working example620 | Formulation 100 | L-histidine | Citric acid | 1:1 | ○ (Readily degradable) |
| Working example621 | Formulation 121 | | Citric acid | 3:2 | ○ (Readily degradable) |
| Working example622 | Formulation 249 | L-serine | L-malic acid | 1:1 | ○ (Readily degradable) |
| Working example623 | Formulation 250 | | Citric acid | 1:1 | ○ (Readily degradable) |

The results in Table 13 suggest that the working examples 620 to 623 are readily degradable in terms of biodegradability, which therefore suggests that the formulations (organic salts) of the present invention have excellent biodegradability and cause less impact on the environment.

## 12. Gel composition preparation

Xanthan gum (Echo Gum T: manufactured by DSP Gokyo Food & Chemical Co., Ltd), carrageenan (SEA-PI GUM FA: manufactured by DSP Gokyo Food & Chemical), gellan gum (KELCOGEL: manufactured by San-Ei-gen F.F.I., INC), guar gum (SUPERGEL CSA 200/50: manufactured by Sansho Co., Ltd.), diutan gum (KELCO-VIS DG: manufactured by Sansho Co., Ltd.) were used as polymer compounds; L-arginine, $\gamma$-aminobutyric acid and L-serine were used as the amino acids of the component (A); and benzoic acid, citric acid, and L-malic acid were used as the component (B).

The respective compositions were prepared by the following method (Table 14). After the components (A) and (B) described in Table 14 were mixed in water, the water was distilled off, and the resulting mixtures or salts were a hydrate and liquid at 25°C.

### <Working example 624> Composition 1

0.059 g of L-arginine as an amine compound (component (A)) and 0.041 g of benzoic acid as an acid (component (B)) (in which molar ratio of the components (A) and (B) is component (A):component (B) = 1:1; concentration of (component (A) + component (B)) is 1wt.%), 0.1 g of xanthan gum as being a polymer compound (concentration of the polymer compound in the composition is lwt.%) and 9.8 g of water are mixed and then heated and stirred at 50° C for 30 minutes for dissolution, after which the mixture was cooled to room temperature over 60 minutes to obtain a composition 1 in the form of a gel.

### <Working examples 625 to 651> Compositions 2 to 28

Similarly, predetermined amounts of amino acids (component (A)), carboxylic acids (component (B)), and polymer compounds as shown in Table 14 were respectively prepared under the same condition as in the working example 624 to obtain gel compositions 2 to 28.

Further, each of the amino acids (component (A)), acid (component (B)) described in the working examples

625 to 651 and water were mixed in advance in the same preparing amounts as described above to synthesize an organic salt, and then a polymer compound was added thereto, which was heated, stirred, dissolved and cooled under the same condition as in the working example 624, as a result of which a gel composition was obtained also in this way. From this, a thickening effect was recognized.

[Table 14]

| | Composition | Formulation | | | | Concentration of (Component (A)+ Component (B)) in composition (Wt.%) | Polymer compound | Polymer compound concentration in composition (Wt. %) | Composition appearance |
|---|---|---|---|---|---|---|---|---|---|
| | | Formulation | Component (A) | Component (B) | Compounding molar ratio (Component (A): Component (B)) | | | | |
| Working example 624 | Composition 1 | Formulation 60 | 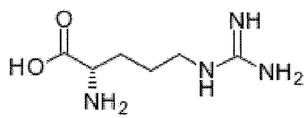 | Benzoic acid | | 1 | Xanthan gum | | Gel |
| Working example 625 | Compositions | | | | | | Carrageenan | | Gel |
| Working example 626 | Composition3 | | | | | | Gellan gum | | Gel |
| Working example 627 | Composition4 | | | | | | Guar gum | | Gel |
| Working example 628 | Compositions | | | | | | Diutan gum | | Gel |
| Working example 629 | Compositions | | | | | 10 | Xanthan gum | | Gel |
| Working example 630 | Composition7 | | | | | | Guar gum | | Gel |
| Working example 631 | Compositions | | | | | | Diutan gum | | Gel |

(continued)

| | Composition | Formulation | | | | Concentration of (Component (A)+ Component (B)) in composition (Wt.%) | Polymer compound | Polymer compound concentration in composition (Wt.%) | Composition appearance |
|---|---|---|---|---|---|---|---|---|---|
| | | Formulation | Component (A) | Component (B) | Compounding molar ratio (Component (A): Component (B)) | | | | |
| Working example 632 | Composition 9 | Formulation 16 | 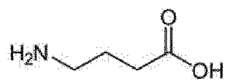 | Citric acid | | 1 | Xanthan gum | | Gel |
| Working example 633 | Composition 10 | | | | | | Guar gum | | Gel |
| Working example 634 | Composition 11 | | | | | | Diutan gum | | Gel |
| Working example 635 | Composition 12 | | | | | 10 | Xanthan gum | | Gel |
| Working example 636 | Composition 13 | | | | | | Guar gum | | Gel |
| Working example 637 | Composition 14 | | | | | | Diutan gum | | Gel |

(continued)

| | Composition | Formulation | | | | Concentration of (Component (A)+ Component (B)) in composition (Wt.%) | Polymer compound | Polymer compound concentration in composition (Wt. %) | Composition appearance |
|---|---|---|---|---|---|---|---|---|---|
| | | Formulation | Component (A) | Component (B) | Compounding molar ratio (Component (A): Component (B)) | | | | |
| Working example 638 | Composition 15 | Formulation 249 | 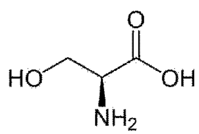 | L-malic acid | 1 : 1 | 1 | Xanthan gum | 1 | Gel |
| Working example 639 | Composition 16 | | | | | | Guar gum | | Gel |
| Working example 640 | Composition 17 | | | | | | Diutan gum | | Gel |
| Working example 641 | Composition 18 | | | | | 10 | Xanthan gum | | Gel |
| Working example 642 | Composition 19 | | | | | | Carrageenan | | Gel |
| Working example 643 | Composition20 | | | | | | Guar gum | | Gel |
| Working example 644 | Composition21 | | | | | | Diutan gum | | Gel |

(continued)

| | Composition | Formulation | | | | Concentration of (Component (A)+ Component (B)) in composition (Wt.%) | Polymer compound | Polymer compound concentration in composition (Wt. %) | Composition appearance |
|---|---|---|---|---|---|---|---|---|---|
| | | Formulation | Component (A) | Component (B) | Compounding molar ratio (Component (A): Component (B)) | | | | |
| Working example 645 | Composition 22 | Formulation 250 | | Citric acid | | 1 | Xanthan gum | | Gel |
| Working example 646 | Composition23 | Formulation 250 | | Citric acid | | 1 | Guar gum | | Gel |
| Working example 647 | Composition24 | Formulation 250 | | Citric acid | | 1 | Diutan gum | | Gel |
| Working example 648 | Composition25 | Formulation 250 | | Citric acid | | 10 | Xanthan gum | | Gel |
| Working example 649 | Composition 26 | Formulation 250 | | Citric acid | | 10 | Carrageenan | | Gel |
| Working example 650 | Composition27 | Formulation 250 | | Citric acid | | 10 | Guar gum | | Gel |
| Working example 651 | Composition28 | Formulation 250 | | Citric acid | | 10 | Diutan gum | | Gel |

### 13. Solubility evaluation of active ingredients

Solubilities of active ingredients were evaluated for the compositions shown in Table 15. As the active ingredients, there were used a poorly-soluble gallic acid having an antioxidant effect, and glutamic acid having a moisture retention effect. While the solubility of the comparative example 28 (Ion- exchange water) was less than 1.0 g, the working examples 652 to 659 exhibited a high solubility of 1.0g or more. A similar tendency was also resulted for the cases of gallic acid. The formulations 182, 354 and 355 were particularly superior for dissolving gallic acid, and the formulations 100, 121 and 182 were particularly superior for dissolving glutamic acid.

A comparison of the working examples 653 to 659 with the working example 652 shows that a hydroxycarboxylic acid to be used as the component (B) increases the solubility.

From a comparison of the working examples 656 to 657 with the working examples 658 to 659, when hydroxycarboxylic acid was used as the component (B) in the case where gallic acid was dissolved, it was shown that an employment of L-proline rather than L-serine leads to an enhanced solubility. Further, from a comparison of the working example 655 with the working example 659, when citric acid was used as the component (B), it was shown that an employment of L-proline or γ-aminobutyric acid rather than L-serine leads to an enhanced solubility.

From a comparison of the working examples 653 to 655 and the working examples 657 and 659, when glutamic acid was dissolved, using γ-aminobutyric acid and L-histidine with an isoelectric point of 7.0 or more as the component (A) increased the solubility. From the working example 653 and the working example 654, it was shown that the solubility increased regardless of the compounding ratio.

[Table 15]

| | Formulation | Component (A) | Component (B) | Compounding molar ratio (Component (A) : Component (B)) | Compound Concentration (wt%) | Solubility (g/100g) | |
|---|---|---|---|---|---|---|---|
| | | | | | | Gallic acid | Glutamic acid |
| Working example652 | Formulation 60 | L-arginine | Benzoic acid | 1:1 | 23 | 1.5 | 1.0 |
| Working example653 | Formulation 100 | L-histidine | Citric acid | 1:1 | | - | ≧2.5 |
| Working example654 | Formulation 121 | | Citric acid | 3:2 | | - | ≧2.5 |
| Working example655 | Formulation 182 | L-amino butyric acid | Citric acid | 1:1 | | ≧3.5 | ≧2.5 |
| Working example656 | Formulation 249 | L-serine | L-malic acid | 1:1 | | 3.0 | 2.0 |
| Working example657 | Formulation 250 | | Citric acid | 1:1 | | 2.0 | 2.0 |
| Working example658 | Formulation 354 | L-proline | L-malic acid | 1:1 | | ≧3.5 | 2.0 |
| Working example659 | Formulation 355 | | Citric acid | 1:1 | | ≧3.5 | 2.0 |
| Comparative example28 | Formulation 438 | Water | | - | - | 1.0 | <1.0 |

## 14. Evaluation of skin surface improvement

The skin permeability test of the working examples 660 to 667 in Table 16 was evaluated by the following microscopic observation of the stratum corneum. Formulation 439 of the comparative example 31 was prepared in the same manner as formulation (salt) 52.

As the evaluation method, first, 1.0wt.% aqueous solutions of the working examples 660 to 667 and the comparative examples 30 and 31 were used as sample solutions. In the comparative example 29, water was used as the sample solution.The obtained sample solution was continuously applied to the skin of the inner side of an upper arm of each of the 10 test subjects by a constant amount once a day for one month. After that, a Cellotape ® (manufactured by Nichiban Co., Ltd.) was applied to the inner side of an upper arm of each of the ten test subjects who have been kept in quiet for 30 minutes under constant temperature and humidity to delaminate the horny cell layer. The Cellotape attached with the horny cell layer was adhered on a slide glass that was thinly coated in advance with a Cemedine for polyvinyl products (manufactured by Cemedine Co., Ltd.) such that the horny cell layer and the resin surface are faced with each other. It was immersed in ethanol for ten minutes and then immersed in xylene for two hours, after which the tape was solely peeled off from the slide glass and then it was immersed in xylene for another one hour and taken out of it for evaporating the xylene on the slide glass. After that, it was subjected to the staining for four minutes in a stain solution (0.5wt.% of brilliant green, 1.0wt.% of gentian violet), washed with running water and dried to make an observation sample. Based on the observation results of the samples, each sample was observed using an optical microscope (Axio Image. A2m manufactured by ZEISS Microscopy) to make evaluations for four items based on the following criteria to thereby evaluate the skin surface improvement in a comprehensive manner.

### 1: Delamination of Multilayered stratum corneum

Good: Delamination of multilayered stratum corneum cells was reduced compared to the one before the application
Unchanged: No change was observed in the delamination of multilayered stratum corneum cells with reference to the one before the application.
No good: Delamination of multilayered stratum corneum cells was increased compared to the one before the application

### 2. Alignment regularity

Good: Horny cells had alignments that were more aligned than those before the application
Unchanged: No change was observed in the alignment of horny cells with reference to the one before the application.
No good: Horny cells had alignments that were less aligned than those before the application

### 3. Surface size uniformity

Good: Horny cells had surfaces that were more uniform in size compared to those before the application.
Unchanged: No change was observed in the size uniformity of horny cells with reference to the one before the application
No good: Horny cells had surfaces that were less uniform in size compared to those before the application.

### 4: Presence or absence of nucleated cells

Good: Horny cells having cell nucleus are decreased in number compared to those before the application.
Unchanged: No change was observed in the number of horny cells having cell nucleus
No Good: Horny cells having cell nucleus are increased in number compared to those before the application.

**Comprehensive evaluation**

◦: Skin surface condition was ameliorated compared to the condition before the application
△: No change was observed in skin surface condition compared to that before the application
×: Skin surface condition got worse condition compared to the condition before the application

[Table 16]

| | Formulation | Component (A) | Component (B) | Compounding molar ratio (Component (A) : Component (B)) | Skin improvement effect |
|---|---|---|---|---|---|
| Working example660 | Formulation 60 | L-arginine | Benzoic acid | 1:1 | ○ |
| Working example661 | Formulation 100 | L-histidine | Citric acid | 1:1 | ○ |
| Working example662 | Formulation 121 | | Citric acid | 3:2 | ○ |
| Working example663 | Formulation 182 | L-amino butyric acid | Citric acid | 1:1 | ○ |
| Working example664 | Formulation 249 | L-serine | L-malic acid | 1:1 | ○ |
| Working example665 | Formulation 250 | | Citric acid | 1:1 | ○ |
| Working example666 | Formulation 354 | L-proline | L-malic acid | 1:1 | ○ |
| Working example667 | Formulation 355 | | Citric acid | 1:1 | ○ |
| Comparative example 29 | Formulation 438 | Water | | - | × |
| Comparative example 30 | Formulation 434 | Petrolatum | | - | Δ |
| Comparative example 31 | Formulation 439 | Tris (hydroxymethyl) aminomethane | Lactic acid | - | Δ |

As indicated from the results shown in Table 16, it was found that the working examples 660 to 667 exhibit favorable skin surface improvement effects compared to the comparative examples 29 to 31. From these results, it was suggested that this favorable improvement in skin surface is superior to the comparative example 30 (petrolatum) that has been conventionally used for the purpose of reducing dryness of skin, or even to the comparative example 31 in which an organic salt that employs no amino acid for the component (A) was used. In view of this, since the amino acids in these formulations are major components of moisturizing ingredients (Natural Moisturizing Factor (NMF)) that are present in horny layer, it may be inferred that the formulations permeated in the skin supplement the NMFs of the skin to thereby promote moisturization, which thereby ameliorated the balance of the skin surface. NMF is a component that plays an important role for retaining moisture in the horny layer by means of hydrogen bonding with water in the skin. It was therefore suggested that that the formulations according to the present invention are effective for improving skin surface.

**15. Evaluation on permeability to Skin**

The following tape stripping method was used for carrying out the evaluation of skin permeability test on each

of the working examples 668 to 675 in Table 17.

As for the evaluation method, into each of 10wt.% aqueous solutions of the working examples 668 to 675 and the comparative example 32 is added ascorbyl glucoside until it has an amount of 5wt.% of ascorbyl glucoside to prepare a sample solution. As for the comparative example 33, ascorbyl glucoside was added so as to be 5% by mass with respect to water to prepare a sample solution. Then, a cotton material impregnated with the prepared 750μL sample solution was applied to a 1cm×3cm area of the inner side of an upper arm for each of the 5 test subjects who have been kept in quiet for 30 minutes under constant temperature and humidity, and allowed to stand still for 5 minutes. After removing the cotton material, a new cotton material was used to remove the sample having been left on the skin surface and then allowed to stand still for 30 minutes. The horns in the first to eighth layers were then harvest from the application site using a Cellotape® (manufactured by Nichiban Co., Ltd.), and all of the layers obtained from the tape were used as samples for assay.

The obtained sample for assay was then extracted with 2ml of ion-exchange water over the course of 10 min. A 0.2μm syringe filter (manufactured by ADVANTEC, INC) was used to remove extraneous materials to obtain an extraction liquid from which a permeation amount of ascorbyl glucoside (μg/$cm^2$) was measured using HPLC. The measured values of the samples for assay that were harvested from the 5 test subjects were averaged, and the averaged value was regarded as the permeation amount (μg/$cm^2$) to make a comparison of the total permeation amounts for the first to eighth layers. Finally, the working examples 668 to 675 and the comparative examples 32 and 33, having been evaluated this time, were compared with each other to evaluate them as the relative infiltration rates.

A high-performance liquid chromatograph (HPLC, Ultimate 3000 manufactured by Thermo Scientific Inc.) was used to perform quantitative analysis of ascorbyl glucoside under the following conditions:

Column: Trinity P1 (100 × 2.1 mm, 3 μm)
Solvent: A: water, B: acetonitrile, C: 200 mM ammonium formate (pH = 4)
Mobile phase: B: 70%, C: 30%.
Detection: UV (270 nm)
Flow rate: 0.3 mL/min.
Column temperature: 30°C
Syringe wash: 200 mM ammonium formate (pH = 4)
Inject: 10 μL

[Table 17]

| | Formulation | Component (A) | Component (B) | Compounding molar ratio (Component (A) : Component (B)) | Relative infiltration rates of active component (ascorbyl glucoside) into skin (comparison among Working examples 666 to 673 and Comparative examples 32 and 33) 1(Slow) ⇔ 5(Fast) |
|---|---|---|---|---|---|
| Working example668 | Formulation 52 | L-arginine | Lactic acid | 1:1 | 1 |
| Working example669 | Formulation 100 | L-histidine | Citric acid | 1:1 | 2 |
| Working example670 | Formulation 121 | | Citric acid | 3:2 | 1 |
| Working example671 | Formulation 182 | L-amino butyric acid | Citric acid | 1:1 | 1 |
| Working example672 | Formulation 249 | L-serine | L-malic acid | 1:1 | 4 |
| Working example673 | Formulation 250 | | Citric acid | 1:1 | 2 |

(continued)

| | Formulation | Component (A) | Component (B) | Compounding molar ratio (Component (A) : Component (B)) | Relative infiltration rates of active component (ascorbyl glucoside) into skin (comparison among Working examples 666 to 673 and Comparative examples 32 and 33) 1(Slow) ⇔ 5(Fast) |
|---|---|---|---|---|---|
| Working example674 | Formulation 354 | L-proline | L-malic acid | 1:1 | 1 |
| Working example675 | Formulation 355 | | Citric acid | 1:1 | 2 |
| Comparative example 32 | Formulation 432 | Glycerin | | | 5 |
| Comparative example 33 | Formulation 438 | Water | | - | 5 |

As it turned out from the results in Table 17, the comparison among the working examples 668 to 675 and the comparative examples 32 and 33 shows that the infiltration rates of the working examples 668 to 675 were slower than those of the comparative examples 32 and 33. The working examples 668 to 671 and 673 to 675 had particularly slow infiltration rates, which indicates that these examples would be effective for the case of slowly infiltrating an active ingredient into the skin where a slow-acting active component is required. Meanwhile, example 672 had a fast infiltration rate which is similar to the comparative examples 32 and 33, indicating that this example would be effective for the case of instantly infiltrating an active ingredient into the skin where a quick-acting active component is required. Further, as the infiltration rates of the inventive formulations varied in accordance with the combinations of components (A) and (B), it can be inferred that the formulation is useful as a material for controlling the infiltration rate of an active component.

## 16. Sensory evaluation 3

For each formulation described in the working examples 676 to 687 and the comparative examples 34 and 35 in Table 18, the formulations (organic salt) obtained in "4. Hygroscopicity evaluation" (they were left to stand still at room temperature for 1 month) were used as evaluation samples. The evaluation concentrations were calculated from the moisture contents obtained in the above-described test. Further, the formulation 440 (Dimeticone) of FUJIFILM Wako Pure Chemical Corporation was used.

As the evaluation method, the evaluation samples were each applied to the hair by a constant amount to evaluate their spreadability when applied to the hair and cohesiveness after applying them to the hair. Five panel members were randomly selected regardless of age and sex, and the average values were calculated and used as the evaluation values.

As for the spreadability during the application, each of the evaluation materials was applied thereto, and a feeling of the skin when spreading the formulation was then evaluated on a scale of 1 to 5, in which 5 was given to examples where a spreadability was felt significantly, 3 was given to examples where a spreadability was felt, and 1 was given to examples where no spreadability was felt.

As for the cohesiveness of hair after the application, each of the evaluation materials was applied thereto to evaluate the cohesiveness of hair when the materials were settled based on a scale of 1 to 5, in which 5 was given to examples where the hair was favorably cohesive, 3 was given to examples where the hair was cohesive, and 1 was given to examples where the hair was not cohesive.

[Table 18]

| | Formulation | Component (A) | Component (B) | Compounding molar ratio (Component (A) : Component (B)) | Concentration (Wt.%) | Sensory evaluation 3 | |
|---|---|---|---|---|---|---|---|
| | | | | | | Spreadability | Cohesiveness |
| Working example 676 | Formulation 7 | L-lysine | Oleic acid | 1:1 | 98 | 4.1 | 4.8 |
| Working example 677 | Formulation 48 | L-arginine | Isostearic acid | 1:1 | 94 | 3.6 | 4.4 |
| Working example 678 | Formulation 49 | | Oleic acid | 1:1 | 91 | 4.2 | 4.7 |
| Working example 679 | Formulation 50 | | Linoleic acid | 1:1 | 96 | 3.6 | 4.4 |
| Working example 680 | Formulation 69 | | Isostearic acid | 1:2 | 99 | 3.9 | 4.5 |
| Working example 681 | Formulation 70 | | Oleic acid | 1:2 | 91 | 4.2 | 4.9 |
| Working example 682 | Formulation 71 | | Linoleic acid | 1:2 | 99 | 3.6 | 4.4 |
| Working example 683 | Formulation 172 | γ-aminobutyric acid | Isostearic acid | 1:1 | 99 | 3.6 | 4.4 |
| Working example 684 | Formulation 173 | | Oleic acid | 1:1 | 99 | 4.2 | 4.7 |
| Working example 685 | Formulation 345 | L-proline | Isostearic acid | 1:1 | 99 | 4.0 | 4.4 |
| Working example 686 | Formulation 346 | | Oleic acid | 1:1 | 99 | 4.3 | 4.7 |
| Working example 687 | Formulation 347 | | Linoleic acid | 1:1 | 99 | 4.0 | 4.6 |
| Comparative example 34 | Formulation 433 | Sodium | Oleic acid | - | 99 | 1.0 | 1.0 |
| Comparative example 35 | Formulation 440 | Dimeticone | | - | 99 | 3.5 | 3.6 |

As shown in Table 18, it was found that the working examples 676 to 687 had superior spreadability when applied to the hair and superior cohesiveness after applying them to the hair compared to those of the comparative examples 34 and 35. Among them, the working examples 676, 678, 681, 684 and 686 employing olein acid as the component (B) had excellent feelings of use. Further, the working examples 676 to 687 employed the components (A) and (B) that were listed in JSQI and therefore were suggested to have a high degree of safety. It can therefore be inferred that the formulations of the present invention are suitable as hair treatment agents.

**17. Evaluation on hair surface improvement**

The following evaluation method was performed for the working examples 668 to 731 in Tables 20A to 20C as the test of the hair surface improvement in which the following formulations 441 to 445 were prepared:

Formulation 441 (L-arginine) manufactured by Fujifilm Wako Pure Chemical
Formulation 442 as prepared by the same method as Formulation (salt) 52
Formulation 443 (L- proline) manufactured by Sigma-Aldrich Japan;
Formulation 444 (Lactic acid) manufactured by Kanto Kagaku Co., Ltd.

Regarding the evaluation method, as an operation of the bleach treatment, 1g of bleaching agent (Table 19) was first applied to 1g by mass of untreated black hair (manufactured by Beaulax Co., Ltd), which was allowed to stand still for 30 minutes and then sufficiently rinsed with 40°C warm water. This bleach treatment operation was repeated 5 times to prepare damaged hairs. Then, the formulations of the working examples 688 to 731 and the comparative examples 36 to 40 were dissolved into 50wt.% ethanol aqueous solution so that they reach 1wt.% to prepare sample materials. Each of the prepared 1g of damaged hairs was immersed into the material for 15 minutes, rinsed with 40°C warm water for 30 seconds, dried with a towel and then subjected to air drying to make a post-treatment hair. Surface conditions of the hair bundles before and after the treatment were observed using a scanning electron microscope (S-3400N SEM manufactured by Hitachi High-Tech Corporation) to evaluate improvements in the hair surfaces based on the following criteria.
◎: No cuticles are raised.

◦: Almost no cuticles are raised.

△: Some of the cuticles are raised

×: Almost all of the cuticles are raised

[Table 19]

| Component | Amount (parts by mass) |
|---|---|
| 30% hydrogen peroxide solution | 9.6 |
| 28% ammonia water | 4 |
| Monoethanolamine | 1.5 |
| Ammonium hydrogen carbonate | 1 |
| Ion-exchange water | Remainder |
| Total | 100 |

[Table 20A]

| | Formulation | Component (A) | Component (B) | Compounding molar ratio (Component (A) : Component (B)) | Hair surface improving effect |
|---|---|---|---|---|---|
| Working example 688 | Formulation 9 | L-lysine | Gluconic acid | 1:1 | ○ |
| Working example689 | Formulation 16 | | Citric acid | 1:1 | ○ |
| Working example690 | Formulation 30 | | Gluconic acid | 1:2 | ◎ |
| Working example691 | Formulation 37 | | Citric acid | 3:2 | ○ |
| Working example692 | Formulation 51 | L-arginine | Gluconic acid | 1:1 | ○ |
| Working example693 | Formulation 52 | | Lactic acid | 1:1 | ○ |
| Working example694 | Formulation 57 | | L-malic acid | 1:1 | ○ |
| Working example695 | Formulation 58 | | Citric acid | 1:1 | ◎ |
| Working example696 | Formulation 59 | | Tartaric acid | 1:1 | ○ |
| Working example697 | Formulation 60 | | Benzoic acid | 1:1 | ○ |
| Working example698 | Formulation 72 | | Gluconic acid | 1:2 | ◎ |
| Working example699 | Formulation 73 | | Lactic acid | 1:2 | ○ |
| Working example700 | Formulation 78 | | L-malic acid | 1:2 | ○ |
| Working example701 | Formulation 70 | | Citric acid | 3:2 | ○ |
| Working example702 | Formulation 80 | | Tartaric acid | 1:2 | ○ |
| Working example703 | Formulation 93 | L-histidine | Gluconic acid | 1:1 | ○ |
| Working example704 | Formulation 100 | | Citric acid | 1:1 | ◎ |
| Working example705 | Formulation 114 | | Gluconic acid | 1:2 | ◎ |
| Working example706 | Formulation 115 | | Lactic acid | 1:2 | ○ |
| Working example707 | Formulation 121 | | Citric acid | 3:2 | ○ |

[Table 20B]

| | Formulation | Component (A) | Component (B) | Compounding molar ratio (Component (A) : Component (B)) | Hair surface improving effect |
|---|---|---|---|---|---|
| Working example 708 | Formulation 142 | Glycine | Citric acid | 1:1 | ○ |
| Working example 709 | Formulation 175 | γ-aminobutyric acid | Gluconic acid | 1:1 | ○ |
| Working example 710 | Formulation 176 | | Lactic acid | 1:1 | ○ |
| Working example 711 | Formulation 182 | | Citric acid | 1:1 | ○ |
| Working example 712 | Formulation 243 | L-serine | Gluconic acid | 1:1 | ○ |
| Working example 713 | Formulation 249 | | L-malic acid | 1:1 | ○ |
| Working example 714 | Formulation 250 | | Citric acid | 1:1 | ○ |
| Working example 715 | Formulation 348 | L-proline | Gluconic acid | 1:1 | ○ |
| Working example 716 | Formulation 349 | | Lactic acid | 1:1 | ○ |
| Working example 717 | Formulation 354 | | L-malic acid | 1:1 | ○ |
| Working example 718 | Formulation 416 | L-glutamic acid | Gluconic acid | 1:1 | ○ |
| Working example 719 | Formulation 422 | | L-malic acid | 1:1 | ○ |
| Working example 720 | Formulation 423 | | Citric acid | 1:1 | ○ |

[Table 20C]

| | Formulation | Component (A) | Component (B) | Compounding molar ratio (Component (A) : Component (B)) | Hair surface improving effect |
|---|---|---|---|---|---|
| Working example 721 | Formulation 48 | L-arginine | Isostearic acid | 1:1 | ○ |
| Working example 722 | Formulation 49 | | Oleic acid | 1:1 | ○ |
| Working example 723 | Formulation 50 | | Linoleic acid | 1:1 | ○ |
| Working example 724 | Formulation 69 | | Isostearic acid | 2:1 | ○ |
| Working example 725 | Formulation 70 | | Oleic acid | 2:1 | ○ |
| Working example 726 | Formulation 71 | | Linoleic acid | 2:1 | ○ |
| Working example 727 | Formulation 46 | | Hexanoic acid | 1:1 | Δ |
| Working example 728 | Formulation 173 | γ-aminobutyric acid | Oleic acid | 1:1 | ○ |
| Working example 729 | Formulation 345 | L-proline | Isostearic acid | 1:1 | Δ |
| Working example 730 | Formulation 346 | | Oleic acid | 1:1 | Δ |
| Working example 731 | Formulation 347 | | Linoleic acid | 1:1 | Δ |
| Comparative example 36 | Formulation 441 | L-arginine | - | - | × |
| Comparative example 37 | Formulation 442 | | hydrochloric acid | 1:1 | × |
| Comparative example 38 | Formulation 443 | L-proline | - | - | × |
| Comparative example 39 | Formulation 444 | - | Lactic acid | - | × |

As shown in Table 20A to 20C, a comparison of the working examples 688 to 731 and the comparative examples 36 to 39 shows that the raisings of cuticles of the working examples 688 to 731 were suppressed, which therefore indicate an improvement in the hair surface. Among them, it was shown that formulations 30, 58, 72, 100 and 114 had particularly excellent hair surface improving effects.

It was found from comparisons between the working examples 692 to 702, 721 to 727 and the comparative example 36 (arginine), between the working examples 715 to 717, 729 to 731 and the comparative example 38, and between the working examples 693, 699, 706, 710, 716 and the comparative example 39 that the coexistence of both components (A) and (B) results in a superior hair surface improving effect compared to the case where only one of the components (A) and (B) is solely used. It was also found from a comparison between the working examples 692 to 702, 721 to 727 and the comparative example 37 that the component (B) of an organic acid leads to improvement in the hair surface. It was found from a comparison between the working examples 692 to 702, 721 to 726 and the working example 727 that the formulations of liquid lead to excellent hair surface improving effects.

Particularly, as shown from the working examples 690, 695, 698, 704 and 705, it was found that the formulations have high levels of hair surface improvement effects in the cases where the component (A) is of basic amino acid and

a ratio of the total number of primary or secondary amino groups in the component (A) to the number of carboxy groups in the component (B) is 1 or less.

That is, since a use of the formulations according to the present invention resulted in an excellent hair surface improvement effect, it was indicated that the formulation is useful as a hair treatment agent.

18. Evaluation on Stabilization of Protein (Keratin) of Hair and Skin

1 g of a powdery keratin (by Tokyo Chemical Industry Co., Ltd.) (pre-test keratin) was added into 2 g of a 10 wt. % aqueous solution of each of the working examples 732 to 749, followed by performing stirring at 25°C for 24 hours. After stirring, filtration was performed, and the resultant powdery keratin was then dried so as to obtain a keratin that had been treated (post-test keratin). The same operation was performed on 2 g of water as a comparative example 41.

Here, 0.3 g of the resultant post-treated keratin was placed into a thermostatic device of 130°C, and left to stand still therein for seven days. After seven days, an IR spectrum of the resultant keratin was measured to observe an absorption derived from the α-helix secondary structure of amide to thereby evaluate a stabilization effect of the hair treatment agent on the structure of keratin.

As a result of measuring the powder of the keratin before heating using IR, an absorption was observed at 1654 $cm^{-1}$ which was derived from the α-helix secondary structure of amide. Next, IR measurement was carried out after performing the heating test at 130°C to obtain the respective peaks derived from each amide and an intensity ratio(s) thereof to a reference peak (reference peak (derived from C-N) 1086 $cm^{-1}$, peak 1654 $cm^{-1}$ derived from the α-helix secondary structure of amide). The intensity ratio (X) was evaluated as "intensity of reference peak: intensity of peak derived from amide = 1: X," by reading the absorption intensity of the reference peak and the intensity of the peak derived from amide, and their comparisons were made based on the following criteria.

◎:$0.6<X\leqq 1.0$
◦:$0.3<X\leqq 0.6$
△:$0<X\leqq 0.3$
×: X=0

[Table 21]

| | Formulation | Component (A) | Component (B) | Compounding molar ratio (Component (A) : Component (B)) | Presence or absence of amide peak (Peak wavelength) | Intensity ratio (X) to reference peak |
|---|---|---|---|---|---|---|
| Working example 732 | Formulation 60 | L-arginine | Benzoic acid | 1:1 | Present (1654$cm^{-1}$) | ◎ |
| Working example 733 | Formulation 48 | | Isostearic acid | 1:1 | Present (1654$cm^{-1}$) | Δ |
| Working example 734 | Formulation 49 | | Oleic acid | 1:1 | Present (1654$cm^{-1}$) | ◎ |
| Working example 735 | Formulation 50 | | Linoleic acid | 1:1 | Present (1654$cm^{-1}$) | ◎ |
| Working example 736 | Formulation 69 | | Isostearic acid | 1:2 | Present (1654$cm^{-1}$) | Δ |
| Working example 737 | Formulation 70 | | Oleic acid | 1:2 | Present (1654$cm^{-1}$) | ◎ |
| Working example 738 | Formulation 71 | | Linoleic acid | 1:2 | Present (1654$cm^{-1}$) | ◎ |

(continued)

| | Formulation | Component (A) | Component (B) | Compounding molar ratio (Component (A) : Component (B)) | Presence or absence of amide peak (Peak wavelength) | Intensity ratio (X) to reference peak |
|---|---|---|---|---|---|---|
| Working example 739 | Formulation 100 | L-histidine | Citric acid | 1:1 | Present ($1654cm^{-1}$) | ○ |
| Working example 740 | Formulation 121 | | Citric acid | 3:2 | Present ($1654cm^{-1}$) | Δ |
| Working example 741 | Formulation 173 | γ-aminobutyric acid | Oleic acid | 1:1 | Present ($1654cm^{-1}$) | ○ |
| Working example 742 | Formulation 182 | | Citric acid | 1:1 | Present ($1654cm^{-1}$) | Δ |
| Working example 743 | Formulation 249 | L-serine | Citric acid | 1:1 | Present ($1654cm^{-1}$) | ◎ |
| Working example 744 | Formulation 250 | | L-malic acid | 1:1 | Present ($1654cm^{-1}$) | ○ |
| Working example 745 | Formulation 345 | L-proline | Isostearic acid | 1:1 | Present ($1654cm^{-1}$) | Δ |
| Working example 746 | Formulation 346 | | Oleic acid | 1:1 | Present ($1654cm^{-1}$) | ○ |
| Working example 747 | Formulation 347 | | Linoleic acid | 1:1 | Present ($1654cm^{-1}$) | ○ |
| Working example 748 | Formulation 354 | | Citric acid | 1:1 | Present ($1654cm^{-1}$) | Δ |
| Working example 749 | Formulation 355 | | L-malic acid | 1:1 | Present ($1654cm^{-1}$) | ○ |
| Comparative example 40 | Formulation 438 | Water | | - | Absent | × |

As can be seen from the results shown in Table 21, in the case of a comparative example 41 of water, it was found that the peak derived from the α-helix secondary structure of amide had disappeared, i.e., the α-helix secondary structure of keratin failed to be maintained. In contrast, the α-helix secondary structure of keratin was confirmed to have been maintained in the cases of the working examples 732 to 749, because a peak(s) derived from the α-helix secondary structure of amide were observed.

It was shown from a comparison between the working examples 734, 735, 737 and 738 and the working examples 741,746 and 747 that an employment of L-arginine as the component (A) rather than γ-aminobutyric acid or L-proline leads to an excellent stabilizing effect of α-helix secondary structure of keratin. It was also found from a comparison between the working examples 732, 734, 735, 736, 738 and the working examples 733, 736 and between the working examples 746, 747 and the working example 743 that an employment of an aromatic carboxylic acid or an unsaturated fatty acid as the component (B) rather than a saturated fatty acid leads to an excellent stabilizing effect of α-helix secondary structure of keratin. Further, it was shown from the working examples 734, 735 and the working examples 736, 738 that an employment of an unsaturated fatty acid as the component (B) leads to an excellent stabilizing effect of α-helix secondary structure of keratin regardless of their compounding molar ratios.

It was shown from a comparison between the working example 741 and the working example 742 that an employment of an unsaturated fatty acid as the component (B) rather than a hydroxycarboxylic acid leads to an excellent stabilizing effect of α-helix secondary structure of keratin.

It was shown from a comparison between the working examples 743, 744 and the working examples 748, 749 that, when a hydroxycarboxylic acid is used as the component (B), an employment of L-proline rather than L-serine

leads to an excellent stabilizing effect of α-helix secondary structure of keratin.

It was found from a comparison between the working examples 739, 742, 748 and the working example 743 that, when a citric acid is used as the component (B), an employment of L- serine rather than L-histidine, γ-aminobutyric acid, L-proline leads to an excellent stabilizing effect of α-helix secondary structure of keratin. These results indicate that an employment of an amino acid, as the component (A), having a hydroxy group or an isoelectric point of 6 or less leads to an excellent stabilizing effect of α-helix secondary structure of keratin.

It was shown from a comparison between the working example 739 and the working example 740 that, when a hydroxycarboxylic acid is used as the component (B), a compounding ratio having an equivalent molar ratio thereof have a superior excellent stabilizing effect of α-helix secondary structure of keratin compared to the compounding ratio in which the total number of primary or secondary amino groups is equivalent to the number of carboxy groups.

In this way, since the formulation of the present invention has an effect of stabilizing keratin, there can be realized a water and moisture retention of the hair, health and quality maintenance of the hair, suppression of hair damages caused by heat from a dryer or the like, and even, for example, water and moisture retention of skin proteins such as cuticles and nails as well as health maintenance thereof.

**19. Dispersibility evaluation of metallic oxide**

As for the working examples 750 to 756 and the comparative example 41 (poly(oxyethylene)nonylphenylether: PERETEX 1225 manufactured by Miyoshi Oil & Fat Co., Ltd.) as shown in table 22, 0.5g of an 80% aqueous solution of each of the formulations and 0.5g of zirconium oxide (IV) (Manufactured by Fujifilm Wako Pure Chemical Corporation; Reagent of special grade; about 5 to 30 μm) were mixed for 1 minutes for five times at 2000rpm using a planetary centrifugal mixer (ARE-310 manufactured by THINKY CORPORATION) after which their dispersing conditions were visually observed.

[Table 22]

| | Formulation | Component (A) | Component (B) | Compounding molar ratio (Component (A) : Component (B)) | Dispersibility (zirconium oxide (IV)) |
|---|---|---|---|---|---|
| Working example 750 | Formulation 60 | L-arginine | Benzoic acid | 1:1 | ○ |
| Working example751 | Formulation 100 | L-histidine | Citric acid | 1:1 | ○ |
| Working example752 | Formulation 121 | | Citric acid | 3:2 | ○ |
| Working example753 | Formulation 182 | L-amino butyric acid | Citric acid | 1:1 | ○ |
| Working example 754 | Formulation 250 | L-serine | Citric acid | 1:1 | ○ |
| Working example755 | Formulation 354 | L-proline | L-malic acid | 1:1 | ○ |
| Working example756 | Formulation 355 | | Citric acid | 1:1 | ○ |
| Comparative example 41 | Formulation 445 | Poly(oxyethylene) nonylphenylether | | - | × |

A comparison between the working examples 750 to 756 and the comparative example 41 as shown in Table 22 shows that the formulations of the working examples each dispersed zirconium oxide (IV) in a favorable manner and resulted in a dispersion liquid while an agglomeration or precipitation of the zirconium oxide (IV) was observed for the comparative example 41. That is, since the formulation of the present invention is comprised of a cation having a hydrogen-bonding functional group (i.e., having hydrogen donating property or coordination), it can be inferred that the structural characteristics of having a high-affinity to the oxygen atom of zirconium oxide (IV)-a hydrogen bond receptor-played an advantageous role for dispersing the zirconium oxide (IV) in a favorable manner. The formulations of the present invention were excellent in the affinity to inorganic oxides, which therefore suggests that the formulations would

be useful for fields that utilize dispersions of such materials, such as the fields of cosmetics, paints, inks, electronic components and batteries.

## Claims

1. A formulation comprising the following components:

   (A) an amino acid or a salt thereof; and
   (B) a carboxylic acid or a salt thereof.

2. The formulation according to claim 1, wherein the component (A) is represented by the following formula:
   [Chemical formula 1]

   $$R1_lNH_mC(R^2)_2(R^3_nCOOX) \qquad (I)$$

   wherein $R^1$ represents a monovalent or bivalent organic group having 1 to 22 carbon atoms, $R^2$ independently represents a hydrogen atom or a monovalent or bivalent organic group having 1 to 22 carbon atoms, $R^3$ represents a bivalent organic group having 1 to 22 carbon atoms, 1 represents any one of 0 to 2, m represents any one of 0 to 2, and n represents 0 or 1, $R^1$ and $R^2$ optionally together form a ring of 3 to 22 carbon atoms, and X represents a hydrogen atom or a monovalent cation.

3. The formulation according to claim 2, wherein X is a hydrogen atom.

4. The formulation according to any one of claims 1 to 3, wherein a ratio of the total number of primary or secondary amino groups to the number of carboxy groups (the total number of primary or secondary amino groups/ the number of carboxy groups) in the component (A) is greater than 1.

5. The formulation according to any one of claims 1 to 3, wherein a ratio of the total number of primary or secondary amino groups to the number of carboxy groups (the total number of primary or secondary amino groups/ the number of carboxy groups) in the component (A) is 1.

6. The formulation according to any one of claims 1 to 3, wherein a ratio of the total number of primary or secondary amino groups to the number of carboxy groups (the total number of primary or secondary amino groups/ the number of carboxy groups) in the component (A) is less than 1.

7. The formulation according to any one of claims 1, 3 and 4, wherein the component (A) has an isoelectric point of greater than 7.

8. The formulation according to any one of claims 1, 3 and 5, wherein the component (A) has an isoelectric point of 4 or more and 7 or less.

9. The formulation according to any one of claims 1, 3 and 6, wherein the component (A) has an isoelectric point of less than 4.

10. The formulation according to any one of the preceding claims, wherein the component (B) is a carboxylic acid.

11. The formulation according to any one of the preceding claims, wherein the component (B) is a carboxylic acid having a hydrogen-bonding functional group(s) in a hydrocarbon moiety.

12. The formulation according to claim 11, wherein the hydrogen-bonding functional group(s) is/are a hydroxy group and/or a carboxy group.

13. The formulation according to claim 12, wherein the hydrogen-bonding functional group(s) is a hydroxy group.

14. The formulation according to claim 12, wherein the formulation comprises both a hydroxy group and a carboxy group as hydrogen-bonding functional groups.

**15.** The formulation according to any one of claim 1 to 10, wherein the component (B) is an unsaturated or branched aliphatic carboxylic acid having 8 to 22 carbon atoms.

**16.** The formulation according to any one of the preceding claims, wherein the formulation comprises an organic salt formed by: a cation originated from the component (A); and an anion originated from an anionic residue of the component (B), wherein said cation optionally contains a cationic residue of the component (B).

**17.** The formulation according to any one of the preceding claims, wherein an anhydride and/or hydrate of a mixture or salt of the components (A) and (B) is liquid at 25°C.

**18.** A composition comprising the formulation according to any one of the preceding claims.

**19.** An organic salt formed by the amino acid and the carboxylic acid according to any one of claims 1 to 17, wherein the organic salt is represented by the following formula (II):
[Chemical formula 2]

$$[R^1{}_lN^+H_mC(R^2)_2(R^3{}_nCOOX)]R^4COO^- \qquad (II)$$

wherein $R^1$ represents a monovalent organic group having 1 to 22 carbon atoms, $R^2$ independently represents a hydrogen atom or a monovalent or bivalent organic group having 1 to 22 carbon atoms, $R^3$ represents a bivalent organic group having 1 to 22 carbon atoms, $R^4$ represents a hydrogen atom or a monovalent organic group having 1 to 21 carbon atoms, 1 represents any one of 0 to 3, m represents any one of 0 to 3, and n represents 0 or 1, $R^1$ and $R^2$ optionally together form a ring of 3 to 22 carbon atoms, and X represents a hydrogen atom or a monovalent cation.

**20.** The organic salt according to claim 19, wherein X is hydrogen atom.

**21.** The organic salt according to claim 19 or 20, wherein a ratio of the total number of primary or secondary amino groups to the number of carboxy groups (the total number of primary or secondary amino groups/ the number of carboxy groups) in the amino acid is greater than 1.

**22.** The organic salt according to claim 19 or 20, wherein a ratio of the total number of primary or secondary amino groups to the number of carboxy groups (the total number of primary or secondary amino groups/ the number of carboxy groups) in the amino acid is 1.

**23.** The organic salt according to claim 19 or 20, wherein a ratio of the total number of primary or secondary amino groups to the number of carboxy groups (the total number of primary or secondary amino groups/ the number of carboxy groups) in the amino acid is less than 1.

**24.** The organic salt according to any one of claims 19 to 21, wherein the amino acid has an isoelectric point of greater than 7.

**25.** The organic salt according to any one of claims 19, 20 and 22, wherein the amino acid has an isoelectric point of 4 or more and 7 or less.

**26.** The organic salt according to any one of claims 19, 20 and 23, wherein the amino acid has an isoelectric point of less than 4.

**27.** The organic salt according to any one of claims 19 to 26, wherein $R^4$ in the formula (II) is a hydrocarbon group having a hydrogen-bonding functional group(s).

**28.** The organic salt according to claim 27, wherein $R^4$ in the formula (II) is a hydrocarbon group having a hydroxy group and/or a carboxy group.

**29.** The organic salt according to claim 28, wherein $R^4$ in the formula (II) is a hydrocarbon group having a hydroxy group.

**30.** The organic salt according to claim 28, wherein $R^4$ in the formula (II) is a hydrocarbon group having both a hydroxy group and a carboxy group.

**31.** The organic salt according to any one of claims 19 to 26, wherein $R^4$ in the formula (II) is an unsaturated or branched aliphatic hydrocarbon group having 7 to 21 carbon atoms.

**32.** The organic salt according to any one of claims 19 to 31, wherein an anhydride and/or hydrate of the organic salt is liquid at 25°C.

**33.** A composition comprising the organic salt according to any one of claims 19 to 32.

**34.** The composition according to claim 18 or 33 as a gel composition, wherein the gel composition comprises water and a polymer compound.

**35.** The gel composition according to claim 34, wherein the polymer compound has a hydrogen-bonding functional group(s).

**36.** The gel composition according to claim 34 or 35, wherein the polymer compound is a polysaccharide.

**37.** The gel composition according to claim 36, wherein the polysaccharide is of at least one species selected from xanthan gum, carrageenan, gellan gum, guar gum and diutan gum.

**38.** The gel composition according to any one of claims 34 to 37, wherein the composition has an enhanced viscosity or the composition is for enhancing viscosity.

**39.** The formulation, organic salt or composition for use in imparting water retentivity according to any one of the preceding claims.

**40.** The formulation, organic salt or composition for use in imparting hygroscopicity according to any one of the preceding claims.

**41.** The formulation, organic salt or composition for use in imparting antibacterial activity according to any one of the preceding claims.

**42.** The formulation, organic salt or composition for use in imparting antibacterial activity according to any one of the preceding claims,

wherein a ratio of the total number of amino groups to the total number of carboxy groups (the total number of amino groups/ the total number of carboxy groups) in the components (A) and (B) according to claim 1 is less than 1, or
wherein a ratio of the total number of amino groups to the total number of carboxy groups (the total number of amino groups/ the total number of carboxy groups) in the amino acid and the carboxylic acid according to claim 19 is less than 1.

**43.** The formulation or composition for use in cosmetics according to any one of the preceding claims.

**INTERNATIONAL SEARCH REPORT**

International application No.
**PCT/JP2022/018556**

**A. CLASSIFICATION OF SUBJECT MATTER**

***A61Q 19/00***(2006.01)i; ***C09K 3/00***(2006.01)i; ***A61Q 5/00***(2006.01)i; ***A61K 8/36***(2006.01)i; ***A61K 8/44***(2006.01)i
FI: C09K3/00 Z; A61K8/44; A61K8/36; A61Q5/00; A61Q19/00

According to International Patent Classification (IPC) or to both national classification and IPC

**B. FIELDS SEARCHED**

Minimum documentation searched (classification system followed by classification symbols)

A61Q19/00; C09K3/00; A61Q5/00; A61K8/36; A61K8/44

Documentation searched other than minimum documentation to the extent that such documents are included in the fields searched

Published examined utility model applications of Japan 1922-1996
Published unexamined utility model applications of Japan 1971-2022
Registered utility model specifications of Japan 1996-2022
Published registered utility model applications of Japan 1994-2022

Electronic data base consulted during the international search (name of data base and, where practicable, search terms used)

JSTPlus/JMEDPlus/JST7580 (JDreamIII); CAplus/REGISTRY (STN); Japio-GPG/FX

**C. DOCUMENTS CONSIDERED TO BE RELEVANT**

| Category* | Citation of document, with indication, where appropriate, of the relevant passages | Relevant to claim No. |
|---|---|---|
| X | JP 2005-534639 A (UNILEVER N. V.) 17 November 2005 (2005-11-17)<br>claims 1-8, table 2 | 1-43 |
| X | JP 2001-342451 A (SHISEIDO CO., LTD.) 14 December 2001 (2001-12-14)<br>tables 1, 2 | 1-43 |
| X | JP 2019-23185 A (MIYOSHI OIL & FAT CO., LTD.) 14 February 2019 (2019-02-14)<br>claims 1-12, paragraph [0073] | 1-43 |
| X | WO 2020/166674 A1 (MIYOSHI OIL & FAT CO., LTD.) 20 August 2020 (2020-08-20)<br>claims 1-15, paragraphs [0181]-[0184] | 1-43 |
| X | WO 2020/166678 A1 (MIYOSHI OIL & FAT CO., LTD.) 20 August 2020 (2020-08-20)<br>claims 1-45, paragraphs [0152]-[0155], tables 1A-1B | 1-43 |
| X | JP 2020-512984 A (L'OREAL) 30 April 2020 (2020-04-30)<br>claims 1-36 | 1-43 |
| X | JP 7-316033 A (KAO CORP.) 05 December 1995 (1995-12-05)<br>claims 1-6, examples 1-7 | 1-43 |

☑ Further documents are listed in the continuation of Box C. ☑ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **24 June 2022** | **12 July 2022** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | Telephone No. |

Form PCT/ISA/210 (second sheet) (January 2015)

**INTERNATIONAL SEARCH REPORT**

International application No.
**PCT/JP2022/018556**

**C. DOCUMENTS CONSIDERED TO BE RELEVANT**

| Category* | Citation of document, with indication, where appropriate, of the relevant passages | Relevant to claim No. |
|---|---|---|
| X | JP 2005-247729 A (NIKKO CHEMICAL CO., LTD.) 15 September 2005 (2005-09-15) claims 1-3, examples 1-8 | 1-43 |
| X | LEISS, Sebastian. Feramentative Herstellung von L-Lysin-L-lactat mittels Fraktionierungssaften aus der Grunen Bioraffinerie, Chemie Ingenieur Technik, 25 June 2010, vol. 82, no. 7, pp. 1091-1095 abstract | 1 |
| A | JP 2018-168081 A (ASANUMA CORP.) 01 November 2018 (2018-11-01) examples | 1-43 |
| A | JP 2014-131974 A (MIYOSHI OIL & FAT CO., LTD.) 17 July 2014 (2014-07-17) examples | 1-43 |
| A | WO 2013/092080 A1 (HENKEL AG & CO. KGAA) 27 June 2013 (2013-06-27) Beispiele | 1-43 |

Form PCT/ISA/210 (second sheet) (January 2015)

**INTERNATIONAL SEARCH REPORT**
**Information on patent family members**

International application No.
**PCT/JP2022/018556**

| Patent document cited in search report | Publication date (day/month/year) | Patent family member(s) | Publication date (day/month/year) |
|---|---|---|---|
| JP 2005-534639 A | 17 November 2005 | JP 2005-530807 A | |
| | | JP 2005-531568 A | |
| | | JP 2005-532328 A | |
| | | JP 2005-538053 A | |
| | | US 2003/0224023 A1 claims 1-7, table 4 | |
| | | US 2003/0224027 A1 | |
| | | US 2004/0185074 A1 | |
| | | WO 2003/099238 A1 | |
| | | WO 2003/099250 A1 | |
| | | WO 2003/099251 A1 | |
| | | WO 2003/099252 A1 | |
| | | WO 2003/099254 A1 | |
| | | EP 1507510 A1 | |
| | | EP 1507512 A1 | |
| | | EP 1509190 A1 | |
| | | EP 1509195 A1 | |
| | | EP 1509197 A1 | |
| | | CA 2483642 A | |
| | | CA 2485335 A | |
| | | CA 2486879 A | |
| | | CA 2486882 A | |
| | | CA 2486905 A | |
| | | AT 319503 T | |
| | | ES 2259764 T | |
| | | AT 353027 T | |
| | | CN 1655758 A | |
| | | CN 1655752 A | |
| | | CN 1665479 A | |
| | | CN 1671352 A | |
| | | ZA 200408659 A | |
| | | ES 2280754 T | |
| | | KR 10-2005-0004248 A | |
| | | KR 10-2005-0007562 A | |
| | | KR 10-2005-0009718 A | |
| | | ZA 200408938 A | |
| | | ZA 200409351 A | |
| | | ZA 200409493 A | |
| | | ZA 200409494 A | |
| | | AR 39906 A | |
| | | AR 39907 A | |
| | | AR 39908 A | |
| | | AR 39909 A | |
| | | AR 39910 A | |
| | | AU 2003232824 A | |
| | | AU 2003238392 A | |
| | | AU 2003240709 A | |
| | | AU 2003240710 A | |
| | | AU 2003240711 A | |
| | | MX PA04011776 A | |

Form PCT/ISA/210 (patent family annex) (January 2015)

**INTERNATIONAL SEARCH REPORT**
**Information on patent family members**

International application No.
**PCT/JP2022/018556**

| Patent document cited in search report | Publication date (day/month/year) | Patent family member(s) | Publication date (day/month/year) |
|---|---|---|---|
| | | MX PA04011779 A | |
| | | MX PA04011904 A | |
| | | MX PA04011905 A | |
| | | TW 200404572 A | |
| | | TW 200404573 A | |
| | | TW 200404574 A | |
| | | TW 200406227 A | |
| | | TW 200500089 A | |
| | | CN 1655757 A | |
| JP 2001-342451 A | 14 December 2001 | US 2003/0072805 A1<br>tables 1, 2 | |
| | | US 2009/0047312 A1 | |
| | | WO 2001/051558 A1 | |
| | | EP 1158021 A1 | |
| | | TW 279416 B | |
| | | KR 10-2001-0102550 A | |
| JP 2019-23185 A | 14 February 2019 | (Family: none) | |
| WO 2020/166674 A1 | 20 August 2020 | EP 3925672 A1<br>claims 1-15, paragraphs [0199], [0200] | |
| | | CN 113423800 A | |
| | | KR 10-2021-0126563 A | |
| WO 2020/166678 A1 | 20 August 2020 | EP 3925671 A1<br>claims 1-45, paragraph [0159], tables 1A-1B | |
| | | CN 113423470 A | |
| | | KR 10-2021-0126551 A | |
| JP 2020-512984 A | 30 April 2020 | US 2018/0280271 A1<br>claims 1-16 | |
| | | WO 2018/183869 A1 | |
| | | EP 3630054 A1 | |
| | | CN 110621290 A | |
| | | ZA 201906236 B | |
| JP 7-316033 A | 05 December 1995 | (Family: none) | |
| JP 2005-247729 A | 15 September 2005 | (Family: none) | |
| JP 2018-168081 A | 01 November 2018 | (Family: none) | |
| JP 2014-131974 A | 17 July 2014 | (Family: none) | |
| WO 2013/092080 A1 | 27 June 2013 | DE 102011089612 A | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2014131974 A
- JP 2014131975 A
- JP 2019023185 A
- WO 2020166674 A1
- WO 2020166678 A1